# EUROPEAN PATENT APPLICATION

(11) **EP 4 248 997 A1**
(43) Date of publication of application: **27.09.2023**
(21) Application number: 21894619.2
(22) Date of filing: 16.11.2021
(51) Int. Cl.: A61K 45/00, A61K 31/192, A61K 31/196, A61K 31/198, A61K 31/216, A61K 31/235, A61K 31/245, A61K 31/27, A61K 31/341, A61K 31/357, A61K 31/36, A61K 31/403, A61K 31/4035, A61K 31/404, A61K 31/405, A61K 31/4439, A61K 31/455, A61K 31/47, A61K 31/4709, A61K 31/472, A61K 31/498

(54) **THERAPEUTIC AGENT OR PROPHYLACTIC AGENT FOR COVID-19**

(30) Priority: 17.11.2020 JP 2020191046
(71) Applicant: Hiroshima University, Higashihiroshima-shi Hiroshima 739-8511 (JP); Tokyo University of Pharmacy & Life Sciences, Hachioji-shi, Tokyo 192-0392 (JP); The University of Tokyo, Bunkyo-ku, Tokyo 113-8654 (JP)
(72) Inventor: ASANO, Tomoichiro, Hiroshima 739-8511 (JP); SAKAGUCHI, Takemasa, Hiroshima 739-8511 (JP); YAMAMOTOYA, Takeshi, Hiroshima 739-8511 (JP); NAKATSU, Yusuke, Hiroshima 739-8511 (JP); ITO, Hisanaka, Tokyo 192-0392 (JP); OKABE, Takayoshi, Tokyo 113-8654 (JP); ENCINAS, Jeffrey, Hyogo 659-0082 (JP)
(74) Representative: J A Kemp LLP
(86) International application number: PCT/JP2021/042013
(87) International publication number: WO 2022/107745

(57) **Abstract**

[Problem] To develop novel therapeutic agents for viral diseases such as coronavirus infections.

[Solution] The present invention provides a therapeutic or prophylactic agent for viral diseases containing a compound with inhibitory activity against the function of Pin1 or a pharmaceutically acceptable salt thereof as an active ingredient.

## Description

### TECHNICAL FIELD

The present invention relates to therapeutic or prophylactic agents for viral diseases caused by viruses that infect humans, such as coronavirus infection, particularly coronavirus infection caused by beta-coronavirus and, more particularly the coronavirus infection COVID-19 caused by SARS coronavirus 2 (SARS-CoV-2), which therapeutic or prophylactic agent contains a compound that inhibits the function of Pin 1 as an active ingredient.

### BACKGROUND ART

Coronaviruses are viruses that infect not only human but also other animals and cause various diseases. Coronaviruses specific to each animal (animal coronaviruses) have been detected for not only domestic animals such as dogs, cats, cows, pigs, chicken, horses, alpacas and camels, but also belugas whales, giraffes, ferrets, shrews, bats and sparrows. Coronaviruses exhibit high species-specificity and rarely jump the species barrier to infect other species. As coronaviruses that infect humans: four kinds of commonly transmitted coronaviruses (Human Coronavirus: HCoV), HCoV-229E, HCoV-OC43, HCoV-NL63 and HCoV-HKU1; and two kinds of severe pneumonia-causing coronaviruses transmitted from animals, Severe Acute Respiratory Syndrome-associated Coronavirus (SARS-CoV) and Middle East Respiratory Syndrome Coronavirus (MERS-CoV) had previously been known.

Around December 2019, infections with a novel coronavirus, SARS coronavirus 2 (SARS-CoV-2: Severe Acute Respiratory Syndrome CoronaVirus 2), were identified and rapidly spread around the world, resulting in a global pandemic that still shows no sign of ending. The infectious disease caused by SARS coronavirus 2 (COVID-19) mainly spreads by human-to-human transmission through airborne droplets expelled by coughs and sneezes of an infected person. COVID-19 causes fever, respiratory symptoms, headache, fatigue and the like and can also cause olfactory or taste disorders. In particular, the elderly and patients with an underlying medical condition (cardiovascular disease, diabetes, chronic respiratory disease, chronic kidney disease, hypertension, or obesity) have higher mortality from COVID-19. Under these circumstances, effective therapeutic and prophylactic drugs for COVID-19 are highly desired.

Coronaviruses are a type of RNA virus (single-stranded RNA virus) with RNA as its genetic material and have an outermost lipid bilayer membrane called an envelope around their viral particles. The particles are spherical with an average diameter of approximately 100 nm and having projections on the surface, giving a crown-like appearance. Coronaviruses cannot proliferate by themselves, but they adhere to and enter cells such as cells of human mucous membranes to proliferate. Virologically, coronaviruses are classified into the order *Nidovirales,* the subfamily *Coronavirinae,* and the family *Coronaviridae,* and according to their genetic characteristics, they are classified into the four groups, alpha, beta, gamma and delta. HCoV-229E and HCoV-NL63 are classified as alpha-coronaviruses, and MERS-CoV, SARS-CoV, SARS-CoV-2, HCoV-OC43 and HCoV-HKU1 are classified as beta-coronaviruses.

Pin1 is a kind of peptidyl-prolyl *cis-trans* isomerase (PPIase) that catalyzes *cis*/*trans* isomerization of proline residues in proteins, and is characterized by specifically acting on proline residues immediately preceded by phosphorylated serine or threonine to change the conformation of those proline residues. Accordingly, Pin1 is a molecule that links phosphorylation of a protein to conformational change of the protein, and is considered to play an important role in intracellular signal transduction. It has been reported that Pin1 expression level is significantly increased in several tissues such as liver, muscle, adipose tissue and kidney from obese mice or diabetic mice (Non-Patent Documents 1-3). It has also been reported that Pin1 facilitates the proliferation of some viruses such as cytomegalovirus, Epstein-Barr virus, hepatitis B virus, hepatitis C virus, human immunodeficiency virus and feline coronavirus (Non-Patent Documents 4-10). However, it is speculated that the molecular mechanisms underlying Pin1-induced facilitation of viral proliferation differ among these viruses.

As compounds that inhibit Pin1, a phenylalaninol-phosphate derivative, an indole- or benzimidazole-alanine derivative, a fredericamycin A compound, a phenyl-imidazole derivative, a naphthyl-substituted amino acid derivative, a glutamate or aspartate derivative, and the like have been reported (Patent Documents 1 to 4 and Non-Patent Documents 11 to 14). Furthermore, it is also known that a Pin1 inhibitor with a specific structure can be used as a therapeutic or prophylactic agent for fatty liver disease, a therapeutic or prophylactic agent for obesity, a therapeutic agent for inflammatory diseases, and a therapeutic agent for cancer (Patent Documents 5-7 and Non-Patent Document 15). In addition, it has also been reported that the Pin1 inhibitor dipentamethylenethiuram monosulfide inhibited replication of feline coronavirus (FCoV) (Non-Patent Documents 10).

### PRIOR ART REFERENCES

### PATENT DOCUMENTS

Patent Document 1: WO 2004/087720
Patent Document 2: WO 2006/040646
Patent Document 3: WO 2005/007123
Patent Document 4: WO 2002/060436
Patent Document 5: WO 2019/031471
Patent Document 6: WO 2019/031472
Patent Document 7: WO 2019/031470

### NON-PATENT DOCUMENTS

Non-Patent Document 1: Yusuke Nakatsu, and 10 other authers, International Journal of Molecular Sciences, September 7, 2016, Vol. 17(9), E1495
Non-Patent Document 2: Yusuke Nakatsu, and 20 other authers, Journal of Biological Chemistry, December 2012, Vol. 287(53), p. 44526-35
Non-Patent Document 3: Yusuke Nakatsu, and 22 other authers, Journal of Biological Chemistry, June 2011, Vol. 286(23), p. 20812-22
Non-Patent Document 4: Martin Schutz, and 9 other authers, Virus Research, August 2020, Vol. 285, 198023
Non-Patent Document 5: Yohei Narita, and 7 other authers, Journal of Virology, February 2013, Vol. 87, No. 4, p. 2120-2127
Non-Patent Document 6: Mayuko Nishi, and 9 other authers, Frontiers in Cell and Developmental Biology, January 2020, Vol. 8, Article 26
Non-Patent Document 7: Yun-Sook Lim, and 4 other authers, JOURNAL OF VIROLOGY, September 2011, Vol. 85, No. 17, p. 8777-8788
Non-Patent Document 8: Hai Hou, and 3 other authers, Gene, June 2015, Vol. 565, Issue 1, p. 9-14
Non-Patent Document 9: Shogo Misumi, and 6 other authers, Journal of Biological Chemistry, August 13, 2010, Vol. 285, No. 33, p. 25185-25195
Non-Patent Document 10: Yoshikazu Tanaka, and 4 other authers, Antiviral Research, Published: February 2016, Vol.126, pp. 1-7
Non-Patent Document 11: Andrew Potter, and 16 other authors, Bioorganic & Medicinal Chemistry Letters (Bioorg. Med. Chem. Lett.), Published: November 15, 2010 (Epub: September 17, 2010), Vol. 20, No. 22, pp. 6483-6488.
Non-Patent Document 12: Andrew Potter, and 14 other authors, Bioorganic & Medicinal Chemistry Letters (Bioorg. Med. Chem. Lett.), Published: January 15, 2010 (Epub: November 22, 2009), Vol. 20, No. 2, pp. 586-590.
Non-Patent Document 13: Liming Dong, and 11 other authors, Bioorganic & Medicinal Chemistry Letters (Bioorg. Med. Chem. Lett.), Published: April 1, 2010 (Epub: February 14, 2010), Vol. 20, No. 7, pp. 2210-2214.
Non-Patent Document 14: Hidehiko Nakagawa, and 6 other authers, Bioorganic & Medicinal Chemistry Letters (Bioorg. Med. Chem. Lett.), Published: December 1, 2015 (Epub: October 22, 2015), Vol.25, pp.5619-5624
Non-Patent Document 15: Tomoichiro Asano, Journal of Japan Foundation for Applied Enzymology, Published: March 1, 2014, No.48, pp.39-40.

### DISCLOSURE OF INVENTION

### PROBLEMS TO BE SOLVED BY THE INVENTION

In view of the existing situation as described above, an object of the present invention is to develop novel therapeutic or prophylactic agents for infectious diseases caused by coronaviruses that infect humans, particularly coronavirus infection caused by beta-coronavirus and, more particularly the coronavirus infection, COVID-19, caused by SARS coronavirus 2 (SARS-CoV-2).

### MEANS FOR SOLVING THE PROBLEMS

The inventors intensively studied to solve the above-described problem. Considering the facts that COVID-19 is associated with high mortality in obese patients and that Pin1 expression level is significantly increased in livers of human subjects having steatosis and other facts, the inventors investigated the effect of Pin1 on proliferation of SARS-CoV-2 and found that proliferation of SARS-CoV-2 can be suppressed using a Pin1 inhibitor, and consequently have accomplished the present invention.

That is, the present invention is therapeutic or prophylactic agents for viral diseases caused by viruses that infect humans, for example therapeutic or prophylactic agents for coronavirus infection, particularly therapeutic or prophylactic agents for coronavirus infection caused by beta-coronaviruses, more particularly coronavirus infection caused by SARS-CoV-2, wherein the therapeutic or prophylactic agents contain a compound with a function of inhibiting Pin1 or a pharmaceutically acceptable salt thereof as an active ingredient. The compound with a function of inhibiting Pin1 may be a compound inhibiting the PPIase activity of the Pin1 enzyme, a compound inhibiting binding between the Pin1 enzyme and a target protein, or a compound facilitating degradation of the Pin1 enzyme.

The aforementioned compound may be a compound represented by the following Formula (I-I) (hereinafter referred to as an "anthranilic acid compound"): 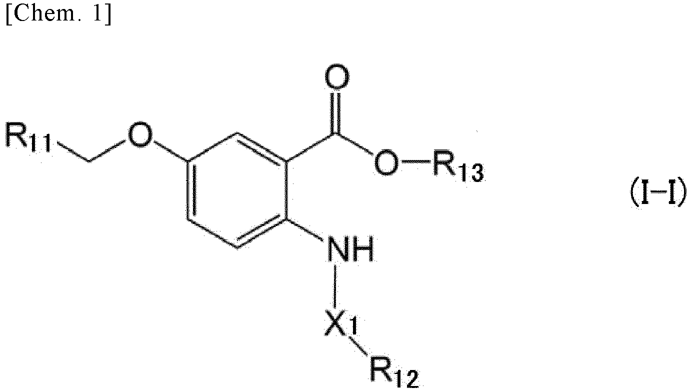 (wherein at least one of R₁₁ and R₁₂ is an optionally substituted polycyclic aryl group, an optionally substituted polycyclic heterocyclic group, or a group represented by the following Formula (I-II): 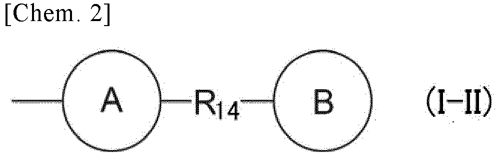 (wherein rings A and B respectively represent an optionally substituted monocyclic or polycyclic aryl group, and R₁₄ represents an optionally substituted alkylene group having 1 to 3 carbon atoms, an optionally substituted alkenylene group having 2 to 3 carbon atoms, or divalent oxy group),
if neither R₁₁ nor R₁₂ is any of the aforementioned groups, R₁₁ or R₁₂ is a hydrogen atom, an optionally substituted hydrocarbon group, or an optionally substituted monocyclic heterocyclic group,
R₁₃ is a hydrogen atom, an optionally substituted hydrocarbon group, or an optionally substituted heterocyclic group, and
X₁ is a single bond, -CO- group, -CO-O-CH₂- group, -CO-CHz-O- group, -SO₂-group, -CH₂-CO-NH- group, -CH₂-CO- group, -CH₂- group, or -CO-CH₂- group).

With respect to the anthranilic acid compound or the salt thereof, at least one of R₁₁ and R₁₂ is preferably an optionally substituted polycyclic aryl group.

In this case, it is more preferable that at least one of R₁₁ and R₁₂ is an optionally substituted naphthyl group.

Even more preferably, R₁₁ is an optionally substituted naphthyl group.

With respect to the anthranilic acid compound or the salt thereof, R₁₃ is preferably a hydrogen atom or a methyl group.

With respect to the anthranilic acid compound or the salt thereof, X₁ is preferably -CO- group.

The aforementioned compound may also be a compound represented by the following Formula (II-I) (hereinafter referred to as an "amide compound"): 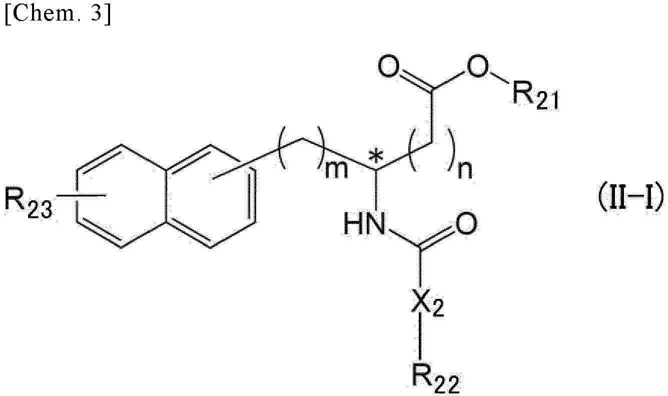 (wherein m represents an integer from 0 to 2, n represents an integer from 0 to 1, and 0≤m+n≤2,
R₂₁ is a hydrogen atom, an optionally substituted hydrocarbon group, an optionally substituted heterocyclic group, or an optionally substituted amino group,
R₂₂ is an optionally substituted aryl group containing a condensed ring of three or more rings, a substituted naphthyl group, or a group represented by the following Formulae (II-II), (II-III), (II-IV), or (II-V): 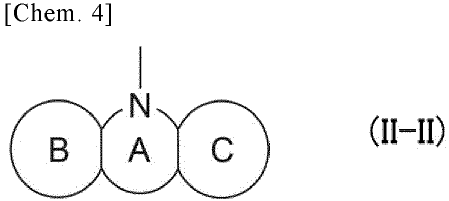 (wherein a ring A represents an optionally substituted monocyclic heterocyclic group, and rings B and C respectively represent an optionally substituted monocyclic or polycyclic aryl group or heterocyclic group, and the rings A, B and C form a condensed ring); 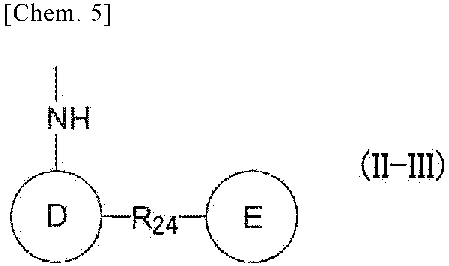 (wherein rings D and E respectively represent an optionally substituted monocyclic or polycyclic aryl group or heterocyclic group, and R₂₄ represents a divalent oxy group or a carbonyl group); 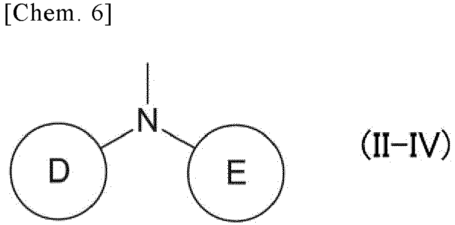 (wherein rings D and E respectively represent an optionally substituted monocyclic or polycyclic aryl group or heterocyclic group); 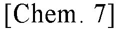 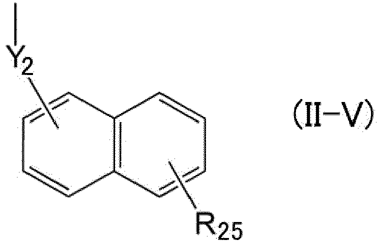 (wherein R₂₅ represents 0 to 7 identical or different substituents attached to a naphthyl group, and Y₂ represents -NH- group or an alkylene group having 1 or 2 carbon atoms),
R₂₃ is 0 to 7 identical or different substituents attached to a naphthyl group, and
X₂ is a single bond, -CH₂- group, or -NH- group).

In the amide compound or salt thereof, the R₂₂ is preferably a group represented by the following Formula (II-II): 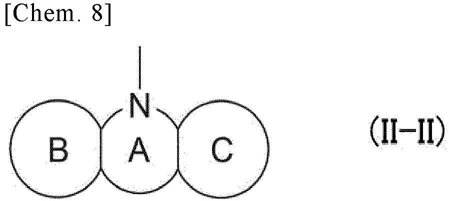 (wherein a ring A represents an optionally substituted monocyclic heterocyclic group, rings B and C respectively represent an optionally substituted monocyclic or polycyclic aryl group or heterocyclic group, and the rings A, B and C form a condensed ring).

In the amide compound or salt thereof, the m is preferably 1, and the n is preferably 0.

In the amide compound or salt thereof, the R₂₁ is preferably a hydrogen atom.

In the amide compound or salt thereof, the X₂ is preferably a single bond.

The aforementioned compound may also be a compound represented by the following Formula (III-I) (hereinafter referred to as a "carboxylic-acid-derivative compound"): 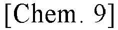 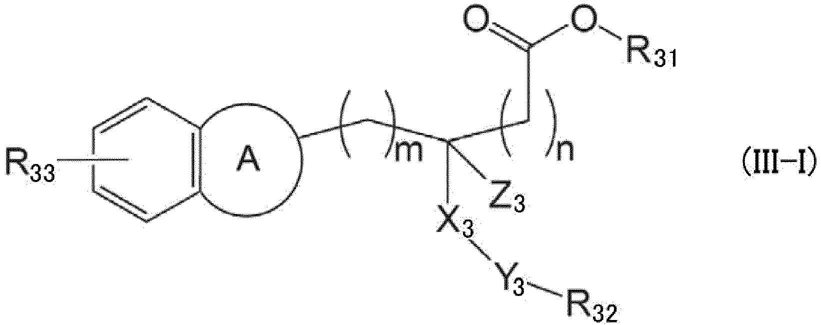 (wherein m represents an integer from 0 to 2, n represents an integer from 0 to 2, and 0≤m+n≤3,
a ring A is an optionally substituted monocyclic aromatic ring or heterocycle,
X₃ is a single bond, -NH- group, -NH-CO- group, -O-CO- group, -CH₂-S-CH₂-group, -CH₂-S-(CH₂)₂-S- group, or -NH-R₃₄- group (R₃₄ is an alkylene group having 1 to 5 carbon atoms, or an alkenylene group having 2 to 5 carbon atoms),
Y₃ is a single bond, -NH- group, -CO- group, -SO₂- group, -O-CO- group, -CO-NR₃₅- group (R₃₅ represents a hydrogen atom, or an optionally substituted alkyl group having 1 to 5 carbon atoms), -O-R₃₆- group (R₃₆ represents an optionally substituted alkylene group having 1 to 5 carbon atoms, or an optionally substituted alkenylene group having 2 to 5 carbon atoms), -NR₃₅-R₃₆- group, -S-R₃₆- group, -CO-R₃₆- group, - SO₂-R₃₆- group, -NR₃₅-CO-R₃₆- group, -R₃₆-NR₃₅-CO- group, an optionally substituted alkylene group having 1 to 6 carbon atoms, or an optionally substituted alkenylene group having 2 to 6 carbon atoms,
Z₃ is a hydrogen atom, an optionally substituted amino group, or an optionally substituted amine,
if the Z₃ is the optionally substituted amine, the X₃ and the Z₃ form a ring,
R₃₁ is a hydrogen atom, an optionally substituted hydrocarbon group, an optionally substituted heterocyclic group, or an optionally substituted amino group,
R₃₂ is an optionally substituted aryl group, an optionally substituted heterocyclic group, an optionally substituted cycloalkyl group, or a group represented by the following Formula (III-II): 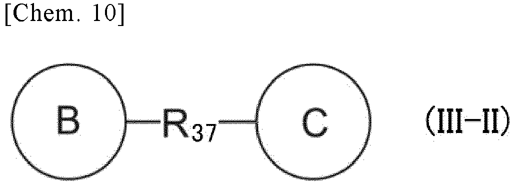 (wherein rings B and C respectively represent an optionally substituted aryl group, an optionally substituted heterocyclic group, or an optionally substituted cycloalkyl group, R₃₇ represents , a single bond, -O- group, -CO- group, -NH- group, -SO₂- group, -CO-NH- group, an optionally substituted alkylene group having 1 to 3 carbon atoms, an optionally substituted alkenylene group having 2 to 3 carbon atoms, -S-R₃₈- group (R₃₈ represents an optionally substituted alkylene group having 1 to 2 carbon atoms), -COR₃₈- group, -O-R₃₈- group, or -SO₂-R₃₈- group, and any of the ring B, ring C and R₃₇ is attached to the Y₃), and
R₃₃ is 0 to 4 identical or different substituents).

In the carboxylic-acid-derivative compound or salt thereof, the m is preferably 1, and the n is preferably 0.

In the carboxylic-acid-derivative compound or salt thereof, the X₃ is preferably -NH-CO- group, or -O-CO- group.

In the carboxylic-acid-derivative compound or salt thereof, the ring A is preferably a benzene ring.

In the carboxylic-acid-derivative compound or salt thereof, the R₃₂ is preferably an optionally substituted polycyclic aryl group, or an optionally substituted polycyclic heterocyclic group.

In the carboxylic-acid-derivative compound or salt thereof, the Z₃ is preferably a hydrogen atom.

In the carboxylic-acid-derivative compound or salt thereof, the Y₃ is preferably a single bond, -CH₂- group, or -CH₂-O- group.

In the carboxylic-acid-derivative compound or salt thereof, the R₃₁ is preferably a hydrogen atom.

The aforementioned compound may also be a compound represented by the following Formula (IV-I) (hereinafter referred to as a "3,5-diaminobenzoic acid compound"): 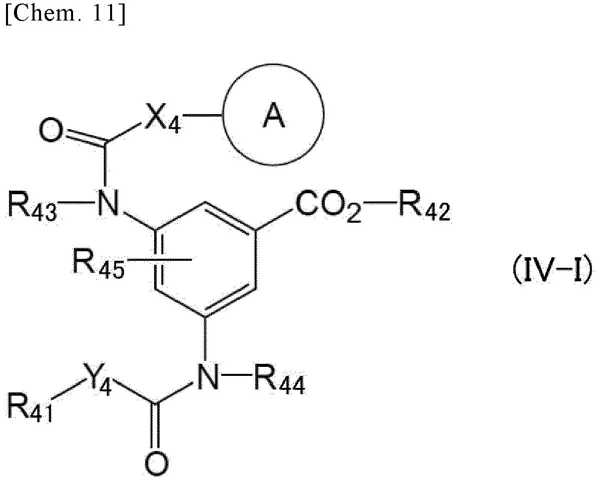 (wherein a ring A represents an optionally substituted monocyclic or polycyclic aromatic ring or heterocycle,
R₄₁ represents an optionally substituted polycyclic aromatic ring or a group represented by any of the following Formulae (IV-II) to (IV-V): 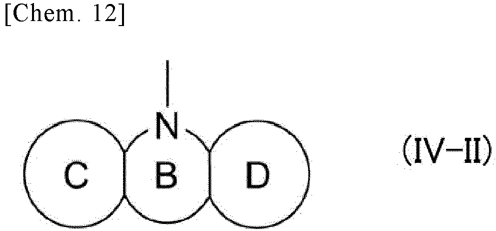 (wherein a ring B represents an optionally substituted monocyclic heterocycle, rings C and D independently represent an optionally substituted monocyclic or polycyclic aromatic ring, heterocycle or cyclic hydrocarbon, and the rings B, C and D form a condensed ring); 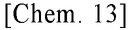 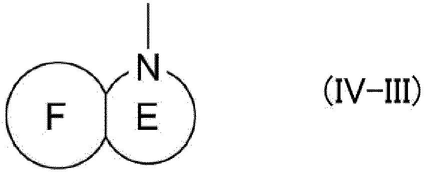 (wherein a ring E represents an optionally substituted monocyclic heterocycle, a ring F represents an optionally substituted monocyclic or polycyclic aromatic ring, heterocycle or cyclic hydrocarbon, and the rings E and F form a condensed ring); 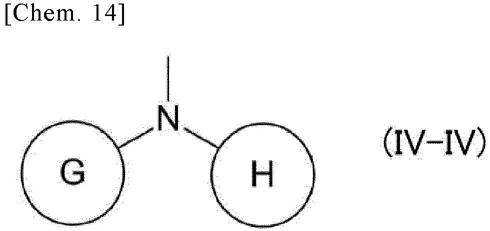 (wherein rings G and H respectively represent an optionally substituted monocyclic or polycyclic aromatic ring or heterocycle); 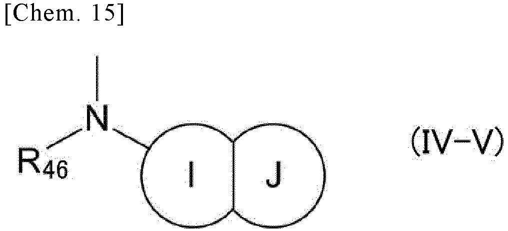 (wherein a ring I represents an optionally substituted monocyclic aromatic ring or heterocycle, a ring J represents an optionally substituted monocyclic or polycyclic aromatic ring, heterocycle or cyclic hydrocarbon, the rings I and J form a condensed ring, and R₄₆ represents a hydrogen atom, an optionally substituted hydrocarbon group, or an optionally substituted heterocyclic group);
R₄₂ represents a hydrogen atom, an optionally substituted hydrocarbon group, an optionally substituted heterocyclic group, or an optionally substituted amino group;
R₄₃ represents a hydrogen atom, an optionally substituted hydrocarbon group, or an optionally substituted heterocyclic group;
R₄₄ represents a hydrogen atom, an optionally substituted hydrocarbon group, or an optionally substituted heterocyclic group;
R₄₅ represents 0 to 3 identical or different substituents attached to a benzene ring;
X₄ represents a single bond, an alkylene group having 1 or 2 carbon atoms, -O-group, -CH₂-O- group, -CH₂-NH-CO- group, or -CH₂-NH-CO-O-CH₂- group; and
Y₄ represents a single bond or an alkylene group having 1 or 2 carbon atoms).

In the 3,5-diaminobenzoic acid compound and the salt thereof, the ring A is preferably an optionally substituted polycyclic aromatic ring or heterocycle.

In this case, the ring A is preferably a ring represented by the following Formula (IV-VI): 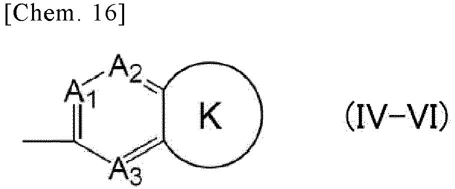 (wherein A₁, A₂, and A₃ independently represent a carbon atom or a nitrogen atom, and a ring K represents an optionally substituted monocyclic or polycyclic aromatic ring, heterocycle or cyclic hydrocarbon).

In the Formula (IV-VI), all of A₁, A₂, and A₃ are preferably a carbon atom.

In 3,5-diaminobenzoic acid compound or the salt thereof, the R₄₁ is preferably a group represented by the following Formula (IV-II): 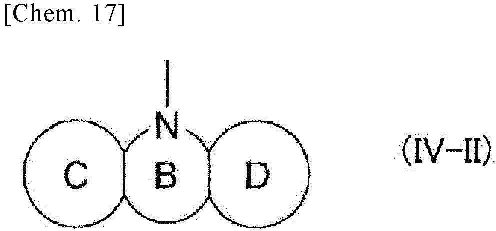 (wherein a ring B represents an optionally substituted monocyclic heterocycle, rings C and D independently represent an optionally substituted monocyclic or polycyclic aromatic ring, heterocycle or cyclic hydrocarbon, and the rings B, C and D form a condensed ring).

In 3,5-diaminobenzoic acid compound or the salt thereof, the R₄₂ is preferably a hydrogen atom.

In 3,5-diaminobenzoic acid compound or the salt thereof, the R₄₃ is preferably a hydrogen atom.

In 3,5-diaminobenzoic acid compound or the salt thereof, the R₄₄ is preferably a hydrogen atom.

In 3,5-diaminobenzoic acid compound or the salt thereof, the X₄ is preferably a single bond.

In 3,5-diaminobenzoic acid compound or the salt thereof, the Y₄ is preferably a single bond.

The present invention also provides a method for screening therapeutic or prophylactic agents for COVID-19, comprising steps of:
A) acquiring a library of Pin1 inhibitors by measuring their activity of inhibiting the function of Pin1 using a cell-free assay for measuring cis-to-trans conversion of a phosphorylated serine/threonine-proline motif contained in a substrate peptide; and
B) measuring ability to inhibit proliferation of SARS-CoV-2 virus of compounds identified as Pin1 inhibitors in the step A using a cell-based assay for measuring increases in viral components.

The present invention also provides a therapeutic or prophylactic agent for COVID-19 containing the Pin1 inhibitors identified by the above-described screening method or pharmaceutically acceptable salts thereof as an active ingredient.

The therapeutic or prophylactic agent for any of coronavirus infections can further contain one or more active ingredients of at least one or more other drugs selected from drugs classified as therapeutic or prophylactic agents for coronavirus infections.

Moreover, the therapeutic or prophylactic agent for any of coronavirus infections may be used in combination with at least one or more other drugs selected from drugs classified as therapeutic or prophylactic agents for coronavirus infections.

In addition, the present invention provides any of the above-described compounds or pharmaceutically acceptable salts thereof for use as a therapeutic or prophylactic agent for coronavirus infection.

The present invention also provides use of any of the above-described compounds or pharmaceutically acceptable salts thereof for manufacture of a medicament for treating or preventing coronavirus infection.

The present invention also provides a method of treating or preventing coronavirus infection by administering to a patient a therapeutically effective amount of the above-described compound or pharmaceutically acceptable salt thereof.

The present invention also provides a compound with a function of inhibiting Pin1 or a pharmaceutically acceptable salt thereof for use in treating or preventing coronavirus infection.

### EFFECTS OF THE INVENTION

The therapeutic or prophylactic agent for viral diseases according to the present invention suppresses the proliferation of viruses and thus has an effect of treating viral diseases or of preventing viral diseases. In particular, it can treat or prevent coronavirus infection caused by coronavirus, specifically beta-coronaviruses, and especially beneficially SARS-CoV2.

### BRIEF DESCRIPTION OF THE DRAWINGS

FIG. 1 (A) shows results of Western blotting of SARS-CoV-2 nucleocapsid, Pin1 and actin in cells when either one of two kinds of Pin1 siRNAs and control siRNA is added, and (B) shows the relative intensity of SARS-CoV-2 nucleocapsid normalized to the detection intensity of SARS-CoV-2 nucleocapsid in control siRNA.
FIG. 2 (A) shows results of Western blotting of SARS-CoV-2 nucleocapsid, Pin1 and actin in cells when a Pin1 inhibitor H-77 is added at a final concentration of 10 µM, and (B) shows results of real-time PCR quantification of SARS-CoV-2 RNA level when H-77 was added at final concentrations of 0, 2, 5, 7.5, and 10 µM.
FIGs. 3 show photographs of VeroE6/TMPRSS2 cells 20 hours after infection with SARS-CoV-2, wherein (A) is the case with the addition of H-77, and (B) is the case with the addition of DMSO as a control.
FIG. 4 shows results of Western blotting of SARS-CoV-2 nucleocapsid and actin in cells when a Pin1 inhibitor included in the Formula (I-I) was added at a final concentration of 5 or 10 µM.
FIG. 5 shows results of Western blotting of SARS-CoV-2 nucleocapsid and actin in cells when a Pin1 inhibitor included in the Formula (II-I) was added at a final concentration of 5 or 10 µM.
FIG. 6 shows results of Western blotting of SARS-CoV-2 nucleocapsid and actin in cells when a Pin1 inhibitor included in the Formula (III-I) was added at a final concentration of 5 or 10 µM.
FIG. 7 shows results of Western blotting of SARS-CoV-2 nucleocapsid and actin in cells when a Pin1 inhibitor included in the Formula (IV-I) was added at a final concentration of 5 or 10 µM.
FIG. 8 (A) shows results of Western blotting of SARS-CoV-2 nucleocapsid, Pin1 and actin in cells when a Pin1 inhibitor ATRA is added at final concentrations of 0, 0.5, 1, 5, 10, 20, and 30µM, and (B) shows the result of real-time PCR quantification of SARS-CoV-2 RNA level.
FIG. 9 (A) shows results of Western blotting of SARS-CoV-2 nucleocapsid, Pin1 and actin in cells when a Pin1 inhibitor H-77 is added 1). 2 hours before infection, 2). at the time of infection, 3). 2 hours after infection, and 4). 6 hours after infection, or 5). when the Pin1 inhibitor H-77 is not added, and (B) shows results of real-time PCR quantification of SARS-CoV-2 RNA level.

### DESCRIPTION OF EMBODIMENTS

### Therapeutic or Prophylactic Agent for Viral Diseases

The compounds that are active ingredients of the present invention are compounds that have inhibitory activity against the function of Pin1, and the compounds include, for example, compounds having the following chemical structures 1-1 to 1-5 or salts thereof.

### 1.Chemical Structure of Active Ingredient

### 1-1. Anthranilic Acid Compound

One of the compounds that are active ingredients of the therapeutic or prophylactic agents for coronavirus disease according to present invention is a compound that has a chemical structure represented by the following Formula (I-I) and inhibitory activity against the function of Pin1:

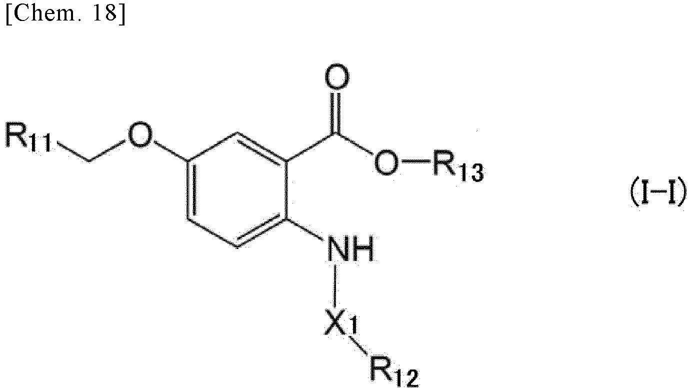

In the Formula (I-I), at least one of R₁₁ and R₁₂ is an optionally substituted polycyclic aryl group, an optionally substituted polycyclic heterocyclic group, or a group represented by the following Formula (I-II): 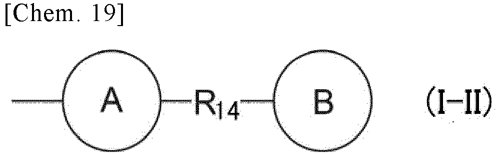 (wherein rings A and B respectively represent an optionally substituted monocyclic or polycyclic aryl group, and R₁₄ represents an optionally substituted alkylene group having 1 to 3 carbon atoms, an optionally substituted alkenylene group having 2 to 3 carbon atoms, or divalent oxy group),
if neither R₁₁ nor R₁₂ is any of the aforementioned groups, that is, R₁₁ nor R₁₂ is any of the optionally substituted polycyclic aryl group, the optionally substituted polycyclic heterocyclic group, and the group represented by the Formula (I-II), R₁₁ or R₁₂ is a hydrogen atom, an optionally substituted hydrocarbon group (except for the optionally substituted polycyclic aryl group), or an optionally substituted monocyclic heterocyclic group,

In the Formula (I-I) "at least one of R₁₁ and R₁₂" means R₁₁, R₁₂, or R₁₁ and R₁₂.

Therefore, when R₁₁ is the optionally substituted polycyclic aryl group, the optionally substituted polycyclic heterocyclic group, or the group represented by the Formula (I-II), R₁₂ may not be any of these groups or may be any one of these groups. In other words, in this case, R₁₂ may be the hydrogen atom, the optionally substituted hydrocarbon group (except for the optionally substituted polycyclic aryl group), or the optionally substituted monocyclic heterocyclic group, or alternatively R₁₂ may be the optionally substituted polycyclic aryl group, the optionally substituted polycyclic heterocyclic group, or the group represented by the Formula (I-II).

Similarly, when R₁₂ is the optionally substituted polycyclic aryl group, the optionally substituted polycyclic heterocyclic group, or the group represented by the Formula (I-II), R₁₁ may not be any of these groups or may be any one of these groups. In other words, in this case, R₁₁ may be the hydrogen atom, the optionally substituted hydrocarbon group (except for the optionally substituted polycyclic aryl group), or the optionally substituted monocyclic heterocyclic group, or alternatively R₁₁ may be the optionally substituted polycyclic aryl group, the optionally substituted polycyclic heterocyclic group, or the group represented by the Formula (I-II).

In the Formula (I-I), although at least one of R₁₁ and R₁₂ is the optionally substituted polycyclic aryl group, the optionally substituted polycyclic heterocyclic group, or the group represented by the Formula (I-II), it is preferable for enhancing the inhibitory activity against the function of Pin1 that at least one of R₁₁ and R₁₂ is the optionally substituted polycyclic aryl group, or the optionally substituted polycyclic heterocyclic group. Either one of R₁₁ and R₁₂ is more preferably the optionally substituted polycyclic aryl group, and still more preferably a polycyclic aryl group.

In the Formula (I-I), at least one of R₁₁ and R₁₂ may be the optionally substituted polycyclic aryl group. In the anthranilic acid compound according to the present invention, the "polycyclic aryl group" is a group derived from an aromatic compound containing a condensed ring of two or more rings consisting only of carbon.

In this respect, it is preferable to use a bicyclic to tetracyclic aryl group as the "polycyclic aryl group".

Examples of the "polycyclic aryl group" in the anthranilic acid compound according to the present invention can include, but are not limited to, an indenyl group, a naphthyl group, a fluorenyl group, an anthryl group, a biphenylenyl group, a phenanthrenyl group, an *as*-indacenyl group, a s-indacenyl group, an acenaphthylenyl group, a phenalenyl group, a fluoranthenyl group, a pyrenyl group, a naphthacenyl group, and a hexacenyl group.

Examples of the specific chemical structure of the "optionally substituted polycyclic aryl group" in the anthranilic acid compound according to the present invention can include, but are not limited to, the following groups:

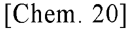

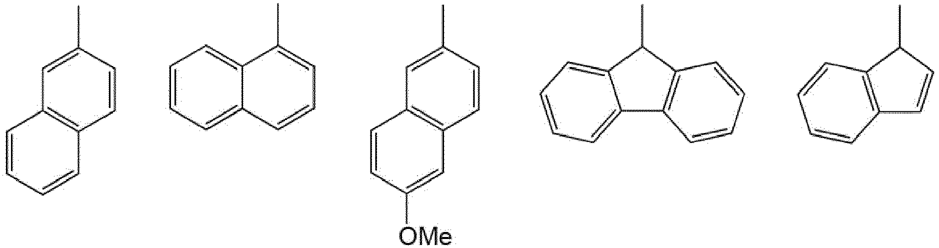

In the anthranilic acid compound according to the present invention, the "optionally substituted polycyclic aryl group" is preferably an optionally substituted naphthyl group. In this respect, the optionally substituted naphthyl group may be attached to the matrix of the compound represented by the Formula (I-I) at position 1 or 2 of the naphthyl group.

This "optionally substituted naphthyl group" can be represented by the following Formula (I-III):

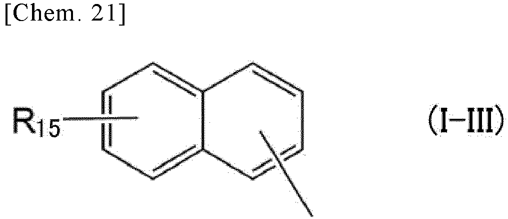

In the Formula (I-III), R₁₅ represents 0 to 7 identical or different substituents attached to a naphthyl group. Although R₁₅ may be provided at any of positions 1 to 8 of the naphthyl group, R₁₅ cannot be provided at a position at which the naphthyl group is attached to the matrix of the compound. Also, R₁₅ may not be provided on the naphthyl group so that the naphthyl group does not have any substituents. If R₁₅ is provided on the naphthyl group, 1 to 7 R₁₅ may be provided, and they may be different substituents from each other, or all or some of them may be the same substituents. R₁₅ is preferably a substituent having 1 to 10 atoms.

In the Formula (I-I), at least one of R₁₁ and R₁₂ can be the optionally substituted polycyclic heterocyclic group. In the anthranilic acid compound according to the present invention, the "polycyclic heterocyclic group" refers to a group containing a condensed ring of two or more rings and consisting of carbon atoms and non-carbon atoms.

It is preferable to use an aromatic heterocyclic ring as the "polycyclic heterocyclic group".

Examples of the "polycyclic heterocyclic group" of the anthranilic acid compound according to the present invention can include, but are not limited to, any of 5- to 14-membered bicyclic to pentacyclic heterocyclic groups having carbon atoms and further having one to four heteroatoms of one or two elements selected from nitrogen, oxygen, and sulfur.

Specific examples of the heterocyclic group can include, but are not limited to, a bicyclic to tetracyclic condensed ring group having carbon atoms and further having one to four heteroatoms selected from oxygen, sulfur, and nitrogen atoms, such as an indolyl group, a benzofuryl group, a benzothiazolyl group, a benzoxazolyl group, a xanthenyl group, a benzimidazolyl group, a quinolyl group, an isoquinolyl group, a phthalazinyl group, a quinazolinyl group, a quinoxalinyl group, an indolizinyl group, a quinolizinyl group, a 1,8-naphthyridinyl group, a dibenzofuranyl group, a carbazolyl group, an acridinyl group, a phenanthridinyl group, a perimidinyl group, a phenazinyl group, a chromanyl group, a phenothiazinyl group, a phenoxazinyl group, and a 7H-pirazino[2,3-c]carbazolyl group.

Examples of the specific chemical structure of the "optionally substituted polycyclic heterocyclic group" in the anthranilic acid compound according to the present invention can include, but are not limited to, the following groups:

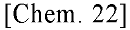

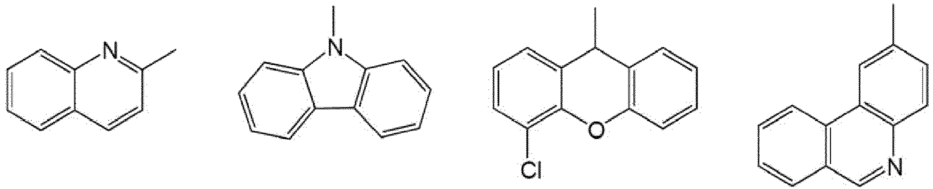

In the Formula (I-I), at least one of R₁₁ and R₁₂ can be a group represented by the following Formula (I-II): 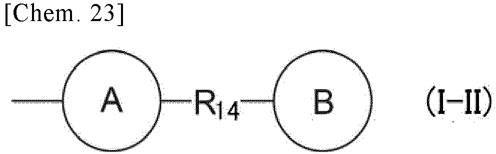 (wherein rings A and B respectively represent an optionally substituted monocyclic or polycyclic aryl group, and R₁₄ represents an optionally substituted alkylene group having 1 to 3 carbon atoms, an optionally substituted alkenylene group having 2 to 3 carbon atoms, or divalent oxy group),

In the anthranilic acid compound according to the present invention, the "monocyclic or polycyclic aryl group" can be, but are not limited to, a phenyl group, an indenyl group, a naphthyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group.

Examples of the "alkylene group having 1 to 3 carbon atoms" in the anthranilic acid compound according to the present invention include, but are not limited to, a methylene group, an ethylene group, a trimethylene group, and the like.

Also, examples of the "alkenylene group having 2 to 3 carbon atoms" in the anthranilic acid compound according to the present invention include, but are not limited to, a vinylene group, a 1-propenylene group, a 2-propenylene group, and the like.

In the Formula (I-II), R₁₄ can be the divalent oxy group, and in this case, the Formula (I-II) can be represented by the following Formula (I-IV): 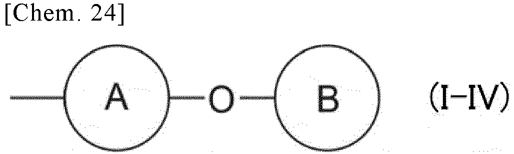 (wherein rings A and B respectively represent an optionally substituted monocyclic or polycyclic aryl group.)

In the Formula (I-I), if neither R₄₁ nor R₄₂ is the optionally substituted polycyclic aryl group, the optionally substituted polycyclic heterocyclic group, and the group represented by the Formula (I-II), R₁₁ or R₁₂ is a hydrogen atom, an optionally substituted hydrocarbon group (except for the optionally substituted polycyclic aryl group), or an optionally substituted monocyclic heterocyclic group, preferably a hydrogen atom or an optionally substituted phenyl group.

In the anthranilic acid compound according to the present invention, the "hydrocarbon group" means a group derived from a compound composed of carbon and hydrogen atoms. Examples of the hydrocarbon group can include, but are not limited to, aliphatic hydrocarbon, monocyclic saturated hydrocarbon, and aromatic hydrocarbon groups, and preferably contain 1 to 16 carbon atoms. Specific examples of the hydrocarbon group include, but are not limited to, an alkyl group, an alkenyl group, an alkynyl group, a cycloalkyl group, and an aryl group.

In this respect, examples of the "alkyl group" include a methyl group, an ethyl group, a propyl group, an isopropyl group, a butyl group, an isobutyl group, a sec-butyl group, a *tert*-butyl group, a pentyl group, and a hexyl group. Examples of the "alkenyl group" include a vinyl group, a 1-propenyl group, an allyl group, an isopropenyl group, a butenyl group, and an isobutenyl group. Examples of the "alkynyl group" include an ethynyl group, a propargyl group, and a 1-propynyl group. Examples of "cycloalkyl group" include a cyclopropyl group, a cyclobutyl group, a cyclopentyl group, and a cyclohexyl group. Examples of "aryl group" include a phenyl group, an indenyl group, a naphthyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group.

In the anthranilic acid compound according to the present invention, the "monocyclic heterocyclic group" can be, but are not limited to, a 5-membered heterocyclic group having carbon atoms and further having one to four heteroatoms selected from oxygen, sulfur, and nitrogen atoms, such as a 2- or 3-thienyl group, a 2- or 3-furyl group, a 1-, 2- or 3-pyrrolyl group, a 1-, 2- or 3-pyrrolidinyl group, a 2-, 4- or 5-oxazolyl group, a 3-, 4- or 5-isooxazolyl group, a 2-, 4- or 5-thiazolyl group, a 3-, 4- or 5-isothiazolyl group, a 3-, 4- or 5-pyrazolyl group, a 2-, 3- or 4-pyrazolidinyl group, a 2-, 4- or 5-imidazolyl group, a 1,2,3-triazolyl group, a 1,2,4-triazolyl group, and a 1H- or 2H-tetrazolyl group. Moreover, the "monocyclic heterocyclic group" can be, but are not limited to a 6-memberd ring group having carbon atoms and further having one to four heteroatoms selected from oxygen, sulfur, and nitrogen atoms, such as a 2-, 3- or 4-pyridyl group, an N-oxide-2-, 3- or 4-pyridyl group, a 2-, 4- or 5-pyrimidinyl group, an N-oxide-2-, 4- or 5-pyrimidinyl group, a thiomorpholinyl group, a morpholinyl group, a piperidino group, a 2-, 3- or 4-piperidyl group, a thiopyranyl group, a 1,4-oxazinyl group, a 1,4-thiazinyl group, a 1,3-thiazinyl group, a piperazinyl group, a triazinyl group, a 3- or 4-pyridazinyl group, a pyrazinyl group, and an N-oxide-3- or 4-pyridazinyl group.

In the Formula (I-I), R₁₃ is a hydrogen atom, an optionally substituted hydrocarbon group or an optionally substituted heterocyclic group. R₁₃ is preferably a hydrogen atom or a methyl group, and more preferably a hydrogen atom.

The anthranilic acid compound according to the present invention has higher activity when R₁₃ is a hydrogen atom and the compound has a carboxyl group. However, when R₁₃ is not a hydrogen atom but an ester body, it can easily become a carboxyl group by hydrolysis so that the compound can exhibit high activity. Therefore, when R₁₃ is the optionally substituted hydrocarbon group or the optionally substituted heterocyclic group, the anthranilic acid compound according to the present invention can also be used as a prodrug.

In the anthranilic acid compound according to the present invention, the "heterocyclic group" refers to a group derived from a cyclic compound composed of atoms of carbon and some other elements. It is preferable to use an aromatic heterocyclic ring as the "heterocyclic group".

In the anthranilic acid compound according to the present invention, the "heterocyclic group" can be, but is not limited to, for example, any of 5- to 14-membered monocyclic to pentacyclic heterocyclic groups each having carbon atoms and further having one to four heteroatoms of one or two elements selected from nitrogen, oxygen, and sulfur. Specific examples of the heterocyclic group can include, but are not limited to, a 5-membered cyclic group having carbon atoms and further having one to four heteroatoms selected from oxygen, sulfur, and nitrogen, such as a 2- or 3-thienyl group, 2- or 3-furyl group, a 1-, 2- or 3-pyrrolyl group, a 1-, 2- or 3-pyrrolidinyl group, a 2-, 4- or 5-oxazolyl group, a 3-, 4- or 5-isooxazolyl group, a 2-, 4- or 5-thiazolyl group, a 3-, 4- or 5-isothiazolyl group, a 3-, 4- or 5-pyrazolyl group, a 2-, 3- or 4-pyrazolidinyl group, a 2-, 4- or 5-imidazolyl group, a 1,2,3-triazolyl group, a 1,2,4-triazolyl group, and a 1H- or 2H-tetrazolyl group. Moreover, specific examples of the heterocyclic group can include, but are not limited to, a 6-membered cyclic group having carbon atoms and further having one to four heteroatoms selected from oxygen, sulfur, and nitrogen, such as a 2-, 3- or 4-pyridyl group, an N-oxide-2-, 3- or 4-pyridyl group, a 2-, 4- or 5-pyrimidinyl group, an N-oxide-2-, 4- or 5-pyrimidinyl group, a thiomorpholinyl group, a morpholinyl group, a piperidino group, a 2-, 3- or 4-piperidyl group, a thiopyranyl group, a 1,4-oxazinyl group, a 1,4-thiazinyl group, a 1,3-thiazinyl group, a piperazinyl group, a triazinyl group, a 3- or 4-pyridazinyl group, a pyrazinyl group, and an N-oxide-3- or 4-pyridazinyl group. Moreover, specific examples of the heterocyclic group can include, but are not limited to, a bicyclic to tetracyclic condensed ring group having carbon atoms and further having one to four heteroatoms selected from oxygen, sulfur, and nitrogen, such as an indolyl group, a benzofuryl group, a benzothiazolyl group, a benzoxazolyl group, a xanthenyl group, a benzimidazolyl group, a quinolyl group, an isoquinolyl group, a phthalazinyl group, a quinazolinyl group, a quinoxalinyl group, an indolizinyl group, a quinolizinyl group, a 1,8-naphthyridinyl group, a dibenzofuranyl group, a carbazolyl group, an acridinyl group, a phenanthridinyl group, a perimidinyl group, a phenazinyl group, a chromanyl group, a phenothiazinyl group, a phenoxazinyl group, and a 7H-pirazino[2,3-c]carbazolyl group.

In the Formula (I-I), X₁ is a single bond, -CO- group, -CO-O-CH₂- group, -CO-CH₂-O- group, -SO₂- group, -CH₂-CO-NH- group, -CH₂-CO- group, -CH₂- group, or - CO-CH₂- group.

X₁ is preferably a single bond or -CO- group (carbonyl group).

When X₁ is a single bond, the Formula (I-I) can be represented by the following Formula (I-V): 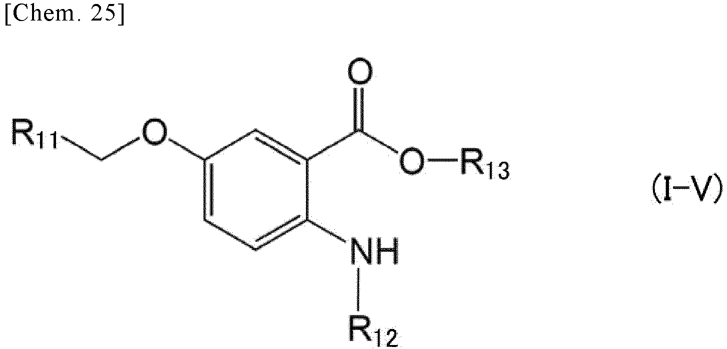 (wherein R₁₁, R₁₂ and R₁₃ are the same as above.)

The "substituent" used with respect to the anthranilic acid compound according to the present invention refers to a halogen (such as, for example, fluorine, chlorine, bromine, or iodine), an alkyl group (for example, a C₁₋₆ alkyl group, such as a methyl group, an ethyl group, a propyl group, an isopropyl group, a butyl group, an isobutyl group, a *sec*-butyl group, a *tert*-butyl group, a pentyl group, or a hexyl group), a cycloalkyl group (for example, a C₃₋₆ cycloalkyl group, such as a cyclopropyl group, a cyclobutyl group, a cyclopentyl group, or a cyclohexyl group), an alkynyl group (for example, a C₂₋₆ alkynyl group, such as an ethynyl group, a 1-propynyl group, or a propargyl group), an alkenyl group (for example, a C₂₋₆ alkenyl group, such as a vinyl group, an allyl group, an isopropenyl group, a butenyl group, or an isobutenyl group), an aralkyl group (for example, a C₇₋₁₁ aralkyl group, such as a benzyl group, an α-methylbenzyl group, or a phenethyl group), an aryl group (for example, a C₆₋₁₀ aryl group, such as a phenyl group or a naphthyl group; preferably a phenyl group), an alkoxy group (for example, a C₁₋₆ alkoxy group, such as a methoxy group, an ethoxy group, a propoxy group, an isopropoxy group, a butoxy group, an isobutoxy group, a *sec*-butoxy group, or a *tert*-butoxy), an aryloxy group (for example, a C₆₋₁₀ aryloxy group, such as phenoxy), an alkanoyl group (for example, a C₁₋₆ alkyl-carbonyl group, such as a formyl group, an acetyl group, a propionyl group, a butyryl group, or an isobutyryl group), an arylcarbonyl group (for example, a C₆₋₁₀ aryl-carbonyl group, such as a benzoyl group or a naphthoyl group), an alkanoyloxy group (for example, a C₁₋₆ alkyl-carbonyloxy group, such as a formyloxy group, an acetyloxy group, a propionyloxy group, a butyryloxy group, or an isobutyryloxy group), an arylcarbonyloxy group (for example, a C₆₋₁₀ aryl-carbonyloxy group, such as a benzoyloxy group or a naphthoyloxy group), carboxyl group, an alkoxycarbonyl group (for example, a C₁₋₆ alkoxy-carbonyl group, such as a methoxycarbonyl group, an ethoxycarbonyl group, a propoxycarbonyl group, an isopropoxycarbonyl group, a butoxycarbonyl group, an isobutoxycarbonyl group, or a *tert*-butoxycarbonyl), an aralkyloxycarbonyl group (for example, a C₇₋₁₁ aralkyloxycarbonyl group, such as a benzyloxycarbonyl group), a carbamoyl group, a halogenated alkyl group (for example, a mono-, di-, or tri-halogenated -C₁₋₄ alkyl group, such as a chloromethyl group, a dichloromethyl group, a trifluoromethyl group, or a 2,2,2-trifluoroethyl group), an oxo group, an amidino group, an imino group, an amino group, an alkylamino group (for example, a mono-C₁₋₄ alkylamino group, such as a methylamino group, an ethylamino group, a propylamino group, an isopropylamino group, or a butylamino group), a dialkylamino group (for example, a di-C₁₋₄ alkylamino group, such as a dimethylamino group, a diethylamino group, a dipropylamino group, a diisopropylamino group, a dibutylamino group, or a methylethylamino group), an alkoxycarbonylamino group (for example, a C₁₋₆ alkoxycarbonylamino group, such as a methoxycarbonylamino group, an isoproxycarbonylamino group, or a *tert-*butoxycarbonylamino group), a cyclic amino group (a 3- to 6-membered cyclic amino group containing carbon atoms and one nitrogen atom and further containing one to three heteroatoms selected from oxygen, sulfur, and nitrogen; such as, for example, an aziridinyl group, an azetidinyl group, a pyrrolidinyl group, a pyrrolinyl group, a pyrrolyl group, an imidazolyl group, a pyrazolyl group, an imidazolidinyl group, a piperidyl group, a morpholinyl group, a dihydropyridyl group, a pyridyl group, an N-methylpiperazinyl group, or an N-ethylpiperazinyl group), an alkylenedioxy group (for example, a C₁₋₃ alkylenedioxy group, such as a methylenedioxy group or an ethylenedioxy group), a hydroxy group, a cyano group, a mercapto group, a sulfo group, a sulfino group, a phosphono group, a sulfamoyl group, a monoalkylsulfamoyl group (for example, a mono-C₁₋₆ alkylsulfamoyl group, such as N-methylsulfamoyl, N-ethylsulfamoyl, N-propylsulfamoyl, N-isopropylsulfamoyl, or N-butylsulfamoyl), a dialkylsulfamoyl group (for example, a di-C₁₋₆ alkylsulfamoyl group, such as an N,N-dimethylsulfamoyl group, an N,N-diethylsulfamoyl group, an N,N-dipropylsulfamoyl group, or an N,N-dibutylsulfamoyl group), an alkylthio group (for example, a C₁₋₆ alkylthio group, such as a methylthio group, an ethylthio group, propylthio group, an isopropylthio group, a butylthio group, a *sec*-butylthio group, or a *tert*-butylthio group), an arylthio group (for example, a C₆₋₁₀ arylthio group, such as a phenylthio group or a naphthylthio group), an alkylsulfinyl group (for example, a C₁₋₆ alkylsulfinyl group, such as a methylsulfinyl group, an ethylsulfinyl group, a propylsulfinyl group, or a butylsulfinyl group), an alkylsulfonyl group (for example, a C₁₋₆ alkylsulfonyl group, such as a methylsulfonyl group, an ethylsulfonyl group, a propylsulfonyl group, or a butylsulfonyl group), or an arylsulfonyl group (for example, a C₆₋₁₀ arylsulfonyl group, such as a phenylsulfonyl group or a naphthylsulfonyl group).

In the anthranilic acid compound according to the present invention, the "substituent containing 1 to 10 atoms" used in R₁₅ is those which contain 1 to 10 atoms among the aforementioned substituents. Examples that can be used as such a substituent include, but are not limited thereto, a halogen atom, a methyl group, an ethyl group, a vinyl group, a methoxy group, an ethoxy group, an acetyl group, a carboxyl group, a methoxycarbonyl group, a chloromethyl group, an amino group, a methylamino group, a hydroxy group, a sulfo group, and a methylthio group.

In the anthranilic acid compound according to the present invention, the phrase "optionally substituted" means that a substituent as described above is present or absent. In cases where a moiety is substituted, two or more substituents may be present within the moiety, and the substituents may be identical to or different from each other. In cases where the anthranilic acid compound according to the present invention is "optionally substituted," the number of substituents within the compound is preferably from 0 to 3.

The anthranilic acid compound can be synthesized using, for example, a method described in WO 2019/031472, although it is not limited thereto.

### 1-2. Amide Compound

Another one of the compounds that are active ingredients of the therapeutic or prophylactic agents for coronavirus infection according to present invention is a compound that has a chemical structure represented by the following Formula (II-I) and inhibitory activity against the function of Pin1:

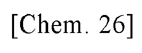

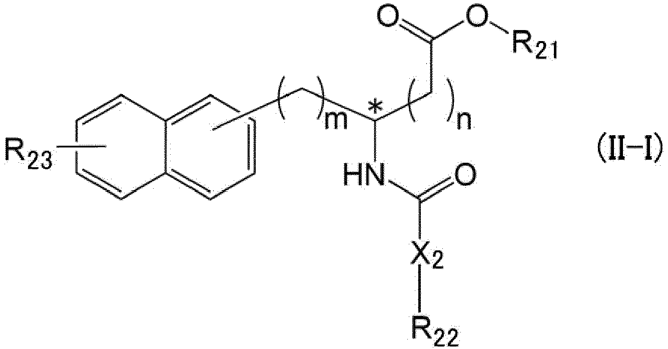

In the Formula (II-I), m represents an integer from 0 to 2, n represents an integer from 0 to 1, and m and n are integers satisfying the relationship 0≤m+n≤2. That is, there are five combinations of (m, n): (0,0), (0,1), (1,0), (1,1), and (2,0).

The amide compound according to the present invention consists of a naphthyl group portion and a chain portion attached thereto. The chain portion can have structures in which the chain portion is attached to any part of the naphthyl group, and these structures can be classified into two structures: a structure in which the chain portion is attached to position 1 of the naphthyl group, and a structure in which the chain portion is attached to position 2 of the naphthyl group.

When the chain portion is attached to position 2 of the naphthyl group, the amide compound according to the present invention can have the five chemical structures represented by the following Formulae (II-VI) to (II-X) on the basis of the five combinations of (m, n):

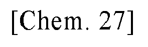

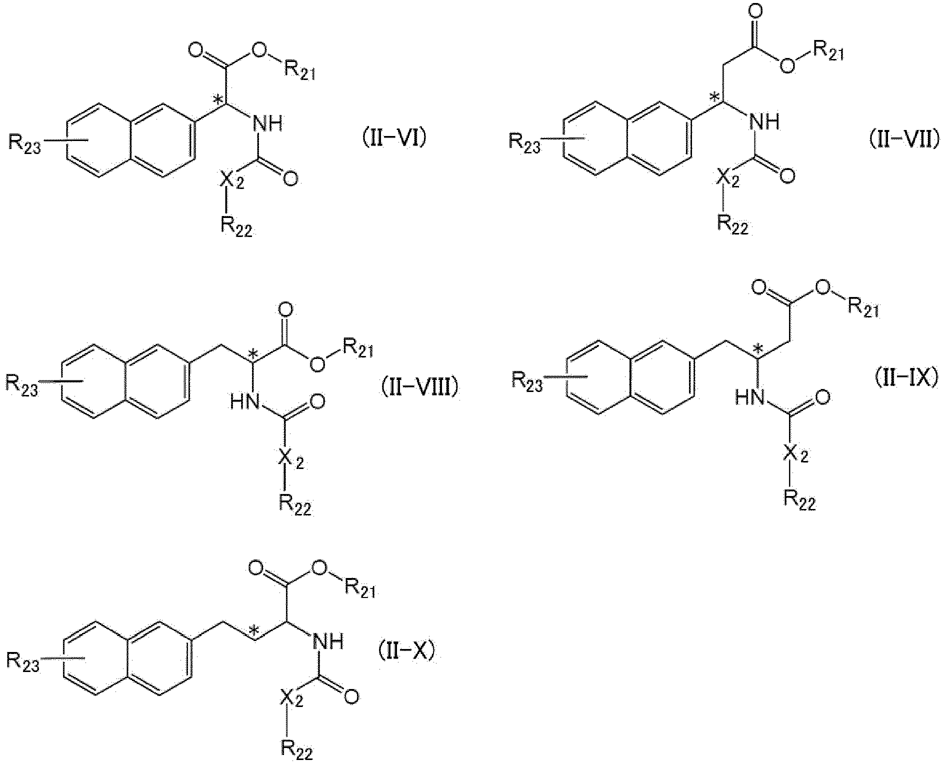

The Formula (II-VI) represents a formula when m=0 and n=0 in the Formula (II-I).

The Formula (II-VII) represents a formula when m=0 and n=1 in the Formula (II-I).

The Formula (II-VIII) represents a formula when m=1 and n=0 in the Formula (II-I).

The Formula (II-IX) represents a formula when m=1 and n=1 in the Formula (II-I).

The Formula (II-X) represents a formula when m=2 and n=0 in the Formula (II-I).

As the amide compound according to the present invention, a compound represented by the Formula (II-VI) to (II-X) is preferable in which the chain portion is attached to position 2 of the naphthyl group, and a compound represented by the Formula (II-VIII) is particularly preferable in which m=1 and n=0.

When the chain portion is attached to position 1 of the naphthyl group, the amide compound according to the present invention can have the five chemical structures represented by the following Formulae (II-XI) to (II-XV) on the basis of the five combinations of (m, n):

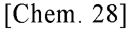

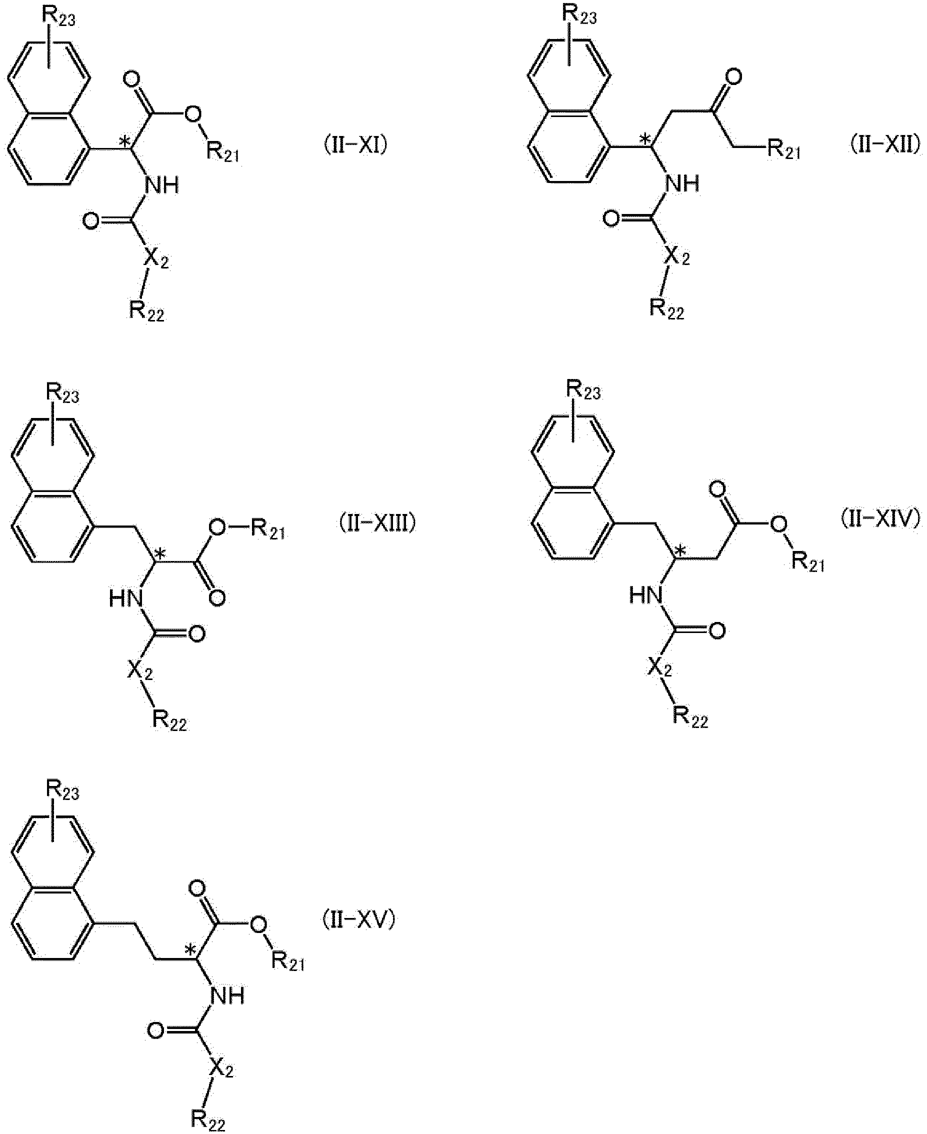

In the Formula (II-I) representing the amide compound according to the present invention, R₂₁ is a hydrogen atom, an optionally substituted hydrocarbon group, an optionally substituted heterocyclic group, or an optionally substituted amino group.

The amide compound according to the present invention has inhibitory activity against the function of Pin1 when R₂₁ is a hydrogen atom to form a carboxyl group. Therefore, R₂₁ is preferably the hydrogen atom.

However, when R₂₁ is the optionally substituted hydrocarbon group, the optionally substituted heterocyclic group, or the optionally substituted amino group, the compound represented by the Formula (II-I) forms an ester bond at R₂₁ so that the compound can be converted to a carboxyl group by hydrolysis. Therefore, even if R₂₁ is the optionally substituted hydrocarbon group, the optionally substituted heterocyclic group, or the optionally substituted amino group, the amide compound according to the present invention can be used as a prodrug.

In the amide compound according to the present invention, the "hydrocarbon group" used in R₂₁ in the Formula (II-I) and the like means a group derived from a compound composed of carbon and hydrogen atoms. Examples of the hydrocarbon group can include, but are not limited to, aliphatic hydrocarbon, monocyclic saturated hydrocarbon, and aromatic hydrocarbon groups, and preferably contain 1 to 16 carbon atoms. Specific examples of the hydrocarbon group include, but are not limited to, an alkyl group, an alkenyl group, an alkynyl group, a cycloalkyl group, an aryl group, and an aralkyl group.

In this respect, examples of the "alkyl group" include a methyl group, an ethyl group, a propyl group, an isopropyl group, a butyl group, an isobutyl group, a sec-butyl group, a *tert*-butyl group, a pentyl group, and a hexyl group. Examples of the "alkenyl group" include a vinyl group, a 1-propenyl group, an a llyl group, an isopropenyl group, a butenyl group, and an isobutenyl group. Examples of the "alkynyl group" include an ethynyl group, a propargyl group, and a 1-propynyl group. Examples of "cycloalkyl group" include a cyclopropyl group, a cyclobutyl group, a cyclopentyl group, and a cyclohexyl group. Examples of "aryl group" include a phenyl group, an indenyl group, a naphthyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group. Examples of "aralkyl group" include a benzyl group, a styryl group, and a phenethyl group.

In the amide compound according to the present invention, the "heterocyclic group" used in R₂₁ in the Formula (II-I) and the like refers to a group derived from a cyclic compound composed of atoms of carbon and some other elements. It is preferable to use an aromatic heterocyclic ring as the "heterocyclic group".

In the amide compound according to the present invention, the "heterocyclic group" can be, but is not limited to, for example, any of 5- to 14-membered monocyclic to pentacyclic heterocyclic groups each having carbon atoms and further having one to four heteroatoms of one or two elements selected from nitrogen, oxygen, and sulfur. Specific examples of the heterocyclic group can include, but are not limited to, a 5-membered cyclic group having carbon atoms and further having one to four heteroatoms selected from oxygen, sulfur, and nitrogen, such as a 2- or 3-thienyl group, 2- or 3-furyl group, a 1-, 2- or 3-pyrrolyl group, a 1-, 2- or 3-pyrrolidinyl group, a 2-, 4- or 5-oxazolyl group, a 3-, 4- or 5-isooxazolyl group, a 2-, 4- or 5-thiazolyl group, a 3-, 4- or 5-isothiazolyl group, a 3-, 4- or 5-pyrazolyl group, a 2-, 3- or 4-pyrazolidinyl group, a 2-, 4- or 5-imidazolyl group, a 1,2,3-triazolyl group, a 1,2,4-triazolyl group, and a 1H- or 2H-tetrazolyl group. Moreover, specific examples of the heterocyclic group can include, but are not limited to, a 6-membered cyclic group having carbon atoms and further having one to four heteroatoms selected from oxygen, sulfur, and nitrogen, such as a 2-, 3- or 4-pyridyl group, an N-oxide-2-, 3- or 4-pyridyl group, a 2-, 4- or 5-pyrimidinyl group, an N-oxide-2-, 4- or 5-pyrimidinyl group, a thiomorpholinyl group, a morpholinyl group, a piperidino group, a 2-, 3- or 4-piperidyl group, a thiopyranyl group, a 1,4-oxazinyl group, a 1,4-thiazinyl group, a 1,3-thiazinyl group, a piperazinyl group, a triazinyl group, a 3- or 4-pyridazinyl group, a pyrazinyl group, and an N-oxide-3- or 4-pyridazinyl group. Moreover, specific examples of the heterocyclic group can include, but are not limited to, a bicyclic to tetracyclic condensed ring group having carbon atoms and further having one to four heteroatoms selected from oxygen, sulfur, and nitrogen, such as an indolyl group, a benzofuryl group, a benzothiazolyl group, a benzoxazolyl group, a xanthenyl group, a benzimidazolyl group, a quinolyl group, an isoquinolyl group, a phthalazinyl group, a quinazolinyl group, a quinoxalinyl group, an indolizinyl group, a quinolizinyl group, a 1,8-naphthyridinyl group, a dibenzofuranyl group, a carbazolyl group, an acridinyl group, a phenanthridinyl group, a perimidinyl group, a phenazinyl group, a chromanyl group, a phenothiazinyl group, a phenoxazinyl group, and a 7H-pirazino[2,3-c]carbazolyl group.

In the amide compound according to the present invention, the "optionally substituted amino group" used for R₂₁ in the Formula (II-I) and the like is a primary amino group, a secondary amino group, or a tertiary amino group. The primary amino group is -NH₂ group. The secondary amino group is an amino group having one substituent, and may include, but are not limited to, for example, alkylamino group, arylamino group, alkoxycarbonylamino group and the like. The tertiary amino group is an amino group having identical or different two substituents, and may include, but not limited to, for example, dialkylamino group, diarylamino group and the like.

In the Formula (II-I) representing the amide compound according to the present invention, R₂₂ is an optionally substituted aryl group containing a condensed ring of three or more rings, a substituted naphthyl group, or a group represented by the Formula (II), (III), (IV), or (V).

It is believed that especially the naphthyl group portion, the carbonic acid portion, and the R₂₂ portion involve the inhibitory activity against the function of Pin1 of the amide compound according to the present invention. The R₂₂ portion can have a wide variety of chemical structures with an aromatic ring or a heterocycle.

In the amide compound according to the present invention, the "aryl group containing a condensed ring of three or more rings" refers to a group derived from aromatic compounds containing a condensed ring of three or more rings consisting only of carbon.

The "aryl group containing a condensed ring of three or more rings" is preferably a tricyclic aryl group.

The "aryl group containing a condensed ring of three or more rings" in the amide compound according to the present invention can be, but are not limited to, a fluorenyl group, an anthryl group, a biphenylenyl group, an indacenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group.

Examples of the specific chemical structure of the "optionally substituted aryl group containing a condensed ring of three or more rings" in the amide compound according to the present invention can include, but are not limited to, the following groups:

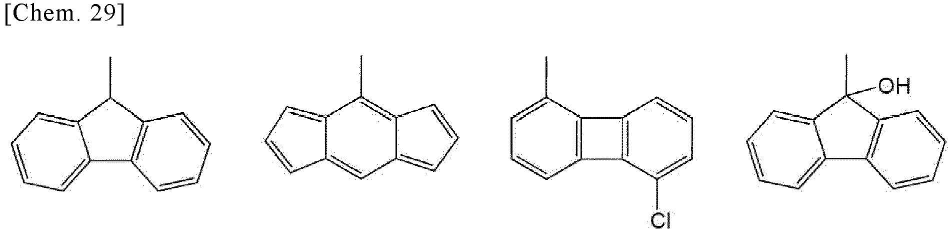

R₂₂ in the Formula (II-I) can be a substituted naphthyl group such as but not limited to 6-methoxycarbonyl-2- naphthyl group and 5-methyl-1- naphthyl group.

R₂₂ in the Formula (II-I) can be a group represented by the Formula (II-II) with the following structure: 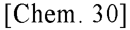 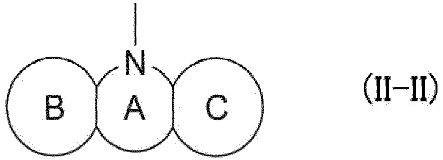 (wherein a ring A represents an optionally substituted monocyclic heterocyclic group, and rings B and C respectively represent an optionally substituted monocyclic or polycyclic aryl group or heterocyclic group, and the rings A, B and C form a condensed ring.)

In the Formula (II-II), rings B and C are preferably a benzene ring.

Examples of the specific chemical structure of the compound represented by the Formula (II-II) can include, but are not limited to, the following groups:

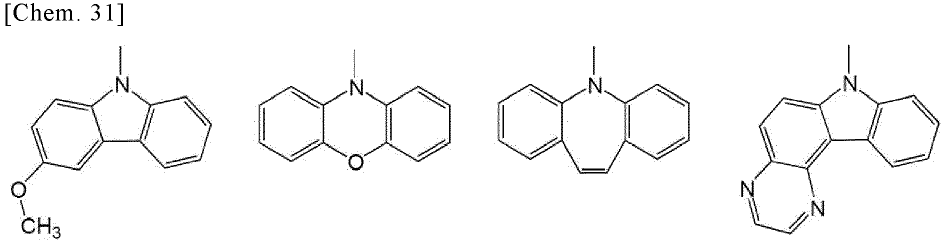

R₂₂ in the Formula (II-I) can be a group represented by the Formula (II-III) with the following structure: 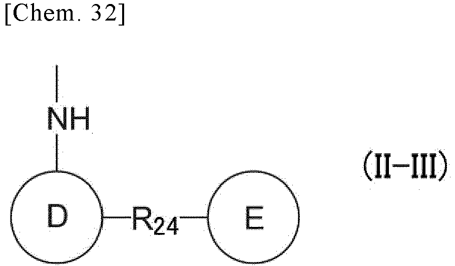 (wherein rings D and E respectively represent an optionally substituted monocyclic or polycyclic aryl group or heterocyclic group, and R₂₄ represents a divalent oxy group or a carbonyl group.)

Examples of the specific chemical structure of the compound represented by the Formula (II-III) can include, but are not limited to, the following groups:

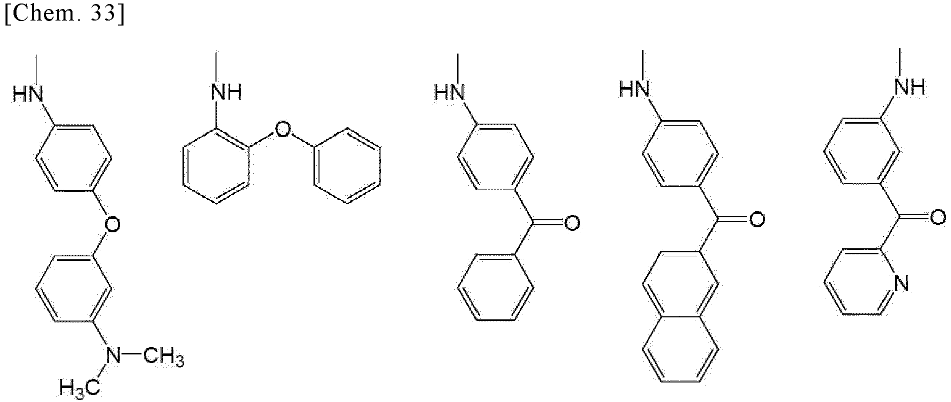

R₂₄ in the Formula (II-I) can be a group represented by the Formula (II-IV) with the following structure: 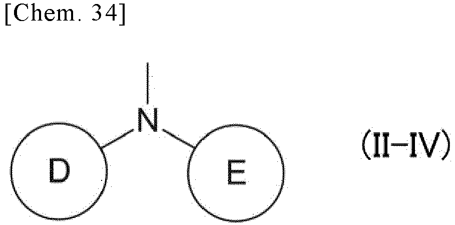 (wherein rings D and E respectively represent an optionally substituted monocyclic or polycyclic aryl group or heterocyclic group.)

Examples of the specific chemical structure of the compound represented by the Formula (II-IV) can include, but are not limited to, the following groups:

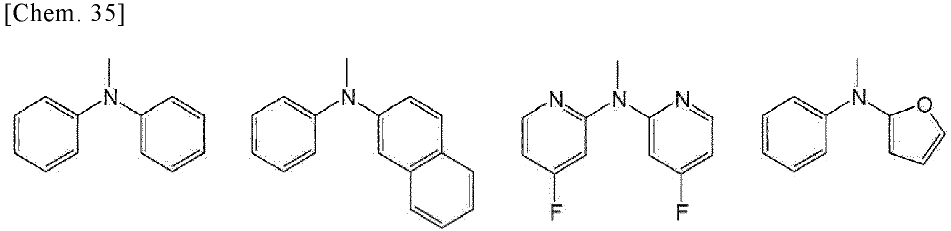

R₂₂ in the Formula (II-I) can be a group represented by the Formula (II-V) with the following structure: 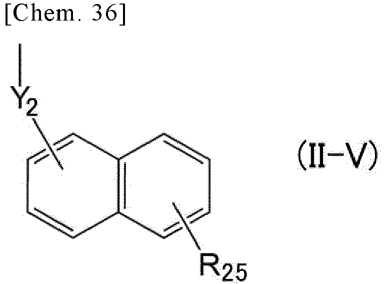 (wherein R₂₅ represents 0 to 7 identical or different substituents attached to a naphthyl group, and Y₂ represents -NH- group or an alkylene group having 1 or 2 carbon atoms.)

Examples of the specific chemical structure of the compound represented by the Formula (II-V) can include, but are not limited to, the following groups:

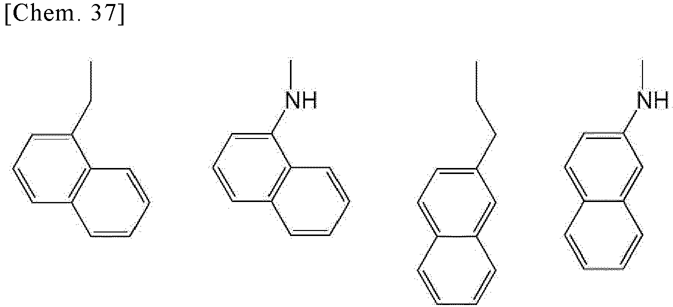

R₂₂ in the Formula (II-I) representing the amide compound according to the present invention is preferably a group represented by the Formula (II-II), the Formula (II-III), or the Formula (II-IV). More preferably, R₂₂ is a group represented by the Formula (II-II).

In the Formula (II-I) representing the amide compound according to the present invention, R₂₃ is 0 to 7 identical or different substituents attached to a naphthyl group.

Although R₂₃ may be provided at any of positions 1 to 8 of the naphthyl group, R₂₃ cannot be provided at a position at which the chain portion of the amide compound according to the present invention is attached to the naphthyl group. Also, R₂₃ may be omitted on the naphthyl group so that the naphthyl group does not have any substituents. If R₂₃ is provided on the naphthyl group, 1 to 7 substituents of R₂₃ may be provided, and they may be different substituents from each other, or all or some of them may be the same substituents. R₂₃ is preferably a substituent having 1 to 10 atoms.

In the Formula (II-I) representing the amide compound according to the present invention, X₂ is a single bond, -CH₂- group, or -NH- group.

X₂ is preferably a single bond.

When X₂ is a single bond, the Formula (II-I) can be represented by the following Formula (II-XVI): 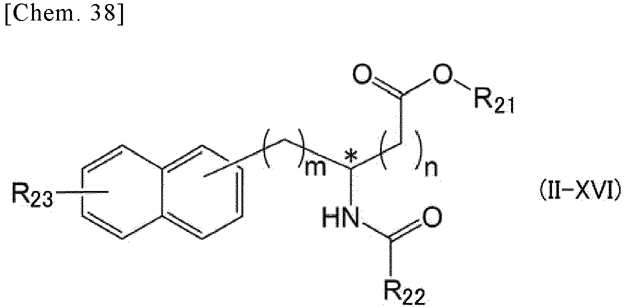 (wherein m, n, R₂₁, R₂₂ and R₂₃ are the same as above.)

The "substituent" used with respect to the amide compound according to the present invention refers to a halogen (such as, for example, fluorine, chlorine, bromine, or iodine), an alkyl group (for example, a C₁₋₆ alkyl group, such as a methyl group, an ethyl group, a propyl group, an isopropyl group, a butyl group, an isobutyl group, a *sec-*butyl group, a *tert*-butyl group, a pentyl group, or a hexyl group), a cycloalkyl group (for example, a C₃₋₆ cycloalkyl group, such as a cyclopropyl group, a cyclobutyl group, a cyclopentyl group, or a cyclohexyl group), an alkynyl group (for example, a C₂₋₆ alkynyl group, such as an ethynyl group, a 1-propynyl group, or a propargyl group), an alkenyl group (for example, a C₂₋₆ alkenyl group, such as a vinyl group, an allyl group, an isopropenyl group, a butenyl group, or an isobutenyl group), an aralkyl group (for example, a C₇₋₁₁ aralkyl group, such as a benzyl group, an α-methylbenzyl group, or a phenethyl group), an aryl group (for example, a C₆₋₁₀ aryl group, such as a phenyl group or a naphthyl group; preferably a phenyl group), an alkoxy group (for example, a C₁₋₆ alkoxy group, such as a methoxy group, an ethoxy group, a propoxy group, an isopropoxy group, a butoxy group, an isobutoxy group, a *sec*-butoxy group, or a *tert-*butoxy), an aryloxy group (for example, a C₆₋₁₀ aryloxy group, such as phenoxy), an alkanoyl group (for example, a C₁₋₆ alkyl-carbonyl group, such as a formyl group, an acetyl group, a propionyl group, a butyryl group, or an isobutyryl group), an arylcarbonyl group (for example, a C₆₋₁₀ aryl-carbonyl group, such as a benzoyl group or a naphthoyl group), an alkanoyloxy group (for example, a C₁₋₆ alkyl-carbonyloxy group, such as a formyloxy group, an acetyloxy group, a propionyloxy group, a butyryloxy group, or an isobutyryloxy group), an arylcarbonyloxy group (for example, a C₆₋₁₀ aryl-carbonyloxy group, such as a benzoyloxy group or a naphthoyloxy group), carboxyl group, an alkoxycarbonyl group (for example, a C₁₋₆ alkoxy-carbonyl group, such as a methoxycarbonyl group, an ethoxycarbonyl group, a propoxycarbonyl group, an isopropoxycarbonyl group, a butoxycarbonyl group, an isobutoxycarbonyl group, or a *tert*-butoxycarbonyl), an aralkyloxycarbonyl group (for example, a C₇₋₁₁ aralkyloxycarbonyl group, such as a benzyloxycarbonyl group), a carbamoyl group, a halogenated alkyl group (for example, a mono-, di-, or tri-halogenated -C₁₋₄ alkyl group, such as a chloromethyl group, a dichloromethyl group, a trifluoromethyl group, or a 2,2,2-trifluoroethyl group), an oxo group, an amidino group, an imino group, an amino group, an alkylamino group (for example, a mono-C₁₋₄ alkylamino group, such as a methylamino group, an ethylamino group, a propylamino group, an isopropylamino group, or a butylamino group), a dialkylamino group (for example, a di-C₁₋₄ alkylamino group, such as a dimethylamino group, a diethylamino group, a dipropylamino group, a diisopropylamino group, a dibutylamino group, or a methylethylamino group), an alkoxycarbonylamino group (for example, a C₁₋₆ alkoxycarbonylamino group, such as a methoxycarbonylamino group, an isoproxycarbonylamino group, or a *tert-*butoxycarbonylamino group), a cyclic amino group (a 3- to 6-membered cyclic amino group containing carbon atoms and one nitrogen atom and further containing one to three heteroatoms selected from oxygen, sulfur, and nitrogen; such as, for example, an aziridinyl group, an azetidinyl group, a pyrrolidinyl group, a pyrrolinyl group, a pyrrolyl group, an imidazolyl group, a pyrazolyl group, an imidazolidinyl group, a piperidyl group, a morpholinyl group, a dihydropyridyl group, a pyridyl group, an N-methylpiperazinyl group, or an N-ethylpiperazinyl group), an alkylenedioxy group (for example, a C₁₋₃ alkylenedioxy group, such as a methylenedioxy group or an ethylenedioxy group), a hydroxy group, a cyano group, a mercapto group, a sulfo group, a sulfino group, a phosphono group, a sulfamoyl group, a monoalkylsulfamoyl group (for example, a mono-C₁₋₆ alkylsulfamoyl group, such as N-methylsulfamoyl, N-ethylsulfamoyl, N-propylsulfamoyl, N-isopropylsulfamoyl, or N-butylsulfamoyl), a dialkylsulfamoyl group (for example, a di-C₁₋₆ alkylsulfamoyl group, such as an N,N-dimethylsulfamoyl group, an N,N-diethylsulfamoyl group, an N,N-dipropylsulfamoyl group, or an N,N-dibutylsulfamoyl group), an alkylthio group (for example, a C₁₋₆ alkylthio group, such as a methylthio group, an ethylthio group, propylthio group, an isopropylthio group, a butylthio group, a *sec*-butylthio group, or a *tert*-butylthio group), an arylthio group (for example, a C₆₋₁₀ arylthio group, such as a phenylthio group or a naphthylthio group), an alkylsulfinyl group (for example, a C₁₋₆ alkylsulfinyl group, such as a methylsulfinyl group, an ethylsulfinyl group, a propylsulfinyl group, or a butylsulfinyl group), an alkylsulfonyl group (for example, a C₁₋₆ alkylsulfonyl group, such as a methylsulfonyl group, an ethylsulfonyl group, a propylsulfonyl group, or a butylsulfonyl group), or an arylsulfonyl group (for example, a C₆₋₁₀ arylsulfonyl group, such as a phenylsulfonyl group or a naphthylsulfonyl group).

In the amide compound according to the present invention, if the "substituents" have 1 to 10 atoms, the substituents are those which contain 1 to 10 atoms among the aforementioned substituents. Examples that can be used as such a substituent include, but are not limited thereto, a halogen atom, a methyl group, an ethyl group, a vinyl group, a methoxy group, an ethoxy group, an acetyl group, a carboxyl group, a methoxycarbonyl group, a chloromethyl group, a primary amino group, a methylamino group, a dimethylamino group, a hydroxy group, a sulfo group, and a methylthio group.

In the amide compound according to the present invention, the phrase "optionally substituted" means that a substituent as described above is present or absent. In cases where a moiety is substituted, two or more substituents may be present within the moiety, and the substituents may be identical to or different from each other. In cases where the amide compound according to the present invention is "optionally substituted," the number of substituents within the compound is preferably from 0 to 3.

The amide compound can be synthesized using, for example, a method described in WO 2019/031470, although it is not limited thereto.

### 1-3. Carboxylic-Acid-Derivative Compound

Another one of the compounds that are active ingredients of the therapeutic or prophylactic agents for coronavirus infection according to present invention is a compound that has a chemical structure represented by the following Formula (III-I) and inhibitory activity against the function of Pin1:

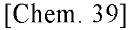

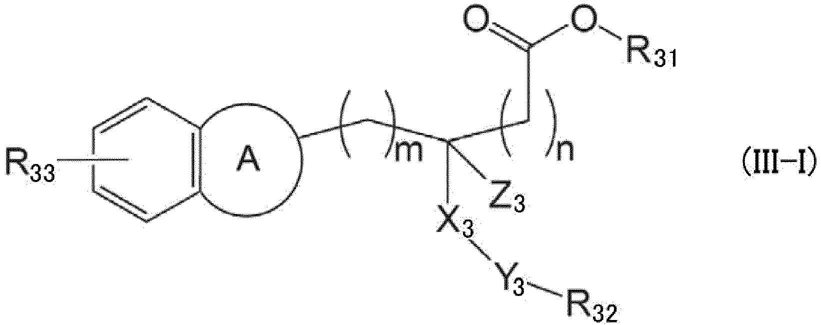

In the Formula (III-I), m represents an integer from 0 to 2, n represents an integer from 0 to 2, and m and n are integers satisfying the relationship 0≤m+n≤3. That is, there are eight combinations of (m, n): (0,0), (0,1), (0,2), (1,0), (1,1), (1,2), (2,0), and (2,1).

The compound that is an active ingredient of the carboxylic-acid-derivative compound according to the present invention can have eight chemical structures on the basis of eight combinations of (m, n).

In the compound that is an active ingredient of the carboxylic-acid-derivative compound according to the present invention, it is preferable that m=1 and n=0.

In the Formula (III-I), a ring A is an optionally substituted monocyclic aromatic ring or an optionally substituted monocyclic heterocycle.

In the carboxylic-acid-derivative compound according to the present invention, the "monocyclic aromatic ring" is a single ring formed by unsaturated organic compounds consisting of carbon and hydrogen, examples of which include, but are not limited to, a benzene ring and a cyclopentadiene ring.

In the carboxylic-acid-derivative compound according to the present invention, the "monocyclic heterocycle" is a single ring formed by unsaturated organic compounds consisting of carbon, hydrogen and other atoms, examples of which include, but are not limited to, a pyrrole ring, an imidazole ring, a pyrazole ring, a furan ring, a thiophene ring, an isothiazole ring, an isoxazole ring, a pyrizine ring, a pyrazine ring, a pyrimidine ring, a pyridazine ring, a pyran ring, and a pyrroline ring.

The ring A and an adjacent benzene ring form a condensed ring so as to form a group containing a bicyclic condensed ring. Examples of the group containing the bicyclic condensed ring include, but are not limited, to a naphthyl group, an indenyl group, an indolyl group, a benzimidazolyl group, an isoindolyl group, a 3H-indolyl group, a 1H-indazolyl group, an isobenzofuranyl group, a quinolyl group, an isoquinolyl group, a phthalazinyl group, a quinoxalinyl group, a quinazolinyl group, a cinolinyl group, and chromenyl group, each of which is optionally substituted.

Among the above-described groups, the optionally substituted naphthyl group, the optionally substituted indolyl group, or the optionally substituted benzimidazolyl group is preferable for the bicyclic condensed ring. It is more preferable that the bicyclic condensed ring is a substituted naphthyl group, and in this case, the ring A is a benzene ring.

The compound that is an active ingredient of the carboxylic-acid-derivative compound according to the present invention has a structure which can be divided into two major portions: a ring portion consisting of a group containing a bicyclic condensed ring having a benzene ring and the ring A; and a chain portion attached thereto.

When the group containing the bicyclic condensed ring (the ring portion) is the optionally substituted naphthyl group, the ring portion can be attached to the chain portion at position 1 or 2 of the naphthyl group. Preferably, the ring portion is attached to chain portion at position 2 of the naphthyl group.

When the group containing the bicyclic condensed ring (the ring portion) is the optionally substituted indolyl group, the ring portion can be attached to the chain portion at position 1, 2 or 3 of the indolyl group. Preferably, the ring portion is attached to chain portion at position 2 or 3 of the indolyl group.

When the group containing the bicyclic condensed ring (the ring portion) is the optionally substituted benzimidazolyl group, the ring portion can be attached to the chain portion at position 1 or 2 of the benzimidazolyl group. Preferably, the ring portion is attached to chain portion at position 2 of the benzimidazolyl group.

Examples of the specific chemical structure of the group containing the bicyclic condensed ring (the ring portion) can include, but are not limited to, the following groups:

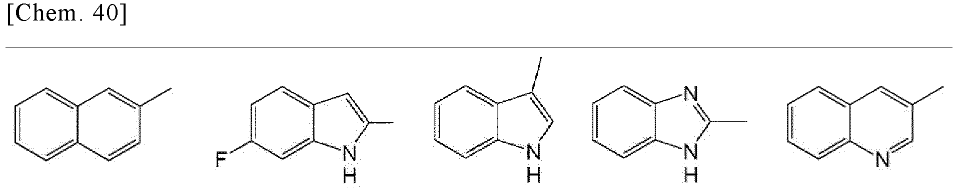

In the Formula (III-I), X₃ is a single bond, -NH- group, -NH-CO- group, -O-CO- group, -CH₂-S-CH₂- group, -CH₂-S-(CH₂)₂-S- group, or -NH-R₃₄- group (R₃₄ is an alkylene group having 1 to 5 carbon atoms, or an alkenylene group having 2 to 5 carbon atoms).

All of these groups are divalent groups which may be attached to Y₃ on either side. For Example, when X₃ is -NH-CO-, either the CO- side or the -NH side may be attached to Y₃.

Examples of the "alkylene group having 1 to 5 carbon atoms" in the carboxylic-acid-derivative compound according to the present invention include, but are not limited to, a linear or branched alkylene group such as a methylene group, an ethylene group, a trimethylene group, a 2-methyltrimethylene group, and a pentamethylene group.

In addition, examples of the "alkenylene group having 2 to 5 carbon atoms" in the carboxylic-acid-derivative compound according to the present invention include, but are not limited to, a linear or branched alkenylene group having 1 to 3 double bond and 2 to 5 carbon atoms, such as a vinylene group, a 1-propenylene group, a 1-methyl-1-propenylene group, a 2-methyl-1-propenylene group, a 2-butenylene group, a 2-pentenylene group, a 1,3-butadienylene group, and a 3-dimethyl-1-propenylene group.

In the Formula (III-I), Y₃ is a single bond, -NH- group, -CO- group, -SO₂- group, -O-CO- group, -CO-NR₃₅- group (R₃₅ represents a hydrogen atom, or an optionally substituted alkyl group having 1 to 5 carbon atoms), -O-R₃₆- group (R₃₆ represents an optionally substituted alkylene group having 1 to 5 carbon atoms, or an optionally substituted alkenylene group having 2 to 5 carbon atoms), -NR₃₅-R₃₆- group, -S-R₃₆-group, -CO-R₃₆- group, -SO₂-R₃₆- group, -NR₃₅-CO-R₃₆- group, -R₃₆-NR₃₅-CO- group, an optionally substituted alkylene group having 1 to 6 carbon atoms, or an optionally substituted alkenylene group having 2 to 6 carbon atoms.

All of these groups are divalent groups which may be attached to R₃₂ on either side. For Example, when Y₃ is -O-CO- group, either the CO- side or the -O side may be attached to R₃₂.

Examples of the "alkylene group having 1 to 6 carbon atoms" in the carboxylic-acid-derivative compound according to the present invention include, but are not limited to, a linear or branched alkylene group such as a methylene group, an ethylene group, a trimethylene group, a 2-methyltrimethylene group, a pentamethylene group, a hexamethylene group, and a 3-methylpentamethylene group.

In addition, examples of the "alkenylene group having 2 to 6 carbon atoms" in the carboxylic-acid-derivative compound according to the present invention include, but are not limited to, a linear or branched alkenylene group having 1 to 3 double bond and 2 to 6 carbon atoms, such as a vinylene group, a 1-propenylene group, a 1-methyl-1-propenylene group, a 2-methyl-1-propenylene group, a 2-butenylene group, a 2-pentenylene group, a 1,3-butadienylene group, a 3-dimethyl-1-propenylene group, a 1, 4-hexadienylene group, and a 4-methyl-3-pentenylene group.

In the Formula (III-I), R₃₁ is a hydrogen atom, an optionally substituted hydrocarbon group, an optionally substituted heterocyclic group, or an optionally substituted amino group.

The carboxylic-acid-derivative compound according to the present invention has inhibitory activity against the function of Pin1 when R₃₁ is the hydrogen atom and the compound is a carboxyl group. Therefore, R₃₁ is preferably the hydrogen atom.

However, when R₃₁ is the optionally substituted hydrocarbon group, the optionally substituted heterocyclic group, or the optionally substituted amino group, the compound represented by the Formula (III-I) forms an ester bond at R₃₁ so that the compound can be converted to a carboxyl group by hydrolysis. Therefore, even if R₃₁ is the optionally substituted hydrocarbon group, the optionally substituted heterocyclic group, or the optionally substituted amino group, the compounds that are active ingredients of the carboxylic-acid-derivative compound according to the present invention can be used as a prodrug.

In the carboxylic-acid-derivative compound according to the present invention, the "hydrocarbon group" used in R₃₁ in the Formula (III-I) and the like means a group derived from a compound composed of carbon and hydrogen atoms. Examples of the hydrocarbon group can include, but are not limited to, aliphatic hydrocarbon, monocyclic saturated hydrocarbon, and aromatic hydrocarbon groups, and preferably contain 1 to 16 carbon atoms. Specific examples of the hydrocarbon group include, but are not limited to, an alkyl group, an alkenyl group, an alkynyl group, a cycloalkyl group, an aryl group, and an aralkyl group.

In this respect, examples of the "alkyl group" include a methyl group, an ethyl group, a propyl group, an isopropyl group, a butyl group, an isobutyl group, a *sec*-butyl group, a *tert*-butyl group, a pentyl group, and a hexyl group. Examples of the "alkenyl group" include a vinyl group, a 1-propenyl group, an a llyl group, an isopropenyl group, a butenyl group, and an isobutenyl group. Examples of the "alkynyl group" include an ethynyl group, a propargyl group, and a 1-propynyl group. Examples of "cycloalkyl group" include a cyclopropyl group, a cyclobutyl group, a cyclopentyl group, and a cyclohexyl group. Examples of "aryl group" include a phenyl group, an indenyl group, a naphthyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group. Examples of "aralkyl group" include a benzyl group, a styryl group, and a phenethyl group.

In the carboxylic-acid-derivative compound according to the present invention, the "heterocyclic group" used in R₃₁ in the Formula (III-I) and the like refers to a group derived from a cyclic compound composed of atoms of carbon and some other elements. It is preferable to use an aromatic heterocyclic ring as the "heterocyclic group".

In the carboxylic-acid-derivative compound according to the present invention, the "heterocyclic group" can be, but is not limited to, for example, any of 5- to 14-membered monocyclic to pentacyclic heterocyclic groups each having carbon atoms and further having one to four heteroatoms of one or two elements selected from nitrogen, oxygen, and sulfur. Specific examples of the heterocyclic group can include, but are not limited to, a 5-membered cyclic group having carbon atoms and further having one to four heteroatoms selected from oxygen, sulfur, and nitrogen, such as a 2- or 3-thienyl group, 2- or 3-furyl group, a 1-, 2- or 3-pyrrolyl group, a 1-, 2- or 3-pyrrolidinyl group, a 2-, 4- or 5-oxazolyl group, a 3-, 4- or 5-isooxazolyl group, a 2-, 4- or 5-thiazolyl group, a 3-, 4- or 5-isothiazolyl group, a 3-, 4- or 5-pyrazolyl group, a 2-, 3- or 4-pyrazolidinyl group, a 2-, 4- or 5-imidazolyl group, a 1,2,3-triazolyl group, a 1,2,4-triazolyl group, and a 1H- or 2H-tetrazolyl group. Moreover, specific examples of the heterocyclic group can include, but are not limited to, a 6-membered cyclic group having carbon atoms and further having one to four heteroatoms selected from oxygen, sulfur, and nitrogen, such as a 2-, 3- or 4-pyridyl group, an N-oxide-2-, 3- or 4-pyridyl group, a 2-, 4- or 5-pyrimidinyl group, an N-oxide-2-, 4- or 5-pyrimidinyl group, a thiomorpholinyl group, a morpholinyl group, a piperidino group, a 2-, 3- or 4-piperidyl group, a thiopyranyl group, a 1,4-oxazinyl group, a 1,4-thiazinyl group, a 1,3-thiazinyl group, a piperazinyl group, a triazinyl group, a 3- or 4-pyridazinyl group, a pyrazinyl group, and an N-oxide-3- or 4-pyridazinyl group. Moreover, specific examples of the heterocyclic group can include, but are not limited to, a bicyclic to tetracyclic condensed ring group having carbon atoms and further having one to four heteroatoms selected from oxygen, sulfur, and nitrogen, such as an indolyl group, a benzofuryl group, a benzothiazolyl group, a benzoxazolyl group, a xanthenyl group, a benzimidazolyl group, a quinolyl group, an isoquinolyl group, a phthalazinyl group, a quinazolinyl group, a quinoxalinyl group, an indolizinyl group, a quinolizinyl group, a 1,8-naphthyridinyl group, a dibenzofuranyl group, a carbazolyl group, an acridinyl group, a phenanthridinyl group, a perimidinyl group, a phenazinyl group, a chromanyl group, a phenothiazinyl group, a phenoxazinyl group, and a 7H-pirazino[2,3-c]carbazolyl group.

In the Formula (III-I), R₃₂ is an optionally substituted aryl group, an optionally substituted heterocyclic group, an optionally substituted cycloalkyl group, or a group represented by the Formula (III-II).

The "aryl group" in the carboxylic-acid-derivative compound according to the present invention can be, but are not limited to, a phenyl group, an indenyl group, a naphathyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group.

R₃₂ in the Formula (III-I) can be a group represented by the Formula (III-II) with the following structure:

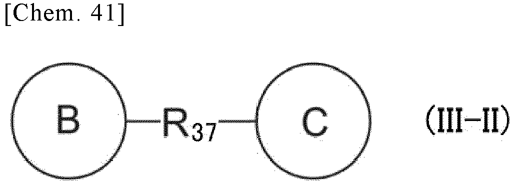

In the Formula (III-II), rings B and C respectively represent an optionally substituted aryl group, an optionally substituted heterocyclic group, or an optionally substituted cycloalkyl group, and R₃₇ represents a single bond, -O- group, -CO- group, -NH- group, -SO₂- group, -CO-NH- group, an optionally substituted alkylene group having 1 to 3 carbon atoms, an optionally substituted alkenylene group having 2 to 3 carbon atoms, -S-R₃₈- group (R₃₈ represents an optionally substituted alkylene group having 1 to 2 carbon atoms), -CO-R₃₈- group, -O-R₃₈- group, or -SO₂-R₃₈- group.

In this respect, the "alkylene group having 1 to 2 carbon atoms" is a methylene group or an ethylene group, and examples of the "alkylene group having 1 to 3 carbon atoms" other than the aforementioned alkylene group inclued a trimethylene group.

In addition, examples of the "alkenylene group having 2 to 3 carbon atoms" include a vinylene group and a 1-propenylene group.

In the Formula (III-I), when R₃₂ is a group represented by the Formula (III-II), any one of the ring B, the ring C, and R₃₇ is attached to Y₃.

The structure when Y₃ is attached to the ring B is represented by the following Formula (III-III), the structure when Y₃ is attached to the ring C is represented by the following Formula (III-IV), and the structure when Y₃ is attached to R₃₇ is represented by the following Formula (III-V):

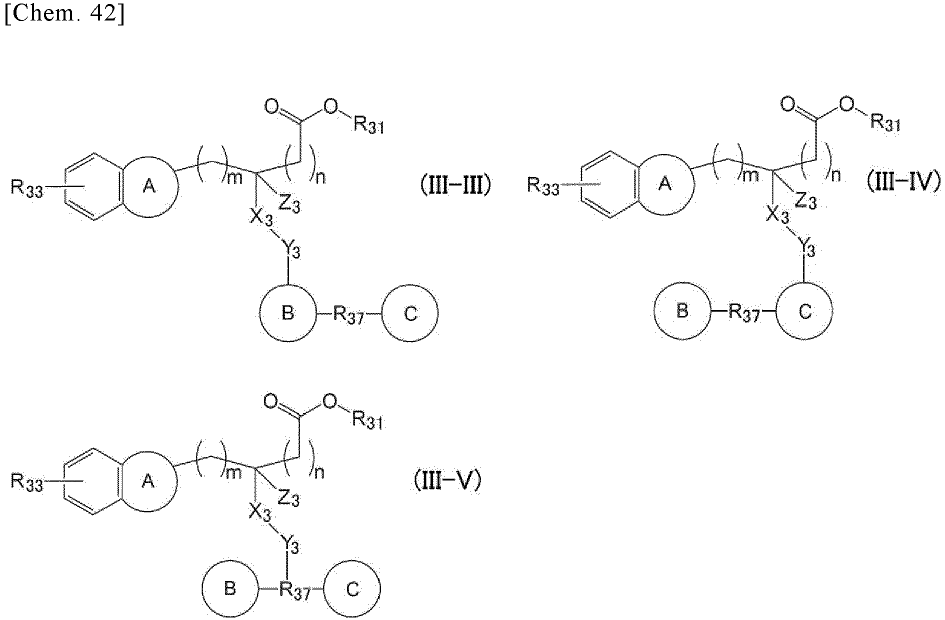

In order to provide the compound with high activity, R₃₂ in the Formula (III-I) is preferably the optionally substituted polycyclic aryl group or the optionally substituted polycyclic heterocyclic group.

In the Formula (III-I), R₃₂ is the same or different substituents.

The "substituent" in the carboxylic-acid-derivative compound according to the present invention refers to a halogen (such as, for example, fluorine, chlorine, bromine, or iodine), an alkyl group (for example, a C₁₋₆ alkyl group, such as a methyl group, an ethyl group, a propyl group, an isopropyl group, a butyl group, an isobutyl group, a *sec-*butyl group, a *tert*-butyl group, a pentyl group, or a hexyl group), a cycloalkyl group (for example, a C₃₋₆ cycloalkyl group, such as a cyclopropyl group, a cyclobutyl group, a cyclopentyl group, or a cyclohexyl group), an alkynyl group (for example, a C₂₋₆ alkynyl group, such as an ethynyl group, a 1-propynyl group, or a propargyl group), an alkenyl group (for example, a C₂₋₆ alkenyl group, such as a vinyl group, an allyl group, an isopropenyl group, a butenyl group, or an isobutenyl group), an aralkyl group (for example, a C₇₋₁₁ aralkyl group, such as a benzyl group, an α-methylbenzyl group, or a phenethyl group), an aryl group (for example, a C₆₋₁₀ aryl group, such as a phenyl group or a naphthyl group; preferably a phenyl group), an alkoxy group (for example, a C₁₋₆ alkoxy group, such as a methoxy group, an ethoxy group, a propoxy group, an isopropoxy group, a butoxy group, an isobutoxy group, a sec-butoxy group, or a *tert-*butoxy), an aryloxy group (for example, a C₆₋₁₀ aryloxy group, such as phenoxy), an alkanoyl group (for example, a C₁₋₆ alkyl-carbonyl group, such as a formyl group, an acetyl group, a propionyl group, a butyryl group, or an isobutyryl group), an arylcarbonyl group (for example, a C₆₋₁₀ aryl-carbonyl group, such as a benzoyl group or a naphthoyl group), an alkanoyloxy group (for example, a C₁₋₆ alkyl-carbonyloxy group, such as a formyloxy group, an acetyloxy group, a propionyloxy group, a butyryloxy group, or an isobutyryloxy group), an arylcarbonyloxy group (for example, a C₆₋₁₀ aryl-carbonyloxy group, such as a benzoyloxy group or a naphthoyloxy group), carboxyl group, an alkoxycarbonyl group (for example, a C₁₋₆ alkoxy-carbonyl group, such as a methoxycarbonyl group, an ethoxycarbonyl group, a propoxycarbonyl group, an isopropoxycarbonyl group, a butoxycarbonyl group, an isobutoxycarbonyl group, or a *tert*-butoxycarbonyl), an aralkyloxycarbonyl group (for example, a C₇₋₁₁ aralkyloxycarbonyl group, such as a benzyloxycarbonyl group), a carbamoyl group, a halogenated alkyl group (for example, a mono-, di-, or tri-halogenated -C₁₋₄ alkyl group, such as a chloromethyl group, a dichloromethyl group, a trifluoromethyl group, or a 2,2,2-trifluoroethyl group), an oxo group, an amidino group, an imino group, an amino group, an alkylamino group (for example, a mono-C₁₋₄ alkylamino group, such as a methylamino group, an ethylamino group, a propylamino group, an isopropylamino group, or a butylamino group), a dialkylamino group (for example, a di-C₁₋₄ alkylamino group, such as a dimethylamino group, a diethylamino group, a dipropylamino group, a diisopropylamino group, a dibutylamino group, or a methylethylamino group), an alkoxycarbonylamino group (for example, a C₁₋₆ alkoxycarbonylamino group, such as a methoxycarbonylamino group, an isoproxycarbonylamino group, or a *tert-*butoxycarbonylamino group), a cyclic amino group (a 3- to 6-membered cyclic amino group containing carbon atoms and one nitrogen atom and further containing one to three heteroatoms selected from oxygen, sulfur, and nitrogen; such as, for example, an aziridinyl group, an azetidinyl group, a pyrrolidinyl group, a pyrrolinyl group, a pyrrolyl group, an imidazolyl group, a pyrazolyl group, an imidazolidinyl group, a piperidyl group, a morpholinyl group, a dihydropyridyl group, a pyridyl group, an N-methylpiperazinyl group, or an N-ethylpiperazinyl group), an alkylenedioxy group (for example, a C₁₋₃ alkylenedioxy group, such as a methylenedioxy group or an ethylenedioxy group), a hydroxy group, a cyano group, a mercapto group, a sulfo group, a sulfino group, a phosphono group, a sulfamoyl group, a monoalkylsulfamoyl group (for example, a mono-C₁₋₆ alkylsulfamoyl group, such as N-methylsulfamoyl, N-ethylsulfamoyl, N-propylsulfamoyl, N-isopropylsulfamoyl, or N-butylsulfamoyl), a dialkylsulfamoyl group (for example, a di-C₁₋₆ alkylsulfamoyl group, such as an N,N-dimethylsulfamoyl group, an N,N-diethylsulfamoyl group, an N,N-dipropylsulfamoyl group, or an N,N-dibutylsulfamoyl group), an alkylthio group (for example, a C₁₋₆ alkylthio group, such as a methylthio group, an ethylthio group, propylthio group, an isopropylthio group, a butylthio group, a *sec*-butylthio group, or a *tert*-butylthio group), an arylthio group (for example, a C₆₋₁₀ arylthio group, such as a phenylthio group or a naphthylthio group), an alkylsulfinyl group (for example, a C₁₋₆ alkylsulfinyl group, such as a methylsulfinyl group, an ethylsulfinyl group, a propylsulfinyl group, or a butylsulfinyl group), an alkylsulfonyl group (for example, a C₁₋₆ alkylsulfonyl group, such as a methylsulfonyl group, an ethylsulfonyl group, a propylsulfonyl group, or a butylsulfonyl group), or an arylsulfonyl group (for example, a C₆₋₁₀ arylsulfonyl group, such as a phenylsulfonyl group or a naphthylsulfonyl group).

Among the aforementioned substituents, low-molecular-weight substituents having 1 to 10 atoms are preferable. Examples that can be used as such a substituent include, but are not limited thereto, a halogen atom, a methyl group, an ethyl group, a vinyl group, a methoxy group, an ethoxy group, an acetyl group, a carboxyl group, a methoxycarbonyl group, a chloromethyl group, an amino group, a methylamino group, a hydroxy group, a sulfo group, and a methylthio group.

In the carboxylic-acid-derivative compound according to the present invention, the phrase "optionally substituted" means that a substituent as described above is present or absent. In cases where a moiety is substituted, two or more substituents may be present within the moiety, and the substituents may be identical to or different from each other. In cases where the carboxylic-acid-derivative compound according to the present invention is "optionally substituted," the number of substituents within the compound is preferably from 0 to 3.

In the Formula (III-I), Z₃ is a hydrogen atom, an optionally substituted amino group, or an optionally substituted amine.

In the carboxylic-acid-derivative compound according to the present invention, the "optionally substituted amino group" is a primary amino group, a secondary amino group, or a tertiary amino group. The primary amino group is -NH₂ group. The secondary amino group is an amino group having one substituent, and may include, but are not limited to, for example, alkylamino group, arylamino group, alkoxycarbonylamino group and the like. The tertiary amino group is an amino group having identical or different two substituents, and may include, but not limited to, for example, dialkylamino group, diarylamino group and the like.

When Z₃ is the optionally substituted amine, X₃ and Z₃ form a ring. The phrase "form a ring" means that Z₃ is -NH-, -N=, or -NR- (R represents a substituent) and is attached to any atom within X₃ to form a ring.

Examples of the ring formed by X₃ and Z₃ include, but are not limited to: a dihydrothiazole ring which is formed in the case that X₃ is -CH₂-S-CH₂- group or -CH₂-S-(CH₂)₂-S- group, Z₃ is -N=, and Z₃ is attached to a carbon atom within X₃ via a double bond; and a piperidine ring which is formed in the case that X₃ is a tetramethylene group, Z₃ is -NH-, and Z₃ is attached to a carbon atom within X₃.

In the Formula (III-I), when X₃ is -CH₂-S-CH₂- group, Z₃ is -N=, and X₃ and Z₃ form a dihydrothiazole ring, the compound can be represented by the following Formula (III-VI): 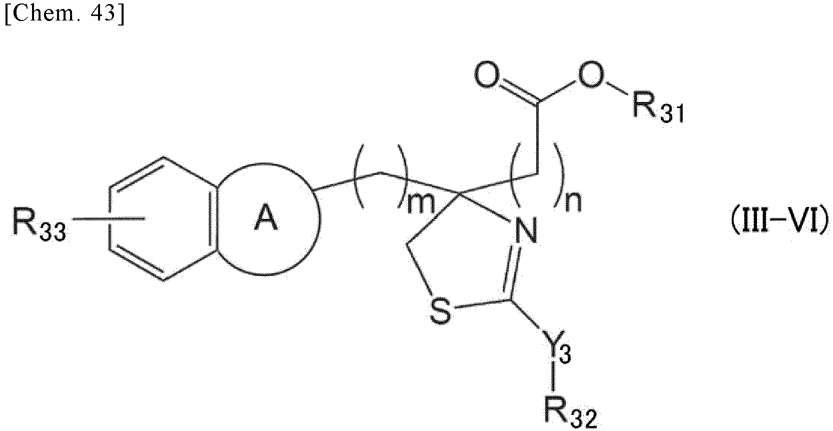 (wherein m, n, A, Y₃, R₃₁, R₃₂, and R₃₃ are the same as above).

Many compounds represented by the Formula (III-VI) have been synthesized in Patent Document 2 (WO 2006/040646), and it has been confirmed that these compounds have inhibitory activity against Pint. The specific examples of chemical structures of some of these compounds are as follows:

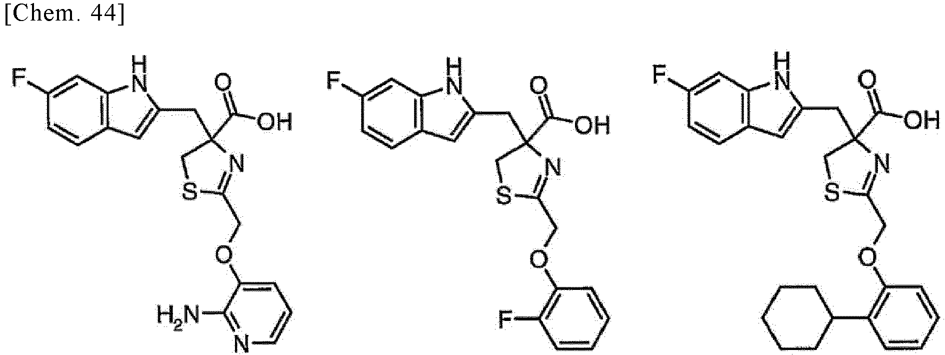

All of these compounds correspond to a compound represented by the Formula (III-VI) in which Y₃ is -O-CH₂- group (-O-R₃₆- group).

In the Formula (III-I), when X₃ is -CH₂-S-(CH₂)₂-S- group, Z₃ is -N= group, and X₃ and Z₃ form a dihydrothiazole ring, the compound can be represented by the following Formula (III-VII): 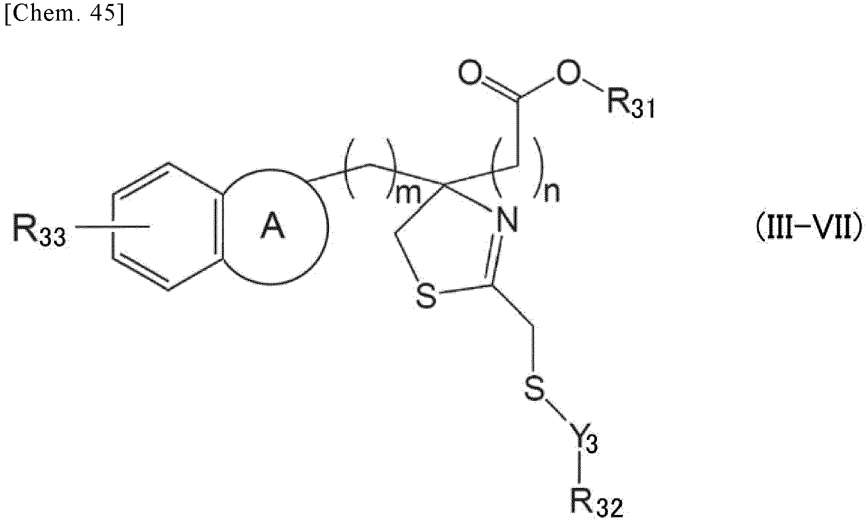 (wherein m, n, A, Y₃, R₃₁, R₃₂, and R₃₃ are the same as above).

Many compounds represented by the Formula (III-VII) have been synthesized in Patent Document 2 (WO 2006/040646), and it has been confirmed that these compounds have inhibitory activity against Pin1. The specific examples of chemical structures of some of these compounds are as follows:

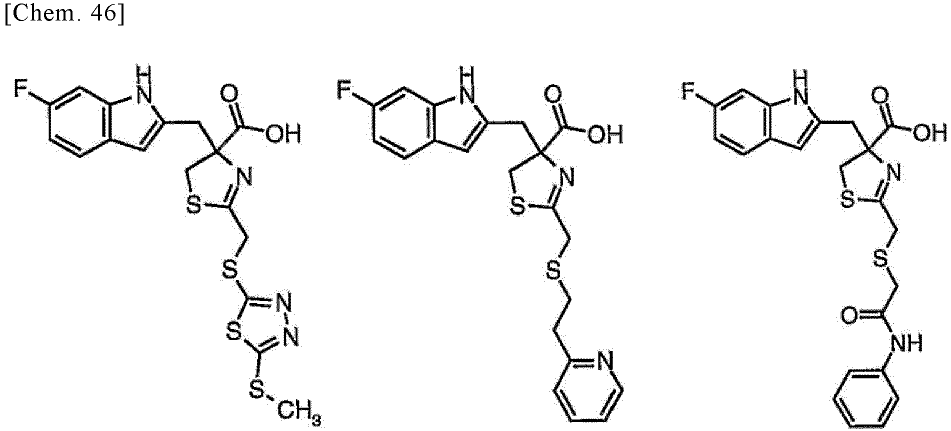

These compounds respectively correspond to compounds represented by the Formula (III-VII) in which Y₃ is a single bond, -CH₂-CH₂- group (alkylene group), and -CH₂-CO-NH- group (-NR₃₅-CO-R₃₆- group).

In the compound represented by the Formula (III-I), X₃ can be -NH-CO- group. In this case, it is preferable that m=1, n=0, and Z₃ is a hydrogen atom because there are abundant raw materials for synthesis such as amino acid derivatives.

In this case, the Formula (III-I) can be represented by the following Formula (III-VIII): 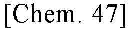 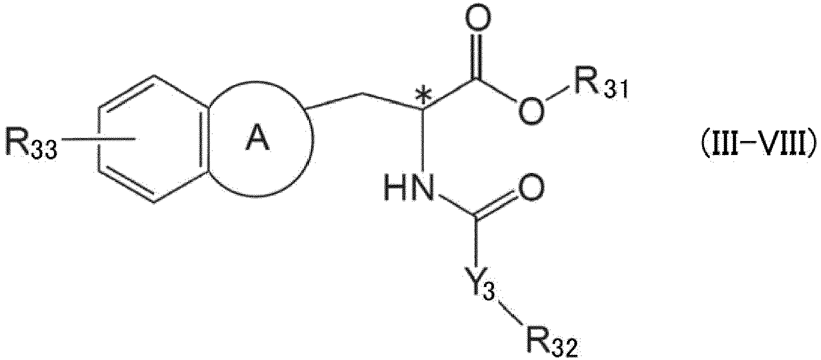 (wherein m, n, A, Y₃, R₃₁, R₃₂, and R₃₃ are the same as above. * indicates the position of an asymmetric carbon atom.)

Many compounds represented by the Formula (III-VIII) have been synthesized in Patent Document 2 (WO 2006/040646), and it has been confirmed that these compounds have inhibitory activity against Pin1. The specific examples of chemical structures of some of these compounds are as follows:

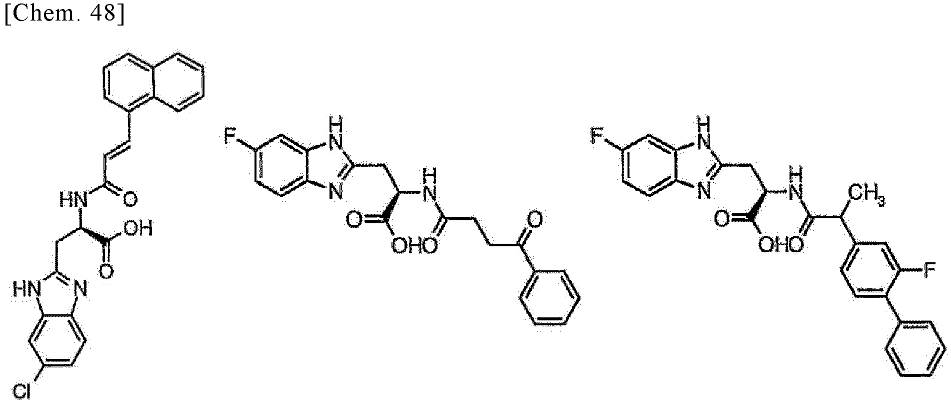

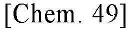

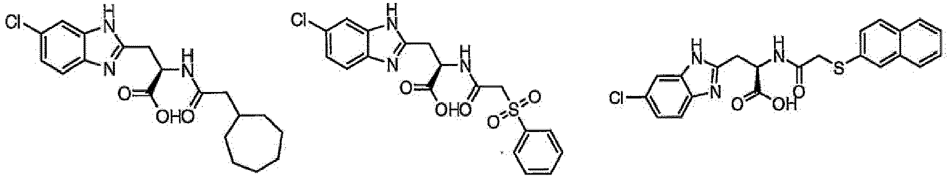

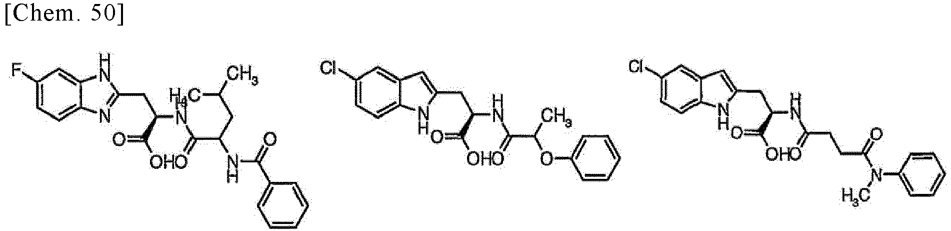

The compounds in the upper row respectively correspond to compounds represented by the Formula (III-VIII) in which Y₃ is, from left to right, -CH=CH- group (alkenylene group), -CH₂-CH₂-CO- group (-CO-R₃₆- group), and -CH(CH₃)- group (substituted alkylene group).

The compounds in the middle row respectively correspond to compounds represented by the Formula (III-VIII) in which Y₃ is, from left to right, -CH₂- group (alkylene group), -CH₂-SO₂- group (-SO₂-R₃₆- group), and -CH₂-S- group (-S-R₃₆-group).

The compounds in the lower row respectively correspond to compounds represented by the Formula (III-VIII) in which Y₃ is -CH(CH₂CH(CH₃)₂)-NH-CO-group (-R₃₆-NR₃₅-CO- group), -CH(CH₃)-O- group (-O-R₃₆- group), and -CH₂-CH₂-CO-N(CH₃)- group (-NR₃₅-CO-R₃₆- group).

Many compounds represented by the Formula (III-VIII) have been synthesized in Non-Patent Document 3 (Bioorg. Med. Chem. Lett., 2010, Vol.20, No.2, pp.586-590), and it has been confirmed that these compounds have inhibitory activity against Pin1. The specific examples of chemical structures of some of these compounds are as follows:

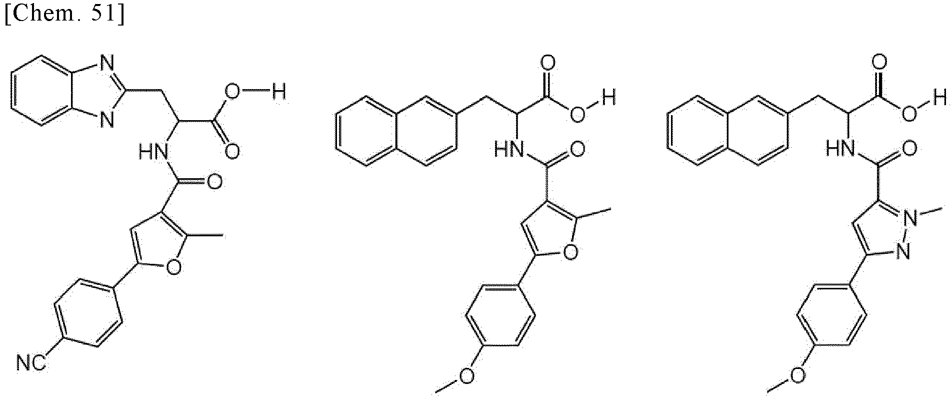

All of these compounds correspond to a compound represented by the Formula (III-VIII) in which Y₃ is a single bond. The middle compound of these three compounds is designated as (R)-2-(5-(4-methoxyphenyl)-2-methylfuran-3-carboxamido)-3-(naphthalene-6-yl) propanoic acid, hereinafter referred to as "C1".

The compound represented by the Formula (III-VIII) has inhibitory activity against the function of Pin1 when R₃₁ is a hydrogen atom and the compound has a carboxyl group. However, even if R₃₁ is a methyl group or a benzyl group, it can easily become a carboxyl group by hydrolysis so that the compound can exhibit inhibitory activity against the function of Pin1.

For example, the compounds synthesized in Example 1 of Patent Document 5 (WO 2019/031471) corresponds to compounds represented by the Formula (III-VIII) in which Y₃ is a single bond, -CH₂- group (alkylene group), or -NH- group.

The compound represented by the Formula (III-VIII) can be synthesized by, for example, but not limited to, using amino acid derivatives as raw materials according to the scheme shown in the following reaction flow chart: 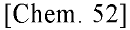 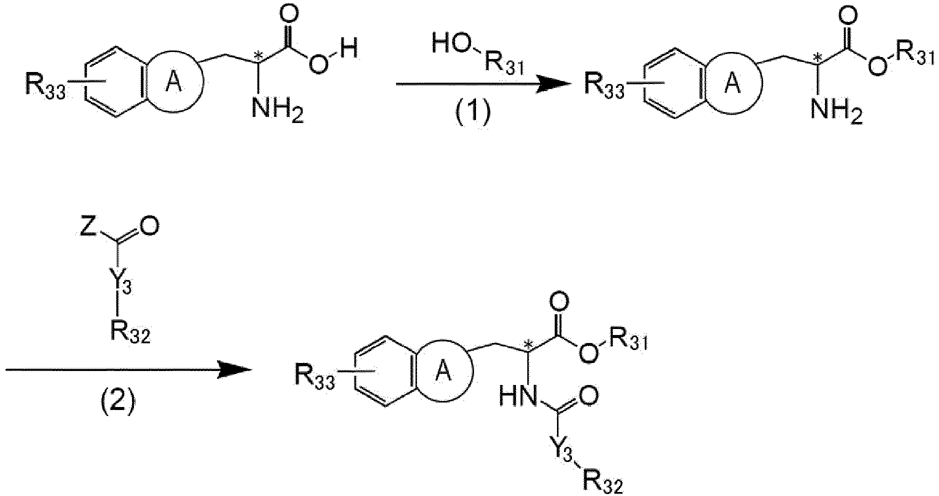 (wherein R₃₁, R₃₂, R₃₃, and Y₃ are the same as above, and Z represents a hydroxyl group or a chlorine atom).

In the reaction (1) in the aforementioned scheme, an alcohol having R₃₁ is reacted with a carboxyl group of an amino acid derivative for dehydration condensation, thereby attaching R₃₁ to the amino acid derivative via an ester bond. Then in the reaction (2), a carboxylic acid or acid chloride having R₃₂ and Y₃ is reacted with an amino group in the compound produced by the reaction (1) for dehydration condensation, thereby attaching Y₃ to R₃₂ via an amide bond.

In this scheme, for synthesizing a compound in which R₃₁ is H, R₃₁ can be detached by hydrolysis or the like after the reactions (1) and (2) so as to obtain the compound in which R₃₁ is H, or alternatively, a carboxylic acid having R₃₂ and Y₃ is directly reacted with an amino acid derivative in which R₃₁ is a hydrogen atom for dehydration condensation so as to obtain the compound in which R₃₁ is H without the reaction (1).

In the compound represented by the Formula (III-I), X₃ can be -O-CO- group. In this case, it is preferable that m=1, n=0, and Z₃ is a hydrogen atom because there are abundant raw materials for synthesis such as amino acid derivatives.

In this case, the Formula (III-I) can be represented by the following Formula (III-IX): 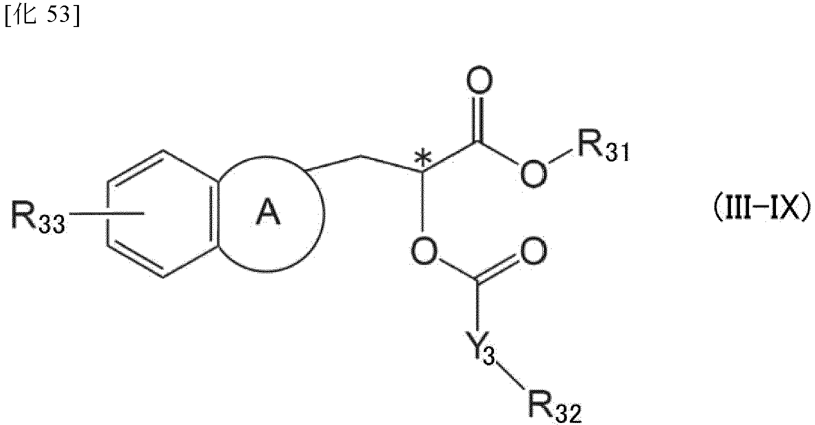 (wherein m, n, A, Y₃, R₃₁, R₃₂, and R₃₃ are the same as above. * indicates the position of an asymmetric carbon atom.)

The compound represented by the Formula (III-IX) can be a therapeutic or prophylactic agent that can be easily decomposed due to its ester bond and has few side effects.

The compound represented by the Formula (III-IX) has inhibitory activity against the function of Pin1 when R₃₁ is a hydrogen atom and the compound has a carboxyl group. However, even if R₃₁ is a benzyl group, it can easily become a carboxyl group by hydrolysis so that the compound can exhibit inhibitory activity against the function of Pin1.

For example, the compounds synthesized in Example 1 of Patent Document 5 (WO 2019/031471) corresponds to compounds represented by the Formula (III-IX) in which Y₃ is a single bond, -CH₂- group (alkylene group), -CHz-O- group (-O-R₃₆- group) or -CH(NHBoc)-CH₂- group (substituted alkylene group).

The compound represented by the Formula (III-IX) can be synthesized by, for example, but not limited to, using amino acid derivatives as raw materials according to the scheme shown in the following reaction flow chart: 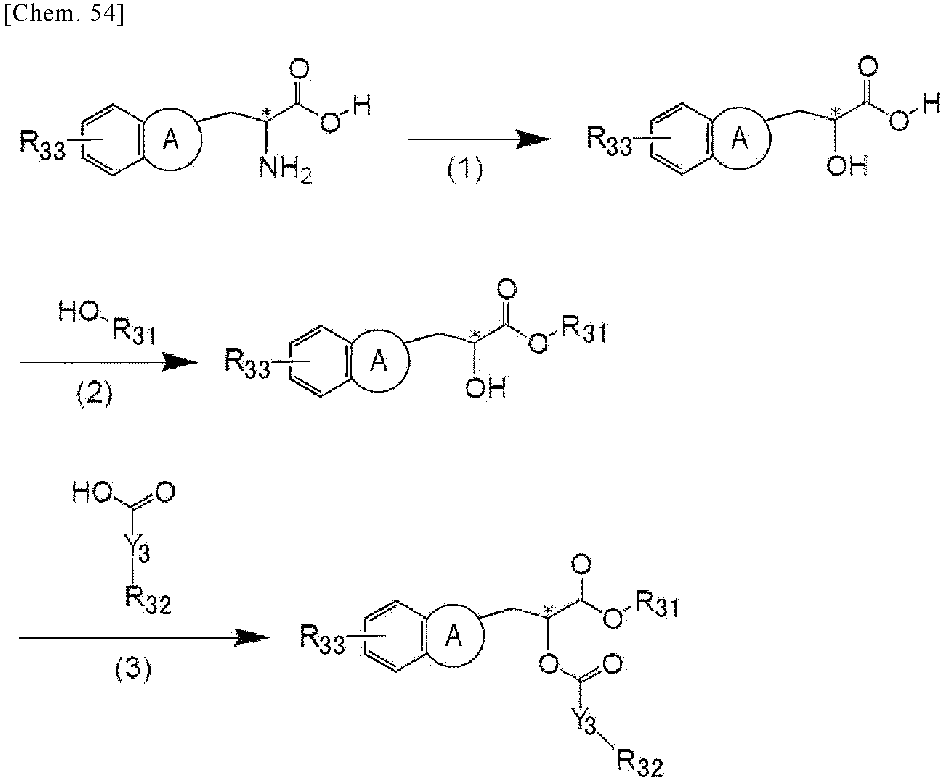 (wherein A, Y₃, R₃₁, R₃₂, and R₃₃ are the same as above).

In the reaction (1) in the aforementioned scheme, an amino group in an amino acid derivative as a raw material becomes a hydroxyl group via a substitution reaction. In the reaction (2), an alcohol having R₃₁ is reacted with a carboxylic acid of an hydroxycarboxylic acid derivative for dehydration condensation, thereby attaching R₃₁ to the amino acid derivative via an ester bond. Then in the reaction (3), a carboxylic acid having R₃₂ and Y₃ is reacted with a hydroxyl group produced by the reaction (1) for dehydration condensation, thereby attaching Y₃ to R₃₂ via an ester bond.

In this scheme, for synthesizing a compound in which R₃₁ is H, R₃₁ can be detached by hydrolysis or the like after the reactions (1) to (3) so as to obtain the compound in which R₃₁ is H, or alternatively, a hydroxycarboxylic acid derivative is directly reacted with a carboxylic acid having R₃₂ and Y₃ for dehydration condensation so as to obtain the compound in which R₃₁ is H without the reaction (2).

In the compound represented by the Formula (III-I), X₃ can be -NH- group. Many compounds represented by the Formula (III-I) in which X₃ is -NH- group have been synthesized in Patent Document 2 (WO 2006/040646), and it has been confirmed that these compounds have inhibitory activity against Pin1. The specific examples of chemical structures of some of these compounds are as follows:

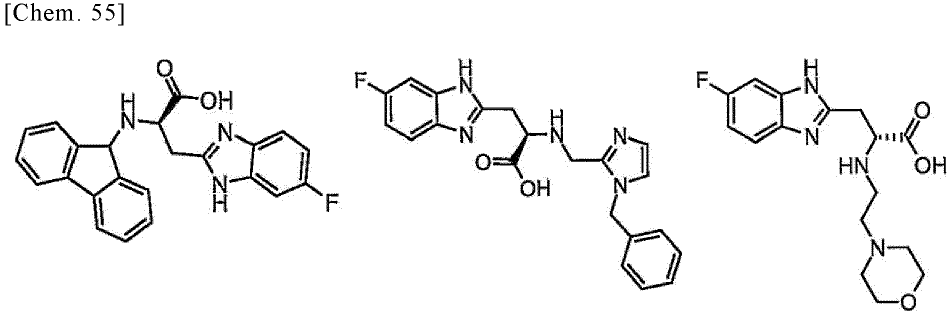

These compounds respectively correspond to compounds represented by the Formula (III-I) in which Y₃ is, from left to right, a single bond, -CH₂- group (alkylene group), and -CH₂-CH₂- group (alkylene group).

In the compound represented by the Formula (III-I), X₃ can be -NH-R₃₄- group (R₃₄ is an alkylene group having 1 to 5 carbon atoms, or an alkenylene group having 2 to 5 carbon atoms). Many compounds represented by the Formula (III-I) in which X₃ is -NH-R₃₄- group have been synthesized in Patent Document 2 (WO 2006/040646), and it has been confirmed that these compounds have inhibitory activity against Pin1. The specific examples of chemical structures of some of these compounds are as follows:

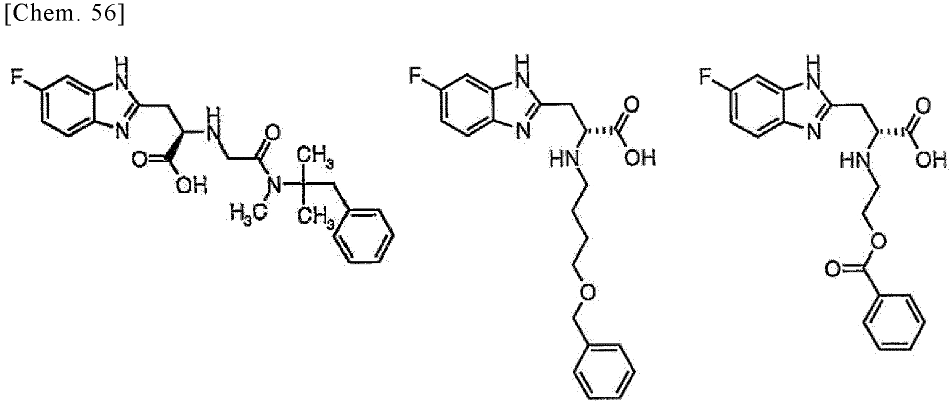

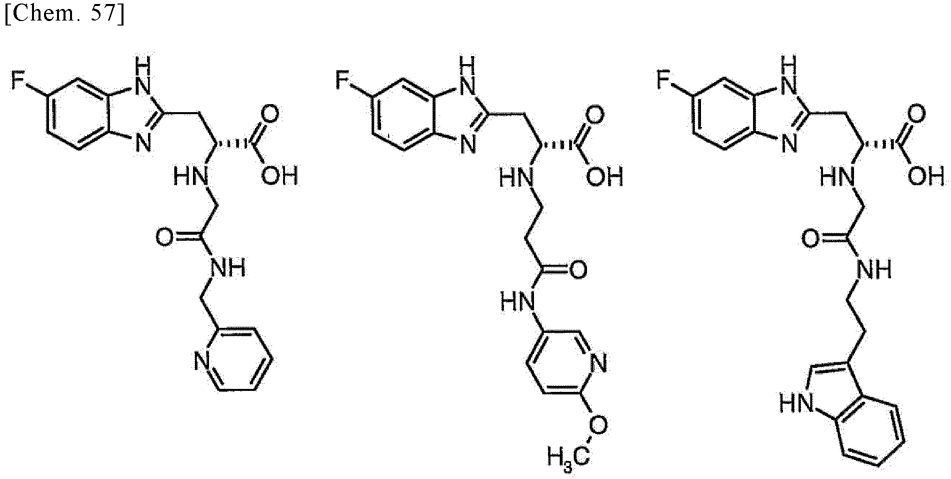

The compound on the left of the upper row corresponds to a compound represented by the Formula (III-I) in which X₃ is -NH-CH₂- group (-NH-R₃₄- group) and Y₃ is -CO-N(CH₃)-C(CH₃)₂-CH₂- group (-R₃₆-NR₃₅-CO- group). The compound in the middle of the upper row corresponds to a compound represented by the Formula (III-I) in which X₃ is -NH-(CH₂)₄- group (-NH-R₃₄- group) and Y₃ is -O-CH₂- group (-O-R₃₆-group). The compound on the right of the upper row corresponds to a compound represented by the Formula (III-I) in which X₃ is -NH-(CH₂)₂- group and Y₃ is -O-CO-group.

The compound on the left of the lower row corresponds to a compound represented by the Formula (III-I) in which X₃ is -NH-CH₂- group (-NH-R₃₄- group) and Y₃ is -CO-NH-CH₂- group (-R₃₆-NR₃₅-CO- group). The compound in the middle of the lower row corresponds to a compound represented by the Formula (III-I) in which X₃ is -NH-(CH₂)₂- group (-NH-R₃₄- group) and Y₃ is -CO-NH-CH₂- group (-CO-NR₃₅-group). The compound on the right of the lower row corresponds to a compound represented by the Formula (III-I) in which X₃ is -NH-CH₂- group (-NH-R₃₄- group) and Y₃ is -CO-NH-(CH₂)₂- group (-R₃₆-NR₃₅-CO- group).

In the compounds represented by the Formula (III-I), both X₃ and Y₃ can be a single bond.

Many compounds represented by the Formula (III-I) in which both X₃ and Y₃ are a single bond have been synthesized in Patent Document 2 (WO 2006/040646), and it has been confirmed that these compounds have inhibitory activity against Pin1. The specific examples of chemical structures of some of these compounds are as follows:

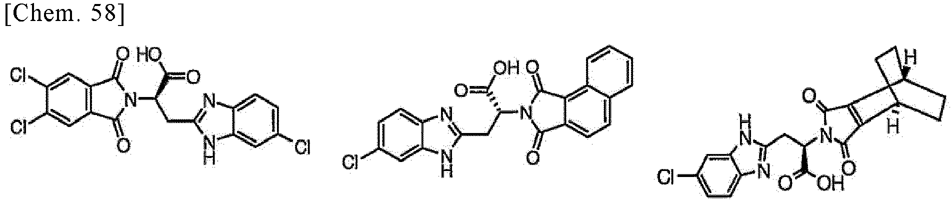

R₃₂ in the Formula (III-I) can be a group represented by the Formula (III-II) with the following structure:

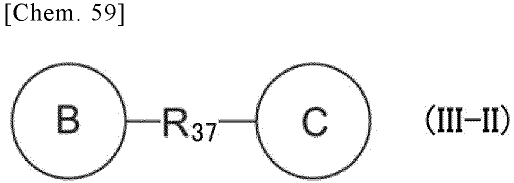

In the Formula (III-II), rings B and C respectively represent an optionally substituted aryl group, an optionally substituted heterocyclic group, or an optionally substituted cycloalkyl group, and R₃₇ represents a single bond, -O- group, -CO- group, -NH- group, -SO₂- group, -CO-NH- group, an optionally substituted alkylene group having 1 to 3 carbon atoms, an optionally substituted alkenylene group having 2 to 3 carbon atoms, -S-R₃₈- group (R₃₈ represents an optionally substituted alkylene group having 1 to 2 carbon atoms), -CO-R₃₈- group, -O-R₃₈- group, or -SO₂-R₃₈- group.

Many compounds represented by the Formula (III-I) in which R₃₂ is the compound represented by the Formula (III-II) have been synthesized in Patent Document 2 (WO 2006/040646), and it has been confirmed that these compounds have inhibitory activity against Pin1. The specific examples of chemical structures of some of these compounds are as follows:

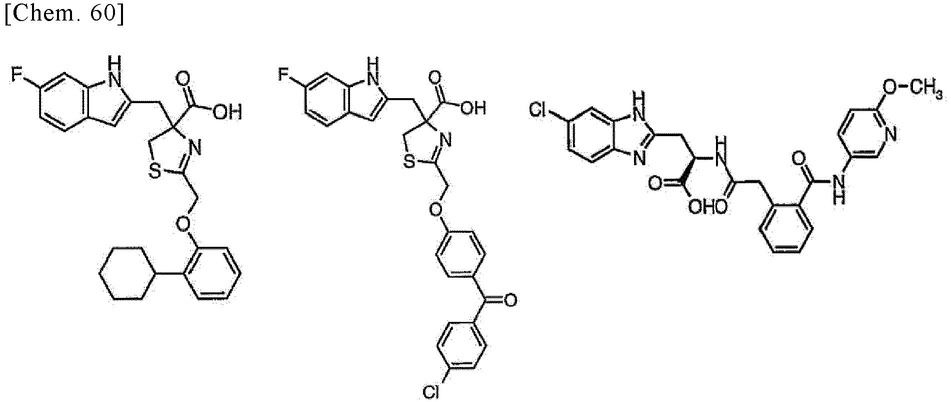

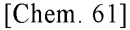

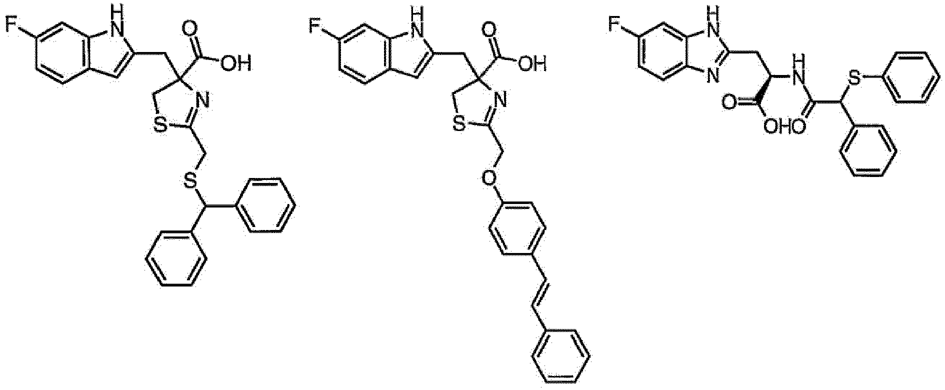

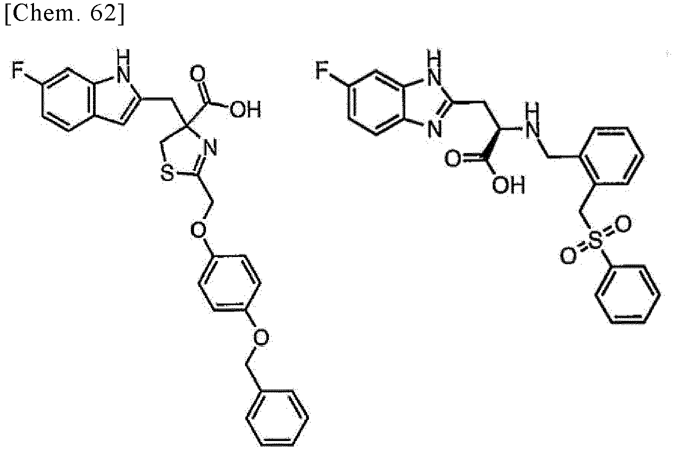

The compounds in the upper row respectively correspond to compounds represented by the Formula (III-II) in which R₃₇ attaching the two rings to each other is, from left to right, a single bond, -CO- group, and -CO-NH- group.

The compounds in the middle row respectively correspond to compounds represented by the Formula (III-II) in which R₃₇ is, from left to right, -CH₂- group (alkylene group), -C=C- group (alkenylene group), and -CH₂-S- group (-S-R₃₈- group).

The compounds in the lower row respectively correspond to compounds represented by the Formula (III-II) in which R₃₇ is -O-CH₂- group (-O-R₃₈- group) and -CH₂-SO₂- group (-SO₂-R₃₈- group).

Examples that can be used as the compounds of the Formula (III-I) in which R₃₂ is a group represented by the Formula (III-II) can include the compounds in which R₃₇ is -O- group, -CO- group, or -NH- group. Examples that can be used as the compounds of the Formula (III-I) in which R₃₂ is a group represented by the Formula (III-II) can include the compounds in which R₃₇ is single bond, -O- group, -CH₂- group (alkylene group), or -CH(OH)- group (substituted alkylene group).

The carboxylic-acid-derivative compound can be synthesized using, for example, a method described in WO 2019/031471, although it is not limited thereto.

### 1-4. 3,5-diaminobenzoic Acid Compound

### 1-4-1. General Formula of the Compound

Another one of the compounds that are active ingredients of the therapeutic or prophylactic agents for coronavirus disease according to present invention is a compound that has a chemical structure represented by the following Formula (IV-I) and inhibitory activity against the function of Pin1:

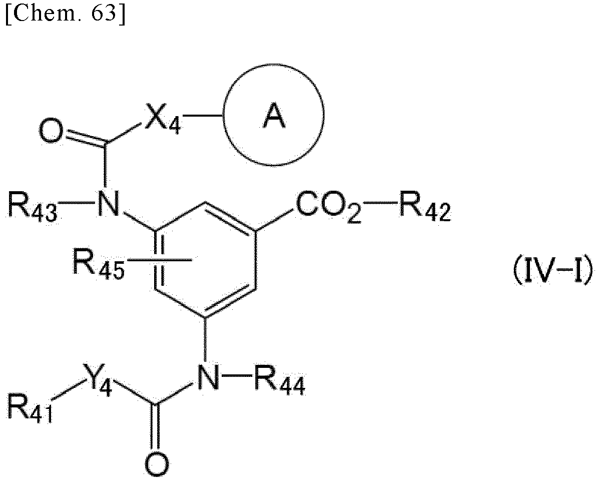

### 1-4-2. Ring A

In the Formula (IV-I), a ring A represents an optionally substituted monocyclic or polycyclic aromatic ring or heterocycle.

The ring A is attached to X₄ via any atom in the monocyclic or polycyclic aromatic ring or heterocycle (preferably a carbon atom or a nitrogen atom). In cases where X₄ is a single bond, the ring A is directly attached to a carbonyl group (-CO-).

In the 3,5-diaminobenzoic acid compound according to the present invention, the "aromatic ring" is an unsaturated carbon organic compound composed of carbon and hydrogen in a form of a ring. Examples of a monocyclic aromatic ring include, but are not limited to, a benzene ring, a cyclopentadiene ring and the like. Examples of a polycyclic aromatic ring include, but are not limited to, a naphthalene ring, an indene ring, an azulene ring, a fluorene ring, a phenanthrene ring, an anthracene ring, a tetracene ring, a pentacene ring, a benzopyrene ring, a chrysene ring, a pyrene ring, a triphenylene ring and the like.

In the 3,5-diaminobenzoic acid compound according to the present invention, the "heterocycle" refers to an organic compound composed of carbon, hydrogen and other atoms in a form of a single ring. Examples of a monocyclic heterocycle include, but are not limited to, a pyrrole ring, an imidazole ring, a pyrrolidine ring, a furan ring, a tetrahydrofuran ring, a 1,3-dioxolane ring, a thiophene ring, a pyridine ring, a pyrazine ring, a pyrimidine ring, a piperazine ring, a pyran ring, a 1,4-dioxane ring and the like. Examples of a polycyclic aromatic ring include, but are not limited to, an indole ring, a quinoline ring, a quinoxaline ring, a quinazoline ring, a purine ring, an isobenzofuran ring, a chroman ring, a benzodioxane ring, a benzodioxole ring, a carbazole ring, an acridine ring, a phenoxazine ring, a 4H-pyrido [2,3-c] carbazole ring and the like.

In the 3,5-diaminobenzoic acid compound according to the present invention, the "cyclic hydrocarbon" refers to a saturated carbon organic compound composed of carbon and hydrogen in a form of a ring. Examples of a monocyclic hydrocarbon include, but are not limited to, cyclopentane, cyclohexane, cycloheptane and the like. Examples of a polycyclic hydrocarbon include, but are not limited to, tricycloheptane, tricyclodecane, perhydro-1,4-ethanoanthracene and the like.

In the 3,5-diaminobenzoic acid compound according to the present invention, the "substituent" refers to a halogen (such as, for example, fluorine, chlorine, bromine, or iodine), an alkyl group (for example, a C₁₋₆ alkyl group, such as a methyl group, an ethyl group, a propyl group, an isopropyl group, a butyl group, an isobutyl group, a *sec-*butyl group, a tert-butyl group, a pentyl group, or a hexyl group), a cycloalkyl group (for example, a C₃₋₆ cycloalkyl group, such as a cyclopropyl group, a cyclobutyl group, a cyclopentyl group, or a cyclohexyl group), an alkynyl group (for example, a C₂₋₆ alkynyl group, such as an ethynyl group, a 1-propynyl group, or a propargyl group), an alkenyl group (for example, a C₂₋₆ alkenyl group, such as a vinyl group, an allyl group, an isopropenyl group, a butenyl group, or an isobutenyl group), an aralkyl group (for example, a C₇₋₁₁ aralkyl group, such as a benzyl group, an α-methylbenzyl group, or a phenethyl group), an aryl group (for example, a C₆₋₁₀ aryl group, such as a phenyl group or a naphthyl group; preferably a phenyl group), an alkoxy group (for example, a C₁₋₆ alkoxy group, such as a methoxy group, an ethoxy group, a propoxy group, an isopropoxy group, a butoxy group, an isobutoxy group, a sec-butoxy group, or a *tert-*butoxy), an aryloxy group (for example, a C₆₋₁₀ aryloxy group, such as phenoxy), an alkanoyl group (for example, a C₁₋₆ alkyl-carbonyl group, such as a formyl group, an acetyl group, a propionyl group, a butyryl group, or an isobutyryl group), an arylcarbonyl group (for example, a C₆₋₁₀ aryl-carbonyl group, such as a benzoyl group or a naphthoyl group), an alkanoyloxy group (for example, a C₁₋₆ alkyl-carbonyloxy group, such as a formyloxy group, an acetyloxy group, a propionyloxy group, a butyryloxy group, or an isobutyryloxy group), an arylcarbonyloxy group (for example, a C₆₋₁₀ aryl-carbonyloxy group, such as a benzoyloxy group or a naphthoyloxy group), a carboxyl group, an alkoxycarbonyl group (for example, a C₁₋₆ alkoxy-carbonyl group, such as a methoxycarbonyl group, an ethoxycarbonyl group, a propoxycarbonyl group, an isopropoxycarbonyl group, a butoxycarbonyl group, an isobutoxycarbonyl group, or a *tert*-butoxycarbonyl), an aralkyloxycarbonyl group (for example, a C₇₋₁₁ aralkyloxycarbonyl group, such as a benzyloxycarbonyl group), a carbamoyl group, a halogenated alkyl group (for example, a mono-, di-, or tri-halogenated -C₁₋₄ alkyl group, such as a chloromethyl group, a dichloromethyl group, a trifluoromethyl group, or a 2,2,2-trifluoroethyl group), an oxo group, an amidino group, an imino group, an amino group, an alkylamino group (for example, a mono-C₁₋₄ alkylamino group, such as a methylamino group, an ethylamino group, a propylamino group, an isopropylamino group, or a butylamino group), a dialkylamino group (for example, a di-C₁₋₄ alkylamino group, such as a dimethylamino group, a diethylamino group, a dipropylamino group, a diisopropylamino group, a dibutylamino group, or a methylethylamino group), an alkoxycarbonylamino group (for example, a C₁₋₆ alkoxycarbonylamino group, such as a methoxycarbonylamino group, an isoproxycarbonylamino group, or a *tert-*butoxycarbonylamino group), a cyclic amino group (a 3- to 6-membered cyclic amino group containing carbon atoms and one nitrogen atom and further containing one to three heteroatoms selected from oxygen, sulfur, and nitrogen; such as, for example, an aziridinyl group, an azetidinyl group, a pyrrolidinyl group, a pyrrolinyl group, a pyrrolyl group, an imidazolyl group, a pyrazolyl group, a imidazolidinyl group, a piperidyl group, a morpholinyl group, a dihydropyridyl group, a pyridyl group, an N-methylpiperazinyl group, or an N-ethylpiperazinyl group), an alkylenedioxy group (for example, a C₁₋₃ alkylenedioxy group, such as a methylenedioxy group or an ethylenedioxy group), a hydroxy group, a cyano group, a mercapto group, a sulfo group, a sulfino group, a phosphono group, a sulfamoyl group, a monoalkylsulfamoyl group (for example, a mono-C₁₋₆ alkylsulfamoyl group, such as N-methylsulfamoyl, N-ethylsulfamoyl, N-propylsulfamoyl, N-isopropylsulfamoyl, or N-butylsulfamoyl), a dialkylsulfamoyl group (for example, a di-C₁₋₆ alkylsulfamoyl group, such as an N,N-dimethylsulfamoyl group, an N,N-diethylsulfamoyl group, an N,N-dipropylsulfamoyl group, or an N,N-dibutylsulfamoyl group), an alkylthio group (for example, a C₁₋₆ alkylthio group, such as a methylthio group, an ethylthio group, a propylthio group, an isopropylthio group, a butylthio group, a sec-butylthio group, or a *tert*-butylthio group), an arylthio group (for example, a C₆₋₁₀ arylthio group, such as a phenylthio group or a naphthylthio group), an alkylsulfinyl group (for example, a C₁₋₆ alkylsulfinyl group, such as a methylsulfinyl group, an ethylsulfinyl group, a propylsulfinyl group, or a butylsulfinyl group), an alkylsulfonyl group (for example, a C₁₋₆ alkylsulfonyl group, such as a methylsulfonyl group, an ethylsulfonyl group, a propylsulfonyl group, or a butylsulfonyl group), or an arylsulfonyl group (for example, a C₆₋₁₀ arylsulfonyl group, such as a phenylsulfonyl group or a naphthylsulfonyl group).

In the 3,5-diaminobenzoic acid compound according to the present invention, the phrase "optionally substituted" means that a substituent as described above is present or absent. In cases where a moiety is substituted, two or more substituents may be present within the moiety, and the substituents may be identical to or different from each other. In cases where the 3,5-diaminobenzoic acid compound according to the present invention is "optionally substituted," the number of substituents within the compound is preferably from 0 to 3, and more preferably 0.

For the "substituent", the substituent containing 0 to 12 carbon atoms is preferred. The substituent containing 0 to 6 carbon atoms is more preferred.

For the "substituent", the substituent containing 1 to 10 atoms is preferred. Examples of such substituent include, but are not limited to, halogen atom, a methyl group, an ethyl group, a vinyl group, a methoxy group, an ethoxy group, an acetyl group, a carboxyl group, a methoxycarbonyl group, a chloromethyl group, an amino group, a methylamino group, a hydroxy group, a sulfo group, and a methylthio group.

The ring A in the Formula (IV-I) is an optionally substituted monocyclic or polycyclic aromatic ring or heterocycle as described above, and preferably an optionally substituted polycyclic aromatic ring or heterocycle.

More preferably, the ring A represents a group represented by the following Formula (IV-VI):

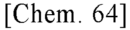

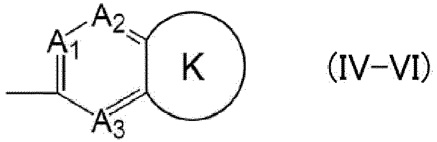

In the Formula (IV-VI), A₁, A₂, and A₃ independently represent a carbon atom or a nitrogen atom, and a ring K represents an optionally substituted monocyclic or polycyclic aromatic ring, heterocycle or cyclic hydrocarbon.

Examples of the group represented by the Formula (IV-VI) include, but are not limited to, groups having the following structure:

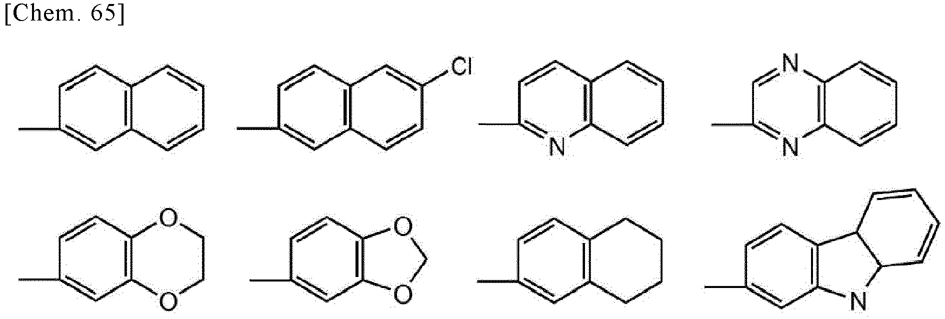

The group represented by the Formula (IV-VI) is preferably a group in which all of A₁, A₂, and A₃ represent a carbon atom. Moreover, the group represented by the Formula (IV-VI) is preferably a group in which the ring K is an optionally substituted monocyclic or polycyclic aromatic ring.

More preferably, a naphthyl group may be used as the group represented by the Formula (IV-VI).

### 1-4-3. R₄₁

In the Formula (IV-I), R₄₁ represents an optionally substituted polycyclic aromatic ring or a group represented by any of the Formulae (IV-II)-(IV-V).

The group represented by the Formula (IV-II) is a group having the following structure:

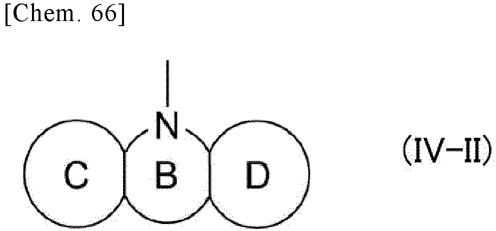

In the Formula (IV-II), a ring B represents an optionally substituted monocyclic heterocycle, rings C and D independently represent an optionally substituted monocyclic or polycyclic aromatic ring, heterocycle or cyclic hydrocarbon. The rings B, C and D form a condensed ring.

N in the ring B represents a nitrogen atom, and the ring B is attached to Y₄ via a nitrogen atom. In cases where Y₄ is a single bond, the ring B is attached to a carbonyl group (-CO-) via a nitrogen atom.

Examples of the group represented by the Formula (IV-II) include, but are not limited to, groups having the following structure:

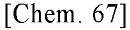

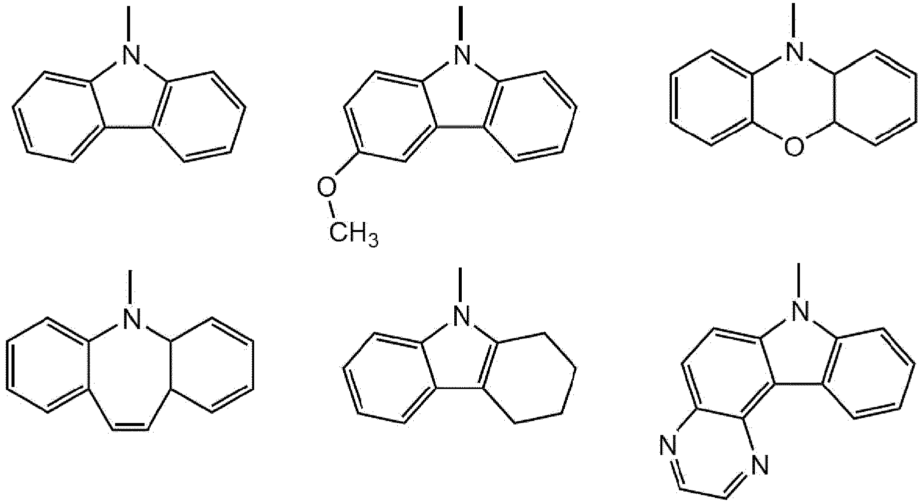

Preferably, rings C and D are optionally substituted monocyclic aromatic rings or heterocycles, and more preferably, both of them are optionally substituted monocyclic aromatic rings. In these cases, the group represented by the Formula (IV-II) is a heterocycle having three rings.

The group represented by the Formula (IV-II) is preferably an optionally substituted carbazolyl group.

The group represented by the Formula (IV-III) is a group having the following structure: 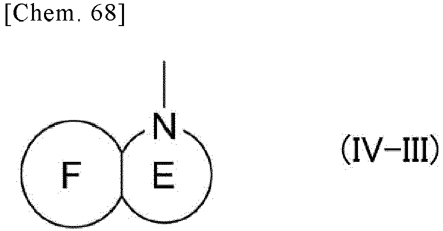 In the Formula (IV-III), a ring E represents an optionally substituted monocyclic heterocycle, and a ring F represents an optionally substituted monocyclic or polycyclic aromatic ring, heterocycle or cyclic hydrocarbon. The rings E and F form a condensed ring.

N in the ring E represents a nitrogen atom, and the ring E is attached to Y₄ via a nitrogen atom. In cases where Y₄ is a single bond, the ring E is attached to a carbonyl group (-CO-) via a nitrogen atom.

Examples of the group represented by the Formula (IV-III) include, but are not limited to, groups having the following structure:

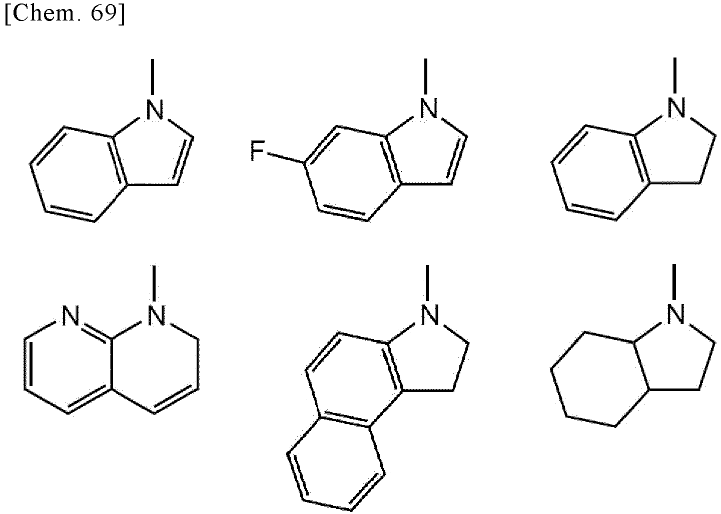

A ring F is preferably an optionally substituted monocyclic aromatic ring or a heterocycle, and more preferably an optionally substituted monocyclic aromatic ring. In these cases, the group represented by the Formula (IV-III) is a heterocycle having two rings.

The group represented by the Formula (IV-IV) is a group having the following structure:

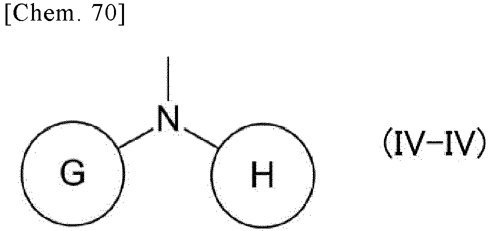

In the Formula (IV-IV), rings G and H independently represent an optionally substituted monocyclic or polycyclic aromatic ring or heterocycle.

Examples of the group represented by the Formula (IV-IV) include, but are not limited to, groups having the following structure:

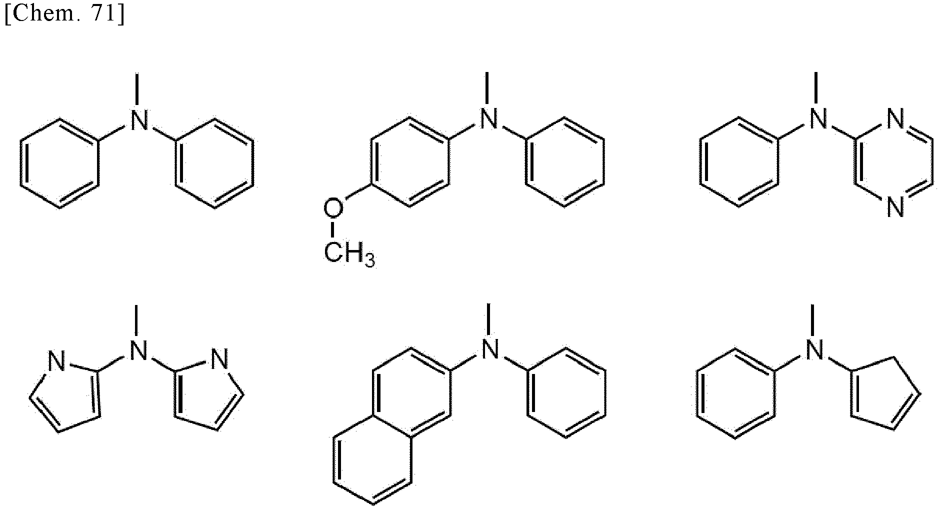

In the Formula (IV-IV), rings G and H are preferably optionally substituted monocyclic aromatic rings or heterocycles. The group represented by the Formula (IV-IV) is preferably an optionally substituted diphenylamino group.

The group represented by the Formula (IV-V) is a group having the following structure:

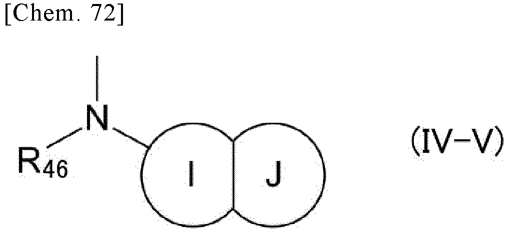

In the Formula (IV-V), a ring I represents an optionally substituted monocyclic aromatic ring or heterocycle, and a ring J represents an optionally substituted monocyclic or polycyclic aromatic ring, heterocycle or cyclic hydrocarbon. The rings I and J form a condensed ring.

In the Formula (IV-V), R₄₆ represents a hydrogen atom, an optionally substituted hydrocarbon group, or an optionally substituted heterocyclic group.

In the 3,5-diaminobenzoic acid compound according to the present invention, the "hydrocarbon group" means a group derived from a compound composed of carbon and hydrogen atoms. Examples of the hydrocarbon group can include, but are not limited to, aliphatic hydrocarbon, monocyclic saturated hydrocarbon, and aromatic hydrocarbon groups, and preferably contain 1 to 16 carbon atoms. Specific examples of the hydrocarbon group include, but are not limited to, an alkyl group, an alkenyl group, an alkynyl group, a cycloalkyl group, and an aryl group.

In this respect, examples of the "alkyl group" include a methyl group, an ethyl group, a propyl group, an isopropyl group, a butyl group, an isobutyl group, a sec-butyl group, a tert-butyl group, a pentyl group, and a hexyl group. Examples of the "alkenyl group" include a vinyl group, a 1-propenyl group, an a llyl group, an isopropenyl group, a butenyl group, and an isobutenyl group. Examples of the "alkynyl group" include an ethynyl group, a propargyl group, and a 1-propynyl group. Examples of "cycloalkyl group" include a cyclopropyl group, a cyclobutyl group, a cyclopentyl group, and a cyclohexyl group. Examples of "aryl group" include a phenyl group, an indenyl group, a naphthyl group, a fluorenyl group, an anthryl group, a biphenylenyl group, a phenanthrenyl group, an *as*-indacenyl group, an s-indacenyl group, an acenaphthylenyl group, a phenalenyl group, a fluoranthenyl group, a pyrenyl group, a naphthacenyl group, and a hexacenyl group.

In addition, in the 3,5-diaminobenzoic acid compound according to the present invention, the "heterocyclic group" refers to a group derived from a cyclic compound composed of atoms of carbon and some other elements. In the present invention, the "heterocyclic group" can be, but is not limited to, for example, any of 5- to 14-membered monocyclic to pentacyclic heterocyclic groups each having carbon atoms and further having one to four heteroatoms of one or two elements selected from nitrogen, oxygen, and sulfur. Specific examples of the heterocyclic group can include but are not limited to a 2- or 3-thienyl group, a 2- or 3-furyl group, a 1-, 2- or 3-pyrrolyl group, a 1-, 2- or 3-pyrrolidinyl group, a 2-, 4- or 5-oxazolyl group, a 3-, 4- or 5-isooxazolyl group, a 2-, 4- or 5-thiazolyl group, a 3-, 4- or 5-isothiazolyl group, a 3-, 4- or 5-pyrazolyl group, a 2-, 3- or 4-pyrazolidinyl group, a 2-, 4- or 5-imidazolyl group, a 1,2,3-triazolyl group, a 1,2,4-triazolyl group, and a 1H- or 2H-tetrazolyl group as 5-membered cyclic groups each having carbon atoms and further having one to four heteroatoms selected from oxygen, sulfur, and nitrogen. Moreover, specific examples of the heterocyclic group can include but are not limited to a 2-, 3- or 4-pyridyl group, an N-oxide-2-, 3- or 4-pyridyl group, a 2-, 4- or 5-pyrimidinyl group, an N-oxide-2-, 4- or 5-pyrimidinyl group, a thiomorpholinyl group, a morpholinyl group, a piperidino group, a 2-, 3- or 4-piperidyl group, a thiopyranyl group, a 1,4-oxazinyl group, a 1,4-thiazinyl group, a 1,3-thiazinyl group, a piperazinyl group, a triazinyl group, a 3- or 4-pyridazinyl group, a pyrazinyl group, and an N-oxide-3- or 4-pyridazinyl group as 6-membered cyclic groups each having carbon atoms and further having one to four heteroatoms selected from oxygen, sulfur, and nitrogen. Moreover, specific examples of the heterocyclic group can include but are not limited to an indolyl group, a benzofuryl group, a benzothiazolyl group, a benzoxazolyl group, a xanthenyl group, a benzimidazolyl group, a quinolyl group, an isoquinolyl group, a phthalazinyl group, a quinazolinyl group, a quinoxalinyl group, an indolizinyl group, a quinolizinyl group, a 1,8-naphthyridinyl group, a dibenzofuranyl group, a carbazolyl group, an acridinyl group, a phenanthridinyl group, a perimidinyl group, a phenazinyl group, a chromanyl group, a phenothiazinyl group, a phenoxazinyl group, and a 7H-pirazino[2,3-c]carbazolyl group as bicyclic to tetracyclic condensed ring groups each having carbon atoms and further having one to four heteroatoms selected from oxygen, sulfur, and nitrogen.

Examples of the group represented by the Formula (IV-V) can include, but are not limited to, groups having the following structure:

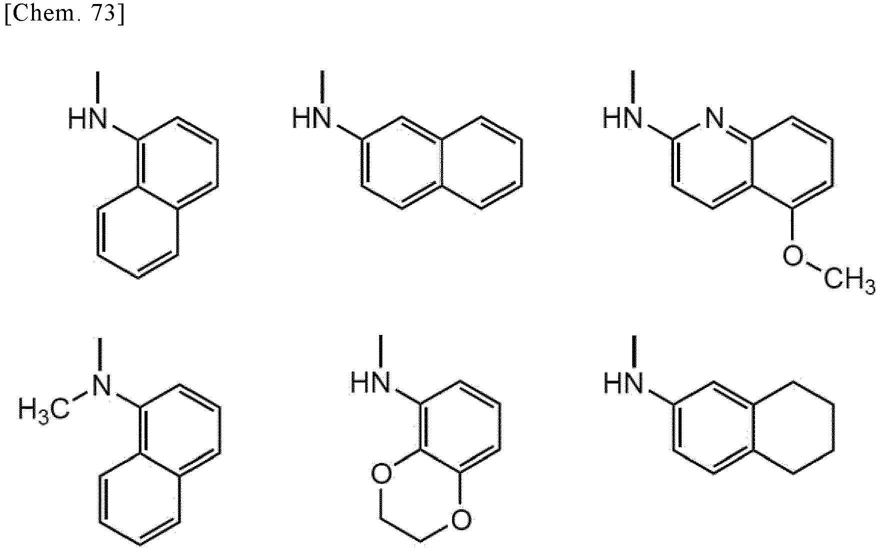

In the Formula (IV-V), a ring J is preferably an optionally substituted monocyclic aromatic ring or a heterocycle. R₄₆ is preferably a hydrogen atom.

Any groups represented by the Formulae (IV-II) - (IV-V) have two or more rings and are characterized by their attachment to Y₄ via a nitrogen atom.

In the 3,5-diaminobenzoic acid compound according to the present invention, from the viewpoint of activity of the compounds, it is preferable to use the compound having the group represented by the Formula (IV-II) or (IV-IV), and more preferable to use the group represented by the Formula IV-(II), among the groups represented by the Formulae (IV-II)-(IV-V).

### 1-4-4. R₄₂ to R₄₅

In the Formula (IV-I), R₄₂ represents a hydrogen atom, an optionally substituted hydrocarbon group, an optionally substituted heterocyclic group, or an optionally substituted amino group.

In the 3,5-diaminobenzoic acid compound according to the present invention, the "optionally substituted amino group" is a primary amino group, a secondary amino group, or a tertiary amino group. The secondary amino group may be an amino group having one substituent, and may include, but are not limited to, for example, alkylamino group, arylamino group, alkoxycarbonylamino group and the like. The tertiary amino group may be an amino group having identical or different two substituents, and may include, but not limited to, for example, dialkylamino group, diarylamino group and the like.

In the 3,5-diaminobenzoic acid compound according to the present invention, from the viewpoint of activity of the compounds, the compound in which R₄₂ is a hydrogen atom or a methyl group is preferably used, and in particular, the compound in which R₄₂ is a hydrogen atom and -CO₂R₄₂ group is a carboxyl group (-CO₂H) is preferably used. However, even if R₄₂ is an optionally substituted hydrocarbon group, an optionally substituted heterocycle group or an optionally substituted amino group, and the activity of the compound is low, the compound may be subject to hydrolysis and easily become a carboxyl group by substitution by hydrogen atoms so that the activity of the compound may be increased. Thus, such a compound can be also used as a prodrug.

In the Formula (IV-I), R₄₃ represents a hydrogen atom, an optionally substituted hydrocarbon group, or an optionally substituted heterocyclic group.

Moreover, R₄₄ represents a hydrogen atom, an optionally substituted hydrocarbon group, or an optionally substituted heterocyclic group.

In the 3,5-diaminobenzoic acid compound according to the present invention, from the viewpoint of activity of the compounds, the compound in which R₄₃ and R₄₄ are hydrogen atoms is preferably used. Such a compound can be represented by the following general formula (IV-VII):

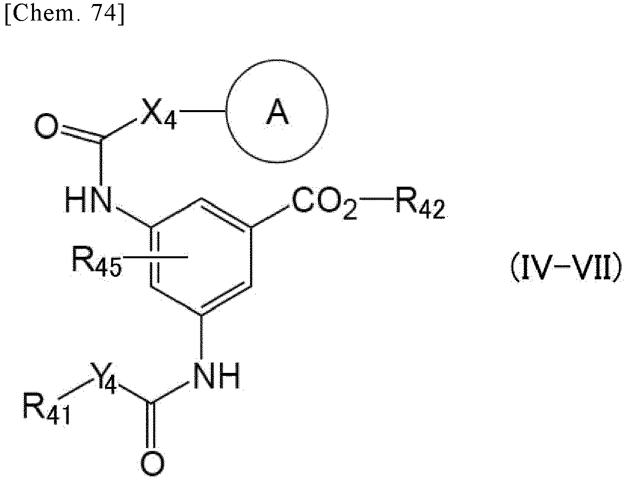

In the Formula (IV-VII), the ring A, R₄₁, R₄₂, R₄₅, X₄ and Y₄ are the same as defined in the Formula (IV-I).

In the Formula (IV-I), R₄₅ represents 0 to 3 identical or different substituents attached to a benzene ring. In this context, "different" also includes the case in which only one of three substituents is different. In the 3,5-diaminobenzoic acid compound according to the present invention, the compound having no R₄₅, namely, the compound having hydrogen atoms at positions 2, 4, and 6 of the benzene ring is preferably used.

### 1-4-5. X₄ and Y₄

In the Formula (IV-I), X₄ represents a single bond, C₁₋₂ alkylene group, -O-group, -CH₂-O- group, -CH₂-NH-CO- group, or -CH₂-NH-CO-O-CH₂- group.

In the 3,5-diaminobenzoic acid compound according to the present invention, from the viewpoint of activity of the compounds, preferably the compound in which X₄ is a single bond, -CH₂-O- group or -CH₂-NH-CO- group may be used, and more preferably the compound in which X₄ is a single bond may be used.

In the Formula (IV-I), Y₄ represents a single bond or C₁₋₂ alkylene group.

In the 3,5-diaminobenzoic acid compound according to the present invention, from the viewpoint of activity of the compounds, preferably the compound in which Y₄ is a single bond or C₁ alkylene group (methylene group) may be used, and more preferably the compound in which Y₄ is a single bond may be used.

In this context, the "single bond" refers to the state that the groups on both sides of X₄ (or Y₄) are attached directly without any linking groups.

In the 3,5-diaminobenzoic acid compound according to the present invention, from the viewpoint of activity of the compounds, in the compound represented by the Formula (IV-I), R₄₃ and R₄₄ are preferably hydrogen atoms, and more preferably X₄ and Y₄ are single bonds. Such compounds can be represented by the following general formula (IV-VIII):

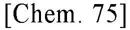

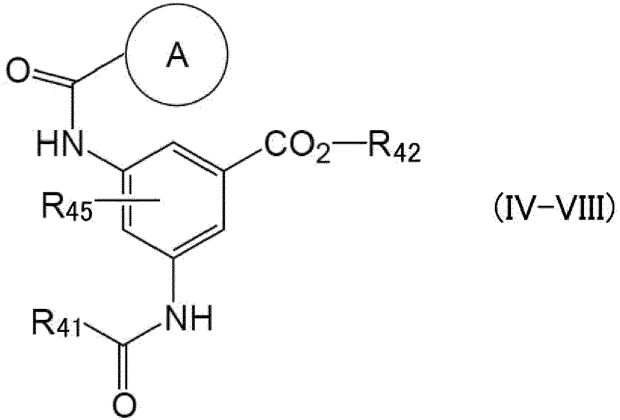

In the Formula (IV-VIII), the ring A, R₄₁, R₄₂ and R₄₅ are the same as defined in the Formula (IV-I).

### 1-4-6. Methods for Producing 3,5-diaminobenzoic acid compound

The aforementioned 3,5-diaminobenzoic acid compound can be synthesized by, for example, but not limited to, using a diaminobenzoate ester, according to the scheme shown in the following reaction flow chart (A):

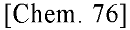

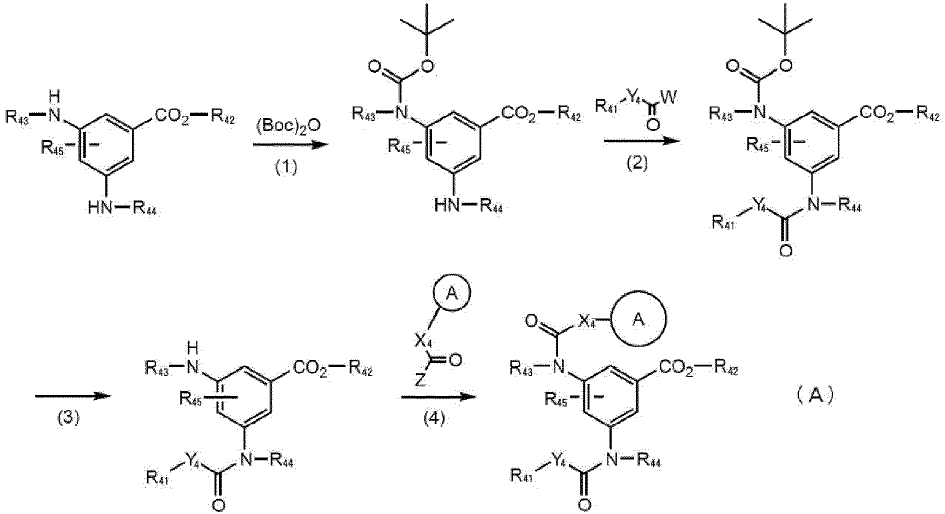

In the reaction flow chart (A), the ring A, R₄₁ to R₄₅, X₄ and Y₄ are the same as defined in the Formula (IV-I). W and Z independently represent a halogen atom or a hydroxy group.

In the reaction flow chart (A), the reaction (1) is a reaction for Boc-protecting amines, in which one amine in diaminobenzoate ester is reacted with (Boc)₂O (di-tert-butyl dicarbonate) in the presence of a base. Then, the reaction (2) is a reaction in which the unreacted other amine in the diaminobenzoate ester is reacted with an acyl halide or a carboxylic acid having R₄₁ and Y₄ to attach a group having R₄₁ and Y₄ to the amine via an amide bond. The reaction (3) is a reaction for deprotection in which a Boc group is detached under an acidic condition. Then, the reaction (4) is a reaction in which the deprotected amine is reacted with an acyl halide or a carboxylic acid having the ring A and X₄ to attach a group having the ring A and X₄ to the amine via an amide bond.

The compound obtained from the reaction flow chart (A) is the compound represented by the Formula (IV-I), and moreover, in the case of R₄₂ being not a hydrogen atom, R₄₂ may be detached in a basic condition to obtain a carboxylic acid in which R₄₂ is substituted with a hydrogen atom.

Furthermore, in the case of R₄₂ being not a hydrogen atom, R₄₂ may also be detached after the reaction (3), followed by performing the reaction (4) to obtain a carboxylic compound.

The 3,5-diaminobenzoic acid compound according to the present invention can also be synthesized by using a diaminobenzoate ester according to the scheme shown in the following reaction flow chart (B):

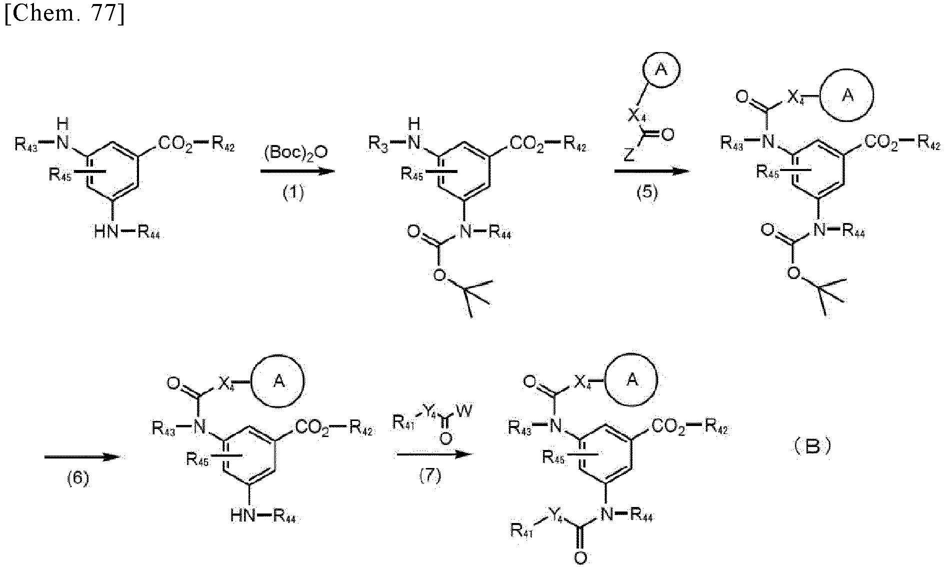

In the reaction flow chart (B), rings A, R₄₁ to R₄₅, X₄ and Y₄ are the same as defined in the Formula (IV-I). W and Z independently represent a halogen atom or a hydroxy group.

In the reaction flow chart (B), the reaction (1) is the same as the reaction (1) in the reaction flow chart (A), that is, a reaction for Boc-protecting amines, in which one amine in diaminobenzoate ester is reacted with (Boc)₂O (di-tert-butyl dicarbonate) in the presence of a base. Then, the reaction (5) is a reaction in which the unreacted other amine in the diaminobenzoate ester is reacted with an acyl halide or a carboxylic acid having rings A and X₄ to attach a group having the rings A and X₄ to the amine via an amide bond. The reaction (6) is a reaction for deprotection in which a Boc group is detached under an acidic condition. Then, the reaction (7) is a reaction in which the deprotected amine is reacted with an acyl halide or a carboxylic acid having R₄₁ and Y₄ to attach a group having R₄₁ and Y₄ to the amine via an amide bond.

The reaction (A) is different from the reaction (B) in that the reaction (A) attaches the group having R₄₁ and Y₄ first, while the reaction (B) attaches the group having the rings A and X₄ first.

The compound obtained from the reaction flow chart (B) is the compound represented by the Formula (IV-I), and moreover, in the case of R₄₂ being not a hydrogen atom, R₄₂ may be detached in a basic condition to obtain a compound of a carboxylic acid in which R₄₂ is substituted with a hydrogen atom.

Furthermore, in the case of R₄₂ being not a hydrogen atom, R₄₂ may also be detached after the reaction (6), followed by performing the reaction (7) to yield a carboxylic compound.

The reaction flow charts (A) and (B) are the reactions involving protection with a Boc group and deprotection. However, these reactions can be accomplished without protection with a Boc group. For example, the compound may be synthesized with the scheme shown in the following reaction flow chart (C):

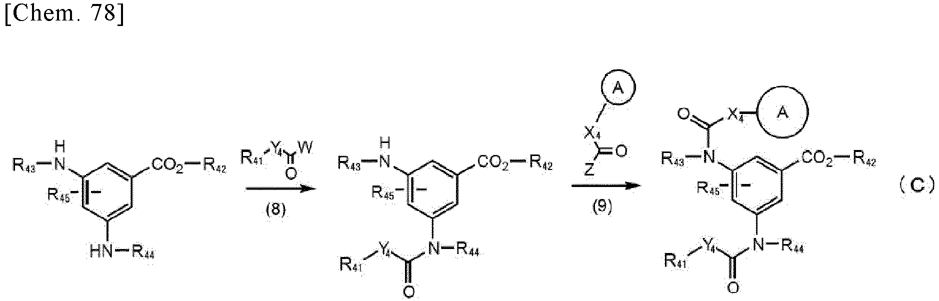

In the reaction flow chart (C), the ring A, R₄₁ to R₄₅, X and Y are the same as defined in the Formula (IV-I). W and Z independently represent a halogen atom or a hydroxy group.

In the reaction flow chart (C), the reaction (8) is a reaction in which one amine in diaminobenzoate ester is reacted with an acyl halide or a carboxylic acid having R₄₁ and Y₄ to attach a group having R₄₁ and Y₄ to the amine via an amide bond. The reaction (9) is a reaction is a reaction in which the unreacted other amine in the diaminobenzoate ester is reacted with an acyl halide or a carboxylic acid having rings A and X₄ to attach a group having the rings A and X₄ to the amine via an amide bond.

The reaction flow chart (C) is a synthesis method in which the group having R₄₁ and Y₄ is first attached as with the reaction flow chart (A). Although the purity is lower because the protection with a Boc group is not performed, it is possible to synthesize it at sufficiently high purity by exploring the reaction conditions.

The compound obtained from the reaction flow chart (C) is the compound represented by the Formula (IV-I), and moreover, in the case of R₄₂ being not a hydrogen atom, R₄₂ may be detached in a basic condition to obtain a carboxylic acid in which R₄₂ is substituted with a hydrogen atom.

Furthermore, in the case of R₄₂ being not a hydrogen atom, R₄₂ may also be detached after the reaction (8), followed by performing the reaction (9) to yield a carboxylic compound.

### 1-5. Other Pin1 Inhibitors

The compounds shown in the following table or salts thereof can also be used as the compound that is an active ingredient of the therapeutic or prophylactic agent for coronavirus disease according to present invention:

**[Table 1]**

| Compound No. | Structural Formula | Reference |
|---|---|---|
| PA-1 | 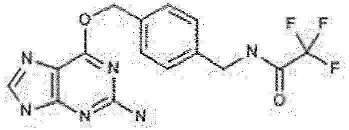 | CN 107098906 |
| PA-2 | 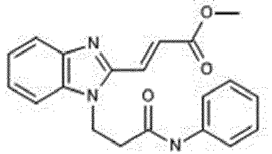 | CN 106632065 |
| PA-3 | 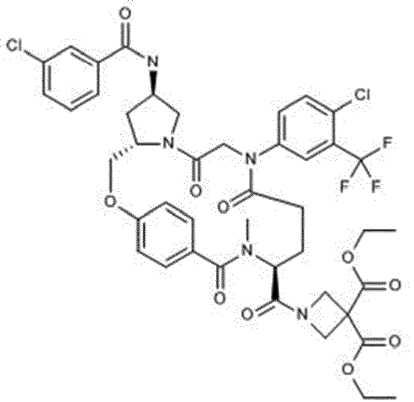 | WO |
| | | 2017/063757 |
| PA-4 | 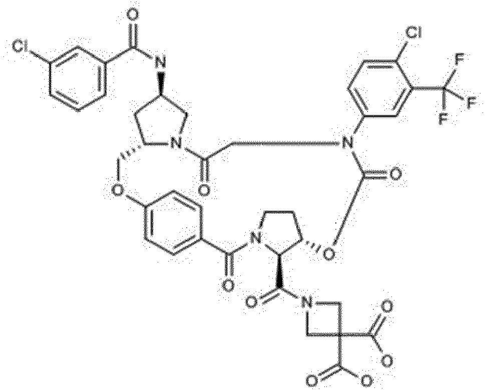 | WO |
| | | 2017/063756 |
| PA-5 | 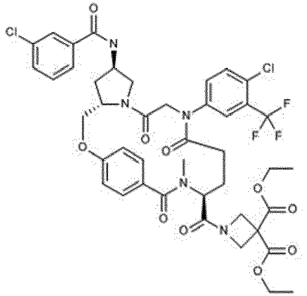 | WO |
| | | 2017/063755 |
| PA-6 | 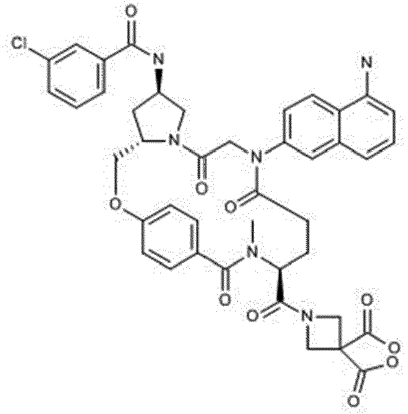 | WO |
| | | 2017/063754 |
| PA-7 | 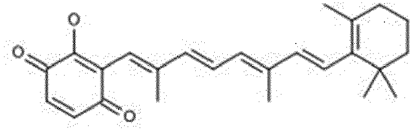 | WO |
| | | 2016/145186 |
| PA-8 | 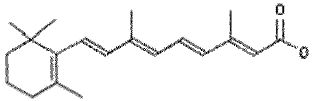 | WO |
| | | 2016/011268 |
| PA-9 | 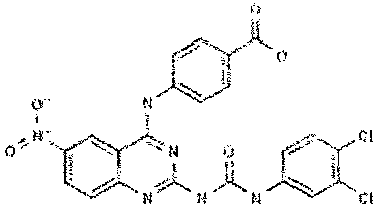 | CN 102250075 |
| PA-10 | 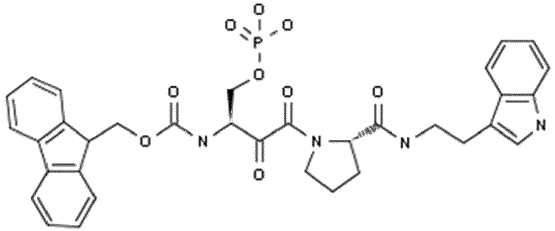 | WO |
| | | 2006/124494 |
| PA-11 | 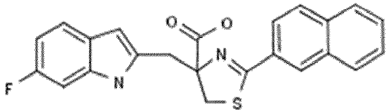 | WO |
| | | 2006/040646 |
| PA-1 2 | 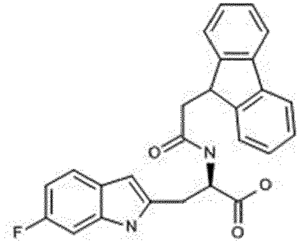 | WO |
| | | 2006/040646 |
| PA-13 | 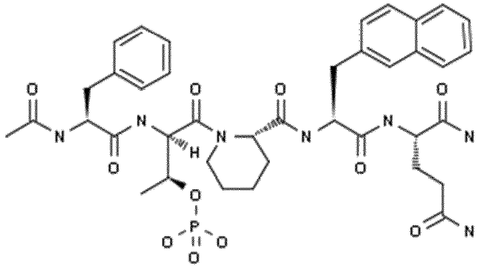 | WO |
| | | 2006/019982 |
| PA-14 | 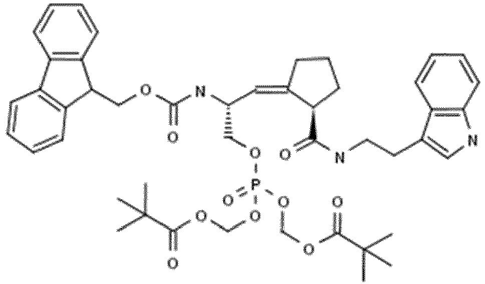 | WO |
| | | 2006/020417 |
| PA-1 5 | 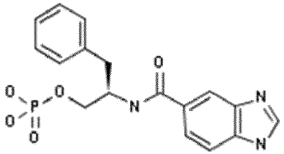 | US 2005250742 |
| PA-16 | 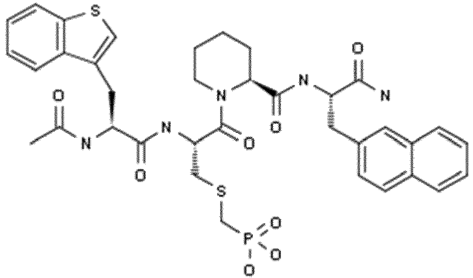 | WO |
| | | 2005/063259 |
| PA-17 | 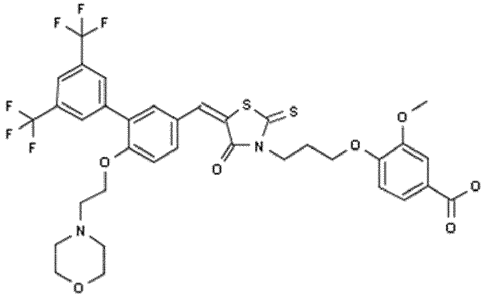 | WO |
| | | 2005/007123 |
| PA-1 8 | 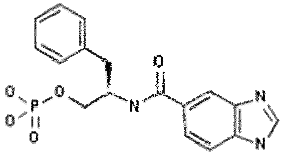 | WO |
| | | 2004/087720 |
| PA-19 | 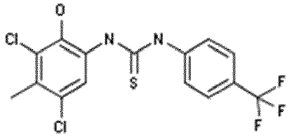 | WO |
| | | 2004/026815 |
| PA-20 | 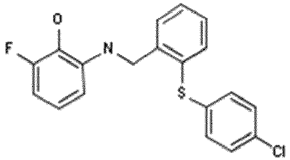 | WO |
| | | 2004/026815 |
| PA-21 | 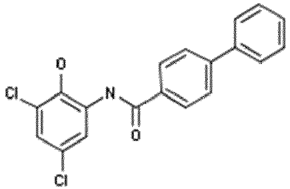 | WO |
| | | 2004/026815 |
| PA-22 | 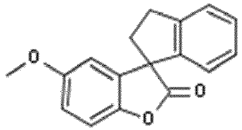 | WO |
| | | 2003/093258 |
| PA-23 | 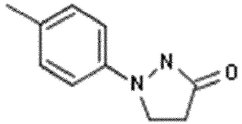 | WO |
| | | 2003/074550 |
| PA-24 | 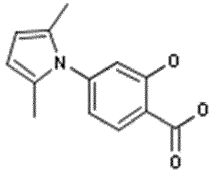 | WO |
| | | 2003/073999 |
| PA-25 | 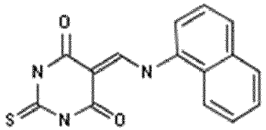 | WO |
| | | 2003/074497 |
| PA-26 | | 232nd Am Chem Soc (ACS) Natl Meet 2006 |
| PA-27 | 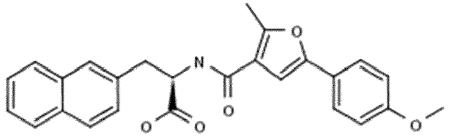 | Bioorg Med Chem Lett 2010, 20(2): 586 |
| PA-28 | 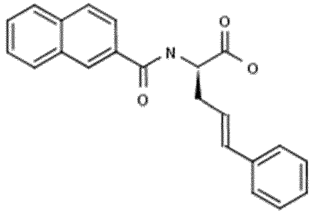 | Bioorg Med Chem Lett 2010, 20(7): 2210 |
| PA-29 | 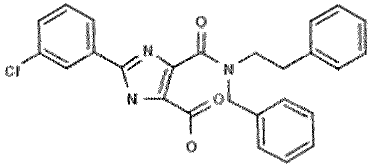 | Bioorg Med Chem Lett 2010, 20(22): 6483 |
| PA-30 | 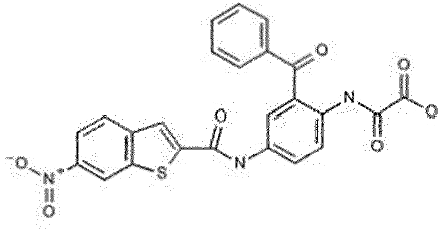 | Bioorg Med Chem 2012, 20(9): 2992 |
| PA-31 | 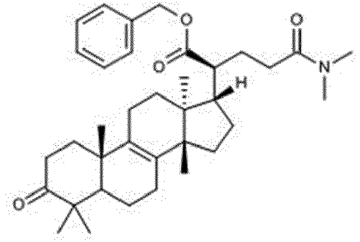 | Bioorg Med Chem Lett 2014, 24(24): 5612 |
| PA-32 | 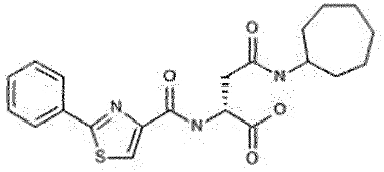 | Bioorg Med Chem Lett 2015, 25(23): 5619 |
| PA-33 | 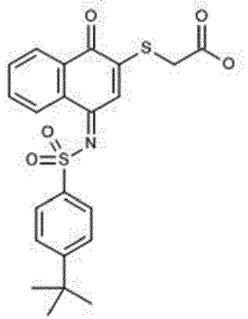 | Nat Commun 2017, 8: 15772 |

### 2. Inhibitory Activity against the Function of Pin1

The compound that is an active ingredient of the present invention is a compound that has inhibitory activity against the function of Pin1. For example, the compounds that have a chemical structure represented by the Formula (I-I), (II-I), (III-I), (IV-I), or Table 1, or salts thereof can be used as the aforementioned compound.

In the present invention, the phrase "inhibit the function of Pin1" means: inhibiting the isomerase activity of Pin1 and/or the activity of Pin1 to associate or interact with another protein, such as IRS-1; and/or facilitating decomposition of the Pin1 enzyme.

In order to examine whether the compound as an active ingredient has inhibitory activity against the function of Pin1, the activity of a Pin1 inhibitor according to the present invention to inhibit the function of Pin1 can be measured by, for example, but not limited to, examining AMPK (AMP-activated protein kinase) phosphorylation level as an index (see Yusuke Nakatsu et al., Journal of Biological Chemistry, 2015, Vol. 290, No. 40, pp. 24255-24266). Alternatively, the activity of a Pin1 inhibitor according to the present invention to inhibit the function of Pin1 can be measured by detecting a change in the isomerase activity of Pin1 against a peptide substrate as a change in absorbance (see Hailong Zhao et al., Bioorganic & Medicinal Chemistry, 2016, Vol. 24, pp. 5911-5920). Alternatively, the activity of a Pin1 inhibitor according to the present invention to inhibit the function of Pin1 can also be measured by detecting the association of the inhibitor with Pin1, which competes with the association of Pin1 with a peptide substrate (see Shuo Wei et al., Nature Medicine, Vol. 21, No. 5, pp. 457-466, online methods).

### 3. Pharmaceutically Acceptable Salt

The therapeutic or prophylactic agents for viral diseases according to the present invention contain the compound that inhibits the function of Pin1 or pharmaceutically acceptable salt thereof as an active ingredient. The compound that inhibits the function of Pin1 includes, for example, a compound that has a chemical structure represented by the Formula (I-I), (II-I), (III-I), (IV-I), or Table 1.

Examples of the "pharmaceutically acceptable salt" can include, but are not limited to, sodium, potassium, and ammonium salts. Additionally, in cases where the compound has a basic functional group in the molecule, examples of a pharmaceutically acceptable salt of the compound can include hydrochloride, phosphate, acetate, phthalate, fumarate, and oxalate salts.

### 4. Applicable Disease

"Viral diseases", which are indications of the therapeutic or prophylactic agent according to the present invention, are diseases caused by viruses, including, e.g., a coronavirus infection caused by coronaviruses.

Coronaviruses that infect humans include alpha-coronaviruses (HCoV-229E and HCoV-NL63) and beta-coronaviruses (MERS-CoV, SARS-CoV, SARS-CoV-2, HCoV-OC43 and HCoV-HKU1). HCoV-229E, HCoV-OC43, HCoV-NL63 and HCoV-HKU1 are causes of common cold, mostly with mild symptoms, however sometimes accompanied by high fever. It is thought that SARS-CoV is derived from a bat-borne coronavirus, which infects human and causes severe pneumonia, while MERS-CoV is a virus that causes cold symptoms in dromedary camels, but causes severe pneumonia if it jumps the species barrier to infect humans. The infectious disease caused by SARS-CoV-2 (COVID-19) mainly spreads with human-to-human transmission via through airborne droplets expelled by coughs and sneezes of an infected person. COVID-19 causes fever, respiratory symptoms, headache, fatigue and the like and can also cause olfactory or taste disorders. The therapeutic or prophylactic agent according to the present invention is especially preferred to be applied to a coronavirus infection caused by beta-coronavirus, and more preferably applied to a coronavirus infection caused by SARS-CoV-2 (COVID-19).

The compounds serving as an active ingredient in the present invention have an effect of inhibiting the function of Pin1 as their mechanism of action and can suppress viral proliferation so that they can be used as a therapeutic or prophylactic agent for viral diseases. The therapeutic or prophylactic agent for viral diseases according to the present invention can be administered as a therapeutic agent or a prophylactic agent not only to patients diagnosed as having a viral disease, but also to patients possibly having a viral disease and patients at risk of developing a viral disease. In addition, the therapeutic or prophylactic agent for viral diseases according to the present invention suppresses proliferation of SARS-CoV-2 virus so that it can be preferably used particularly to treat or prevent coronavirus infection caused by SARS-CoV-2. Namely, the present invention can provide a method of treating or preventing COVID-19, the method comprising administrating a therapeutically effective amount of a Pin1 inhibitor (including those identified by the screening method of Example 6), or a pharmaceutically acceptable salt thereof, having the function as a therapeutic or prophylactic agent for a viral disease, to the subjects in need thereof, e.g., patients suffering from COVID-19, medical workers who have frequent contact with COVID-19 patients or SARS-CoV-2, service workers who serve many people, and the like.

### 5. Dosage Form

The therapeutic or prophylactic agents for viral diseases according to the present invention can be prepared by combining a compound as an active ingredient or a pharmaceutically acceptable salt thereof and a pharmaceutically acceptable carrier to form a pharmaceutical composition, and may be made in the form of, for example, but not limited to, tablets, granules, capsules, powders, liquids, injection solutions, suppositories, patches, eye drops, and inhalants. Preferred dosage forms include: for example, tablets, granules, capsules, powders, and liquids for oral administration.

As a pharmaceutically acceptable carrier which can be used in the therapeutic or prophylactic agents for viral diseases according to the present invention, various inorganic or organic carrier materials can be used. When the pharmaceutical composition is prepared in solid formulation, such as a tablet or a granule, an excipient, a lubricant, a binder, a disintegrator, and the like can be used. When the pharmaceutical composition is prepared in liquid formulation, such as a liquid or an injection solution, a solvent, a solubilizing agent, a suspending agent, a buffering agent, and the like can be used. Moreover, additives such as antioxidant, antiseptic agent, and coloring agent can also be used as necessary.

Non-limiting examples of an excipient that can be used include lactose, D-mannitol, and starch; non-limiting examples of a lubricant that can be used include magnesium stearate and talc; non-limiting examples of a binder that can be used include crystalline cellulose and gelatin; non-limiting examples of a disintegrator that can be used include carboxymethyl cellulose. Moreover, examples of a solvent that can be used include distilled water, alcohols, and propylene glycol; examples of a solubilizing agent that can be used include polyethylene glycol and ethanol; examples of a suspending agent that can be used include stearyl triethanolamine and sodium lauryl sulfate; examples of a buffering agent that can be used include phosphate and acetate salts.

The therapeutic or prophylactic agent for viral diseases according to the invention may be administered to a patient, e.g., preferably at a daily dose of 0.01 to 100 mg, more preferably 0.1 to 10 mg of active ingredient per kg of patient's body weight.

The therapeutic or prophylactic agent for coronavirus diseases according to the present invention may contain, in addition to the compound according to the present invention or pharmaceutically acceptable salts thereof, one or more active ingredients of at least one or more drugs selected from drugs classified as therapeutic or prophylactic agents for coronavirus diseases. Examples of such an active ingredient which can be used include, but not limited to, remdesivir, favipiravir, chloroquine, hydroxychloroquine, nafamostat, interferon and the like. The therapeutic or prophylactic agent for viral diseases according to the invention can be used in combination with other therapeutic or prophylactic agents for viral diseases.

Now, the present invention will be described in detail by reference to Examples, but the present invention is not limited thereto.

### EXAMPLES

### 6. Examples

### 6-1. Example 1: Effect of Pin1 on SARS-CoV-2 Proliferation

In this Example, in order to investigate the effect of Pin1 on SARS-CoV-2 proliferation, SARS-CoV-2 proliferation was observed in VeroE6/TMPRSS2 cells transduced with siRNA targeting Pin1. VeroE6 cells overexpressing TMPRSS2 (hereinafter referred to as "VeroE6/TMPRSS2 cell") (Shutoku Matsuyama and other 17 authers, PNAS, March 31, 2020, Vol. 117, p. 7001-7003) were transfected with the following two types of Pin1 siRNAs (SEQ ID No. 1 and SEQ ID No. 2) or a control siRNA at a final concentration of 20 nM by reverse transfection.
SEQ ID No. 1: cggcaacagc agcaguggug gcaaa
SEQ ID No. 2: gcccuggagc ugaucaacgg cuaca

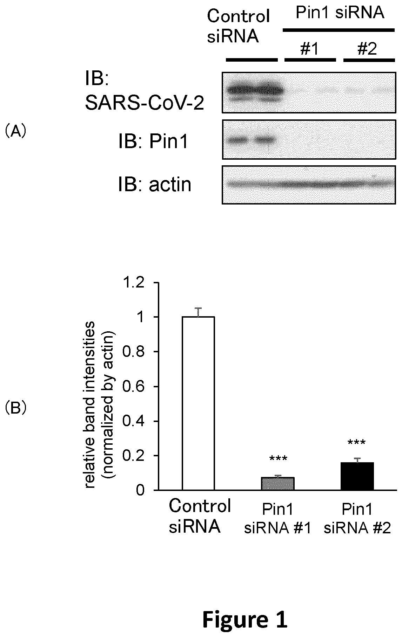

After 3 days, SARS-CoV-2 was infected to these cells at multiplicity of infection (MOI) of 0.01, followed by recovering cell lysate 24 hours after the infection and then detecting SARS-CoV-2 nucleocapsid, Pin1, and actin as an internal standard in the cells by Western blotting. SARS-CoV-2 proliferation was evaluated on the basis of the expression level of SARS-CoV-2 nucleocapsid in the cells. FIG. 1 (A) shows results of Western blotting, and (B) shows the relative intensity of SARS-CoV-2 nucleocapsid normalized to the detection intensity of SARS-CoV-2 nucleocapsid in control siRNA. As shown in Figs. 1, both of the two types of Pin1 siRNAs significantly reduced the expression level of the Pin1 protein. In addition, in the cells transfected with the two types of Pin1 siRNAs, the detection intensity of SARS-CoV-2 nucleocapsid was significantly reduced compared to the control siRNA. Thus, it was confirmed that the proliferation of SARS-CoV-2 can be suppressed by inhibiting the expression of Pin1.

### 6-2-1. Example 2-1: Production of Pin1 Inhibitor (H-77)

In this Example, an anthranilic acid compound was produced. First, intermediates (H-122 and H-64) used to synthesize the compound according to the present invention were produced. The known compound (H-122) represented by the following structural formula was synthesized in two steps according to the method described in J. Org. Chem., 2001, vol.66, pp.2784-2788 using anthranilic acid as a raw material.

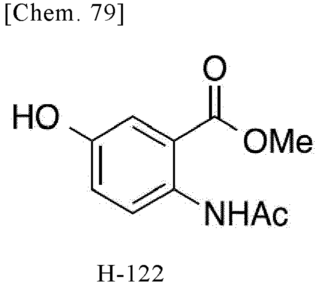

The known compound (H-64) represented by the following structural formula was synthesized in four steps according to the method described in J. Org. Chem., 2001, vol.66, pp.2784-2788 using anthranilic acid as a raw material.

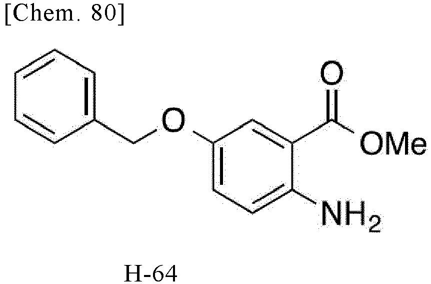

Triethylamine (316 mg, 0.44 mL, 3.12 mmol) was added to a solution of H-64 (400 mg, 1.56 mmol) and 2-naphthoyl chloride (327 mg, 1.72 mmol) in dichloromethane (5 mL) at room temperature and stirred for 13 hours at the same temperature. Water was added to the mixture, and the mixture was extracted with ethyl acetate. The organic layer was washed with saturated saline solution, dried over anhydrous magnesium sulfate, and then filtered and concentrated under reduced pressure. The residue was purified by silica gel column chromatography (chloroform: ethyl acetate, 15:1) to give H-68 as white crystals (531 mg, 1.29 mmol, 83%).

The measured NMR spectrum and HR-ESI-MS result of H-68 are as follows. ¹H NMR (400 MHz, CDCl₃) δ 3.99 (3H, s), 5.11 (2H, s), 7.28 (1H, dd, J = 9.2, 3.2 Hz), 7.32-7.48 (5H, m), 7.54-7.62 (2H, m), 7.70 (1H, d, J = 3.2 Hz), 7.88-7.93 (1H, m), 7.97 (1H, d, J = 8.8 Hz), 8.01-8.05 (1H, m), 8.09 (1H, dd, J = 8.7, 1.8 Hz), 8.57 (1H, bs), 8.92 (1H, d, J = 9.2 Hz), 12.0 (1H, bs); HRESIMS calcd for C₂₆H₂₂NO₄ [M+H]⁺ 412.1549, found 412.1550.

The identified chemical structure of H-68 is as follows:

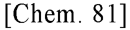

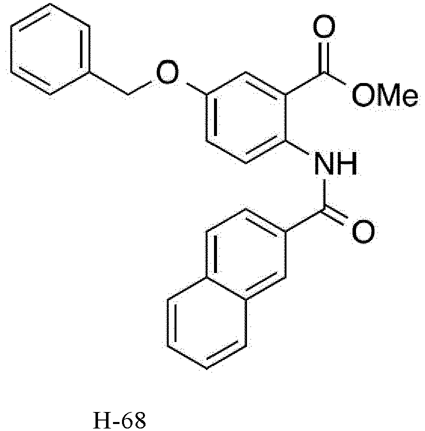

Lithium hydroxide aqueous solution (1 M, 2.2 mL, 2.2 mmol) was added to a solution of H-68 (300 mg, 0.73 mmol) in THF (8 mL) at room temperature and stirred for 4 hours at the same temperature. The mixture was neutralized with 1M hydrochloric acid and extracted with ethyl acetate. The organic layer was washed with saturated saline solution, dried over anhydrous magnesium sulfate, and then filtered and concentrated under reduced pressure to give H-77 as white crystals (280 mg, 0.705 mmol, 97%).

The measured NMR spectrum and HR-ESI-MS result of H-77 are as follows. ¹H NMR (400 MHz, DMSOd₆) δ 5.16 (2H, s), 7.31-7.43 (4H, m), 7.45-7.49 (2H, m), 7.60-7.69 (3H, m), 7.99 (1H, dd, J = 8.7, 1.8 Hz), 8.00-8.05 (1H, m), 8.06-8.12 (2H, m), 8.55 (1H, bs), 8.61 (1H, d, J = 9.1 Hz), 12.0 (1H, bs); HRESIMS calcd for C₂₅H₁₉NO₄Na [M+Na]⁺ 420.1212, found 420.1218.

The identified chemical structure of H-77 is as follows:

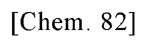

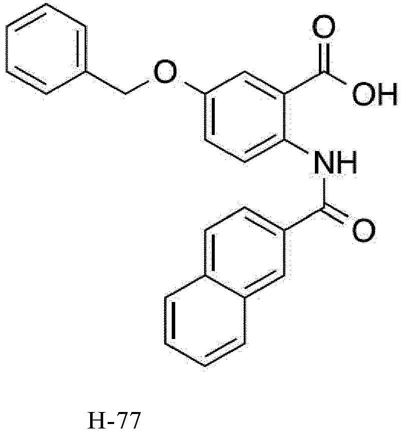

### 6-2-2. Example 2-2: Production of Other Pin1 Inhibitors

Other anthranilic acid compounds used in Example 3 were synthesized by using the production method described in WO 2019/031472 and the known methods. Amide compounds used in Example 3 were synthesized by using the production method described in WO 2019/031470 and the known methods. Carboxylic-acid-derivative compounds used in Example 3 were synthesized by using the production method described in WO 2019/031471 and the known methods.

### 6-3-1. Suppression of SARS-CoV-2 Proliferation by Pin1 Inhibitor

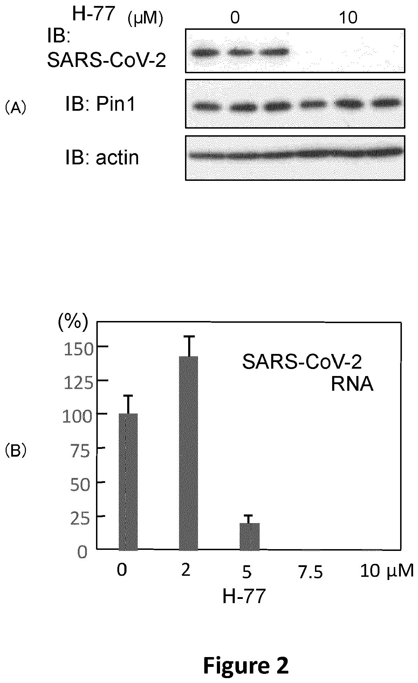

In this Example, the inhibitory effect of SARS-CoV-2 proliferation by the Pin1 inhibotor H-77. First, H-77 produced in Example 2-1 was added to VeroE6 / TMPRSS2 cells to the final concentration of 10 µM, to which SARS-CoV-2 was infected at multiplicity of infection (MOI) of 10 after 2 hours, followed by recovering cell lysate 8 hours after the infection and then detecting SARS-CoV-2 nucleocapsid, Pin1 and actin as an internal standard in the cells by Western blotting. FIG. 2 (A) shows results of Western blotting.

Also, H-77 was added to VeroE6/TMPRSS2 cells to the final concentration of 0, 2, 5, 7.5, and 10 µM, to which SARS-CoV-2 was infected at multiplicity of infection (MOI) of 0.01 after 2 hours, followed by recovering culture supernatant 24 hours after the infection and then quantifying the SARS-CoV-2 RNA level in the supernatant by real-time PCR. FIG. 2 (B) shows results of quantification, in which the proliferation of SARS-CoV-2 was suppressed by adding 5 µM of H-77, and in particular, the proliferation of SARS-CoV-2 was substantially suppressed by adding 7.5 µM of H-77.

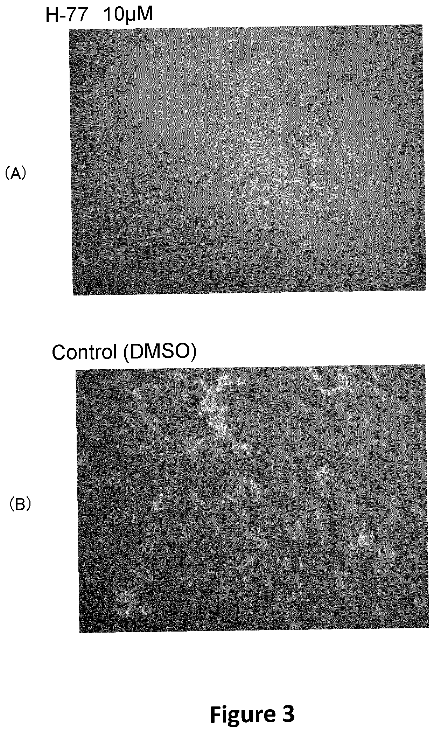

FIGs. 3 (A) and (B) show photographs of the cells infected with SARS-CoV-2. In FIG. 3 (A), H-77 was added to VeroE6/TMPRSS2 cells at the final concentration of 10 µM, then the cells were infected with SARS-CoV-2 at multiplicity of infection (MOI) of 0.01 to 0.02 after 2 hours, followed by taking a photograph 20 hours after infection. FIG. 3 (B) is a photograph similar to that in FIG. 3 (A) except that DMSO was added as a control instead of H-77.

### 6-3-2. Examples 3-2 to 3-108

Table 2 shows results of the confirmation test of a suppressing effect of various Pin1 inhibitors against SARS-CoV-2 proliferation. In the confirmation test, a Pin1 inhibitor was added to VeroE6 / TMPRSS2 cells to the final concentration of 20 µM, to which SARS-CoV-2 was infected at multiplicity of infection (MOI) of 10 after 2 hours, followed by recovering cell lysate 8 hours after the infection and then detecting SARS-CoV-2 nucleocapsid, Pin1 and actin as an internal standard in the cells by Western blotting. The inhibition rate was determined by defining the band area when Pin1 inhibitor was not added as 0 % inhibition, then calculating the comparative ratio of the band area when Pin1 inhibitor was added under the same test condition. When a band area is 0 (when no band is observed), the inhibition rate is 100 %. In Table 2, the "Class" means the system of the Pin1 inhibitors. Examples 3-2 to 3-16 are the Pin1 inhibitors of the anthranilic acid compound (the Formula (I-I)), Examples 3-17 to 3-23 are the Pin1 inhibitors of the amide compound (the Formula (II-I)) and are simultaneously the Pin1 inhibitors of the carboxylic-acid-derivative compound (the Formula (III-I)). Examples 3-24 to 3-56 are the Pin1 inhibitors of the carboxylic-acid-derivative compound (the Formula (III-I)). Examples 3-57 to 3-75 are the Pin1 inhibitors of the 3,5-diaminobenzoic acid compound (the Formula (IV-I)). Examples 3-76 to 108 are the Pin1 inhibitors that are not encompassed by these Formulae.

**[Table 2]**

| Ex No. | Comp. No. | Inhibition Rate | Structural Formula of Pin 1 Inhibitor | Class |
|---|---|---|---|---|
| 3-2 | H-77 | 96 | 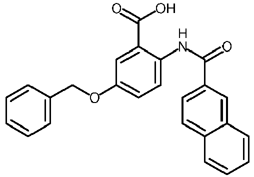 | (I-I) |
| 3-3 | H-338 | 99 | 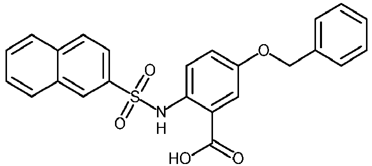 | (I-I) |
| 3-4 | H-339 | 76 | 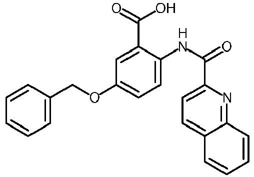 | (I-I) |
| 3-5 | H-362 | 99 | 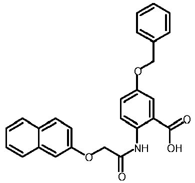 | (I-I) |
| 3-6 | H-363 | 99 | 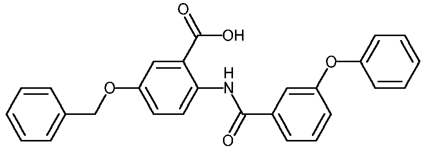 | (I-I) |
| 3-7 | H-370 | 98 | 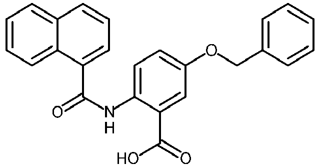 | (I-I) |
| 3-8 | H-371 | 99 | 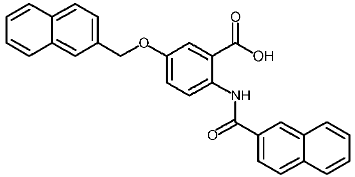 | (I-I) |
| 3-9 | H-429 | 99 | 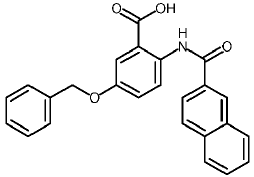 | (I-I) |
| 3-10 | H-487 | 63 | 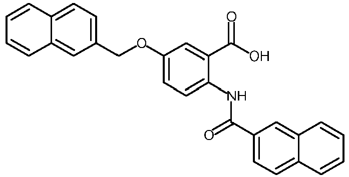 | (I-I) |
| 3-11 | H-537 | 96 | 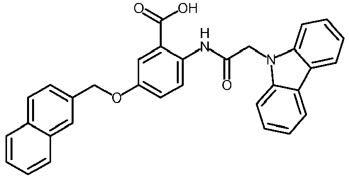 | (I-I) |
| 3-12 | H-787 | 97 | 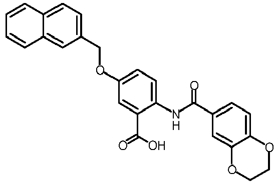 | (I-I) |
| 3-13 | H-800 | 91 | 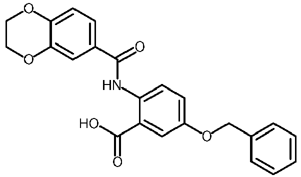 | (I-I) |
| 3-14 | H-835 | 99 | 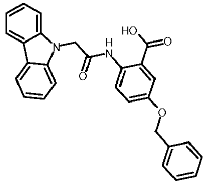 | (I-I) |
| 3-15 | H-839 | 83 | 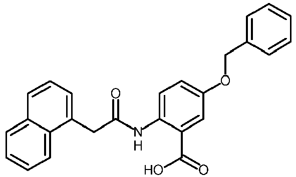 | (I-I) |
| 3-16 | H-103 | 100 | 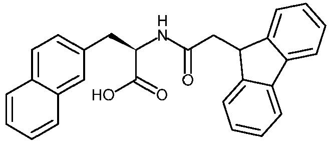 | (II-I) (III-I) |
| 3-17 | H-105 | 100 | 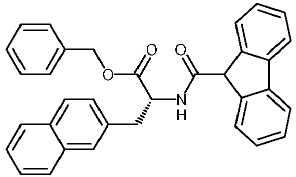 | (II-I) (III-I) |
| 3-18 | H-162 | 99 | 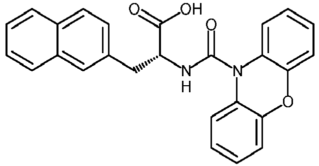 | (II-I) (III-I) |
| 3-19 | H-393 | 80 | 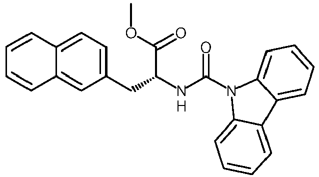 | (II-I) (III-I) |
| 3-20 | H-509 | 98 | 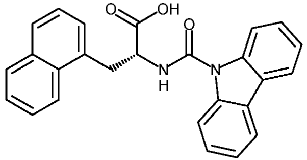 | (II-I) (III-I) |
| 3-21 | H-536 | 75 | 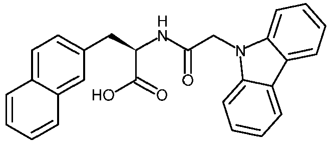 | (II-I) (III-I) |
| 3-22 | H-853 | 99 | 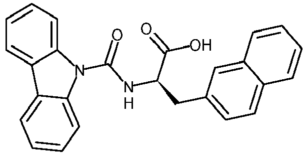 | (II-I) (III-I) |
| 3-23 | H-30 | 99 | 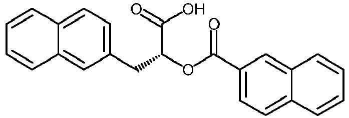 | (III-I) |
| 3-24 | H-32 | 86 | 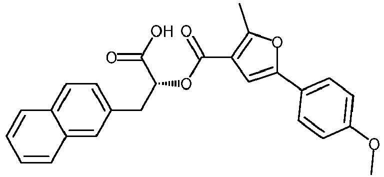 | (III-I) |
| 3-25 | H-33 | 100 | 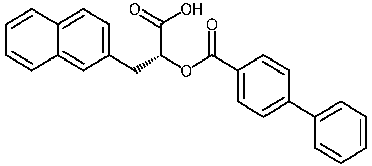 | (III-I) |
| 3-26 | H-95 | 95 | 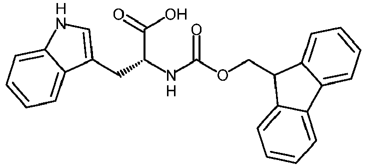 | (III-I) |
| 3-27 | H-106 | 100 | 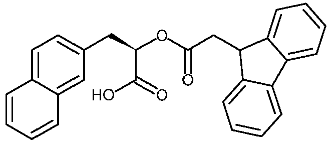 | (III-I) |
| 3-28 | H-123 | 99 | 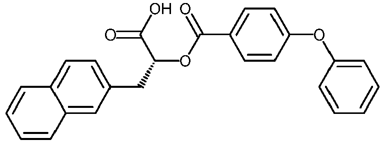 | (III-I) |
| 3-29 | H-130 | 100 | 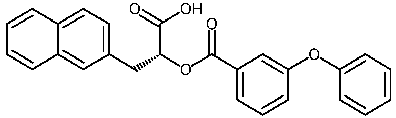 | (III-I) |
| 3-30 | H-132 | 99 | 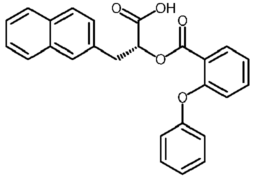 | (III-I) |
| 3-31 | H-134 | 98 | 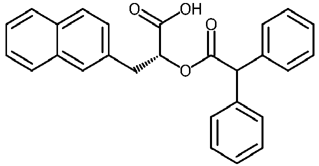 | (III-I) |
| 3-32 | H-149 | 99 | 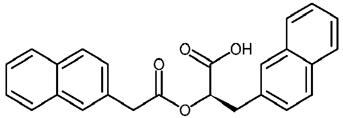 | (III-I) |
| 3-33 | H-151 | 98 | 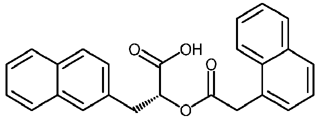 | (III-I) |
| 3-34 | H-157 | 100 | 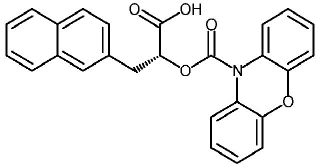 | (III-I) |
| 3-35 | H-175 | 100 | 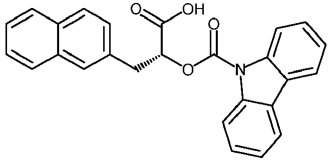 | (III-I) |
| 3-36 | H-177 | 99 | 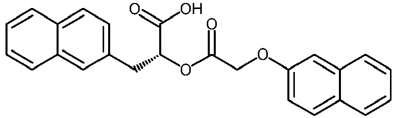 | (III-I) |
| 3-37 | H-179 | 97 | 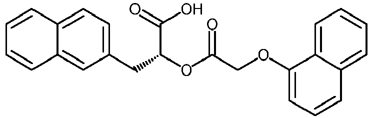 | (III-I) |
| 3-38 | H-181 | 99 | 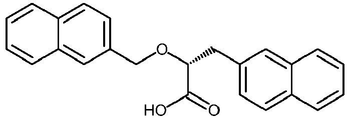 | (III-I) |
| 3-39 | H-198 | 67 | 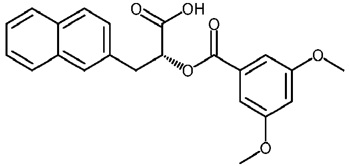 | (III-I) |
| 3-40 | H-200 | 98 | 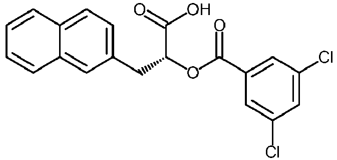 | (III-I) |
| 3-41 | H-210 | 99 | 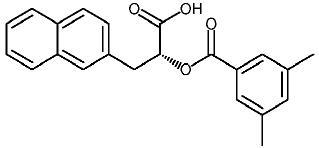 | (III-I) |
| 3-42 | H-248 | 88 | 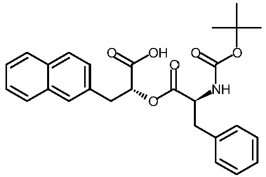 | (III-I) |
| 3-43 | H-254 | 94 | 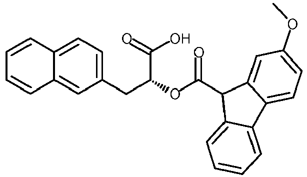 | (III-I) |
| 3-44 | H-262 | 94 | 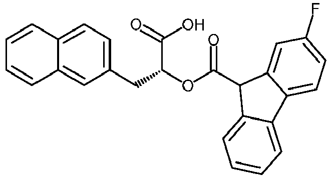 | (III-I) |
| 3-45 | H-263 | 54 | 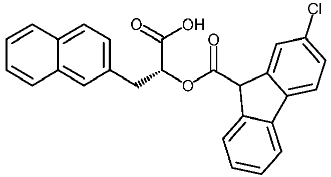 | (III-I) |
| 3-46 | H-269 | 98 | 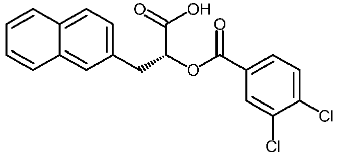 | (III-I) |
| 3-47 | H-438 | 99 | 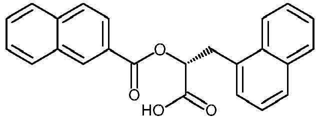 | (III-I) |
| 3-48 | H-452 | 52 | 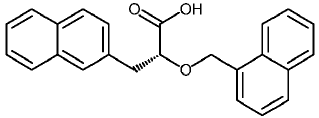 | (III-I) |
| 3-49 | H-464 | 100 | 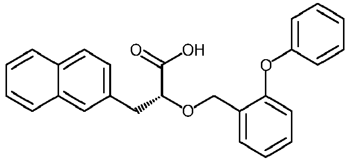 | (III-I) |
| 3-50 | H-507 | 99 | 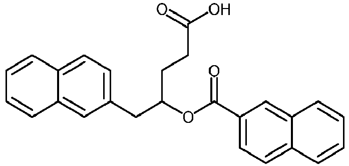 | (III-I) |
| 3-51 | H-512 | 90 | 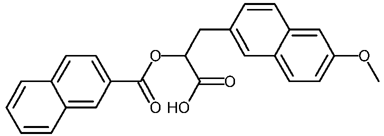 | (III-I) |
| 3-52 | H-514 | 99 | 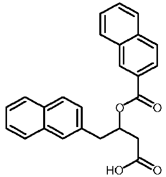 | (III-I) |
| 3-53 | H-614 | 96 | 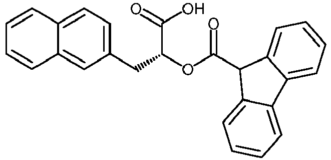 | (III-I) |
| 3-54 | H-596 | 99 | 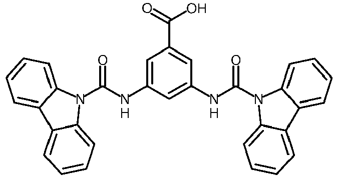 | (IV-I) |
| 3-55 | H-680 | 53 | 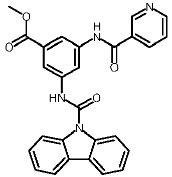 | (IV-I) |
| 3-56 | H-684 | 66 | 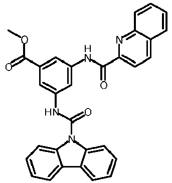 | (IV-I) |
| 3-57 | H-685 | 58 | 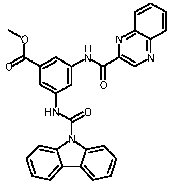 | (IV-I) |
| 3-58 | H-688 | 98 | 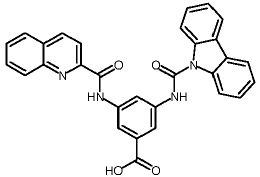 | (IV-I) |
| 3-59 | H-691 | 52 | 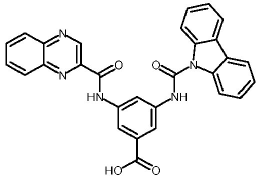 | (IV-I) |
| 3-60 | H-693 | 77 | 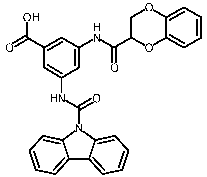 | (IV-I) |
| 3-61 | H-762 | 83 | 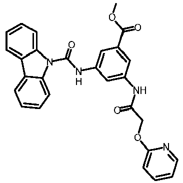 | (IV-I) |
| 3-62 | H-763 | 97 | 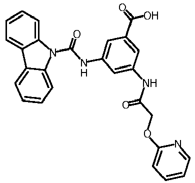 | (IV-I) |
| 3-63 | H-819 | 95 | 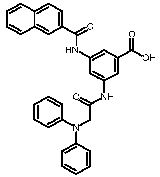 | (IV-I) |
| 3-64 | H-821 | 95 | 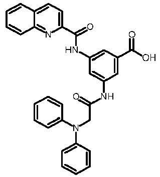 | (IV-I) |
| 3-65 | H-857 | 55 | 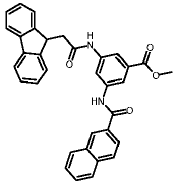 | (IV-I) |
| 3-66 | H-859 | 89 | 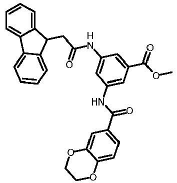 | (IV-I) |
| 3-67 | H-870 | 97 | 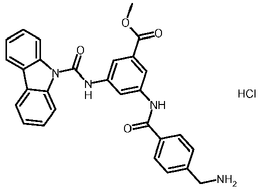 | (IV-I) |
| 3-68 | H-871 | 61 | 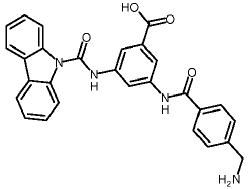 | (IV-I) |
| 3-69 | H-876 | 69 | 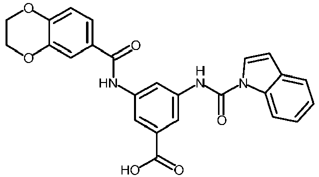 | (IV-I) |
| 3-70 | H-878 | 56 | 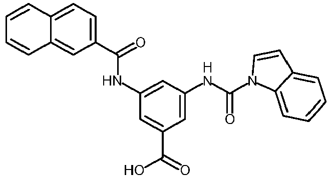 | (IV-I) |
| 3-71 | H-881 | 88 | 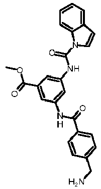 | (IV-I) |
| 3-72 | H-890 | 54 | 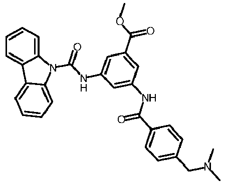 | (IV-I) |
| 3-73 | H-78 | 96 | 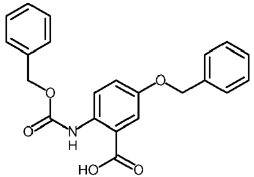 | |
| 3-74 | H-336 | 98 | 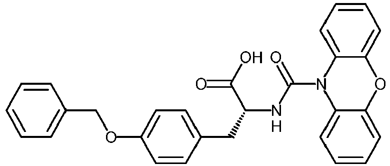 | |
| 3-75 | H-337 | 100 | 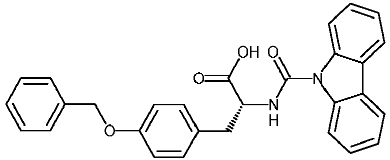 | |
| 3-76 | H-348 | 81 | 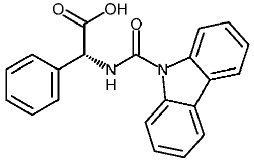 | |
| 3-77 | H-444 | 55 | 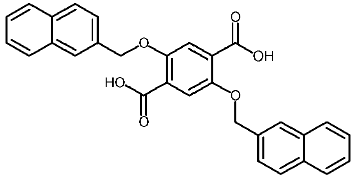 | |
| 3-78 | H-448 | 100 | 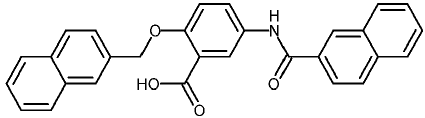 | |
| 3-79 | H-488 | 100 | 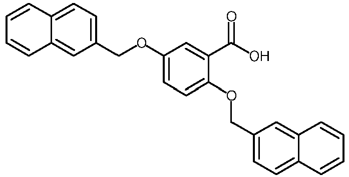 | |
| 3-80 | H-489 | 99 | 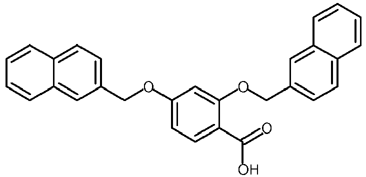 | |
| 3-81 | H-491 | 91 | 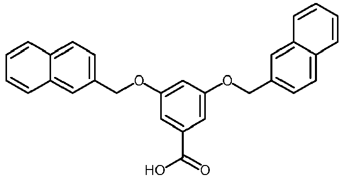 | |
| 3-82 | H-546 | 83 | 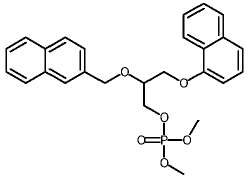 | |
| 3-83 | H-619 | 94 | 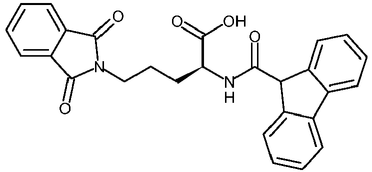 | |
| 3-84 | H-626 | 88 | 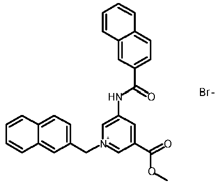 | |
| 3-85 | H-644 | 95 | 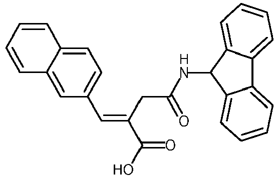 | |
| 3-86 | H-669 | 64 | 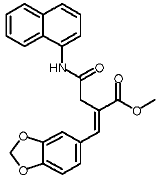 | |
| 3-87 | H-674 | 50 | 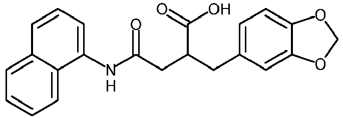 | |
| 3-88 | H-678 | 87 | 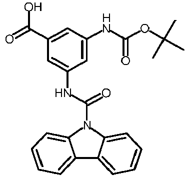 | |
| 3-89 | H-696-2 | 98 | 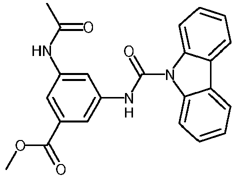 | |
| 3-90 | H-707 | 55 | 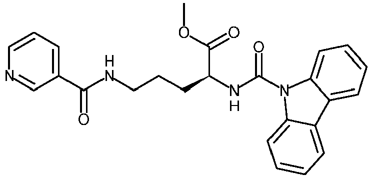 | |
| 3-91 | H-708 | 62 | 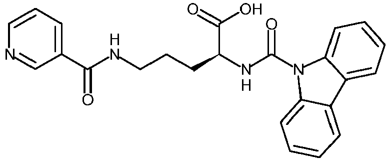 | |
| 3-92 | H-715 | 63 | 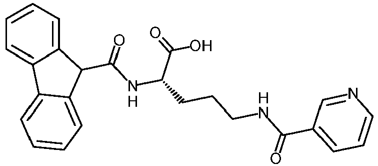 | |
| 3-93 | H-717 | 87 | 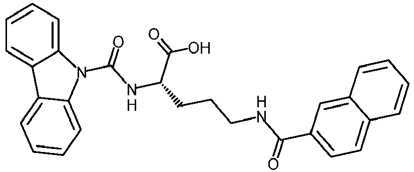 | |
| 3-94 | H-725-2 | 81 | 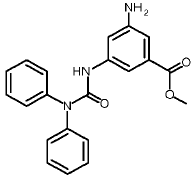 | |
| 3-95 | H-727 | 50 | 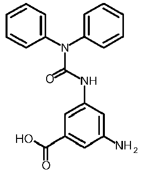 | |
| 3-96 | H-739 | 51 | 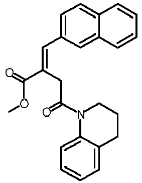 | |
| 3-97 | H-742 | 57 | 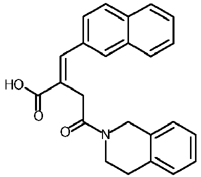 | |
| 3-98 | H-743 | 59 | 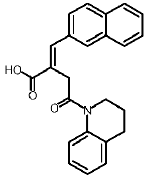 | |
| 3-99 | H-755 | 78 | 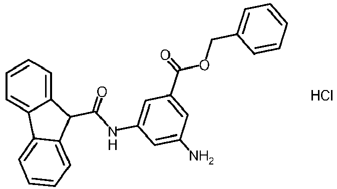 | |
| 3-100 | H-755-2 | 75 | 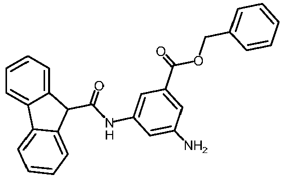 | |
| 3-101 | H-816 | 63 | 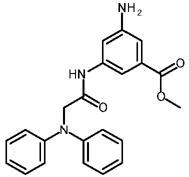 | |
| 3-102 | H-817 | 61 | 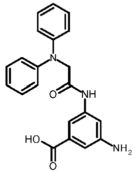 | |
| 3-103 | H-832 | 65 | 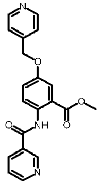 | |
| 3-104 | H-848 | 53 | 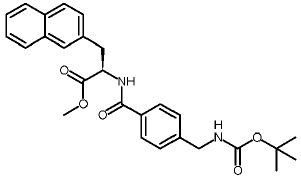 | |
| 3-105 | H-849 | 50 | 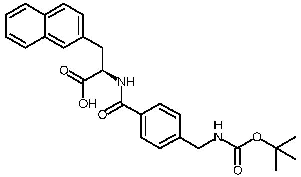 | |
| 3-106 | H-851 | 59 | 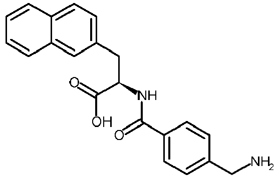 | |
| 3-107 | H-872 | 78 | 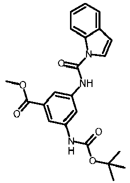 | |
| 3-108 | H-873 | 67 | 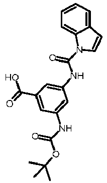 | |

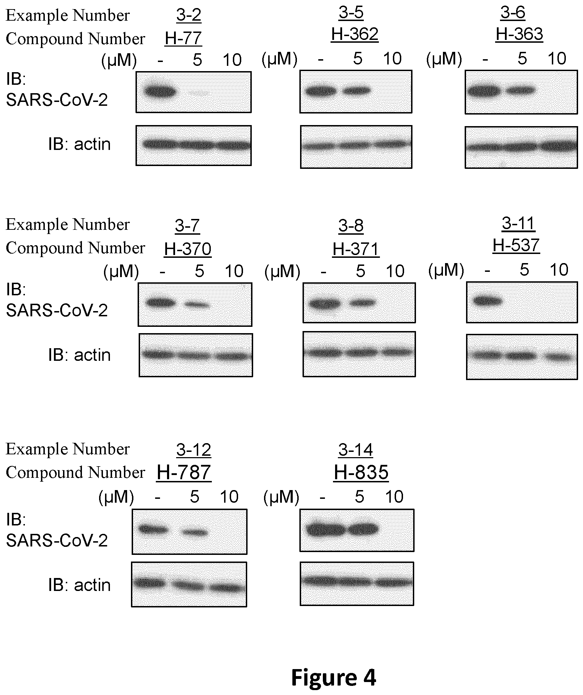

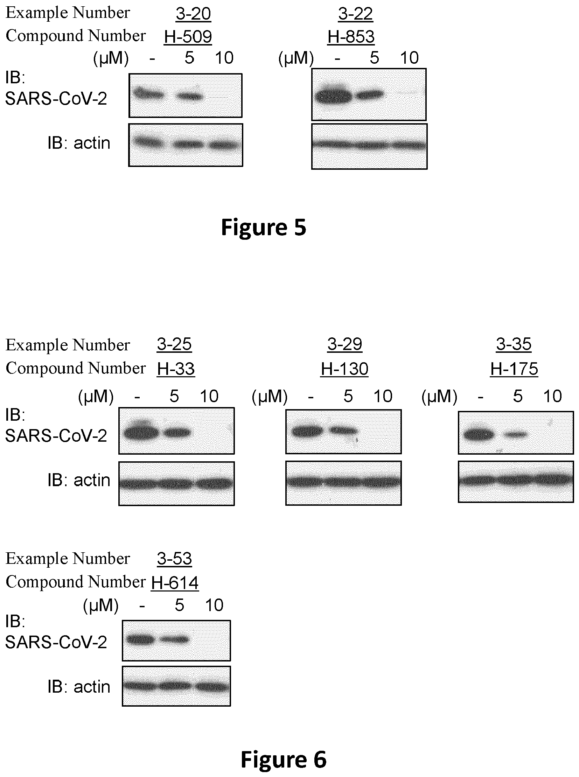

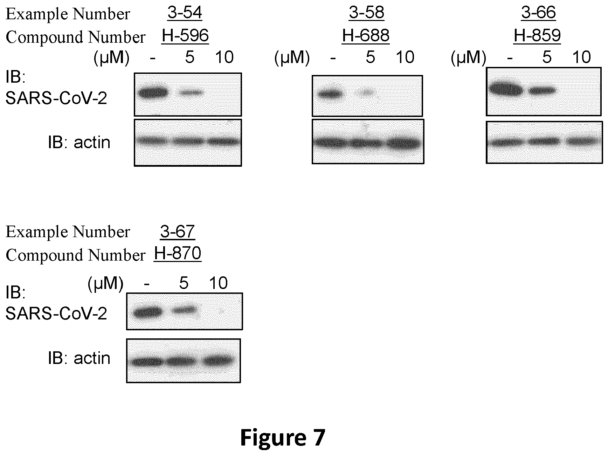

In addition, in Table 2, the Pin1 inhibitors which showed high inhibition rate were observed to confirm a suppressing effect against SARS-CoV-2 proliferation when added at 5 µM or 10 µM. To 1 × 10⁵ VeroE6 / TMPRSS2 cells /well was added a Pin1 inhibitor to a final concentration of 5 or 10 µM, to which SARS-CoV-2 was infected at multiplicity of infection (MOI) of 10 after 2 hours, followed by recovering cell lysate 8 hours after the infection and then detecting SARS-CoV-2 nucleocapsid and actin as an internal standard in the cells by Western blotting. FIG. 4 shows the result of adding the Pin1 inhibitors of the Formula (I-I) (H-77 (Example 3-2), H-362 (Example 3-5), H-363 (Example 3-6), H-370 (Example 3-7), H-371 (Example 3-8), H-537 (Example 3-11), H-787 (Example 3-12), and H-835 (Example 3-14)). FIG. 5 shows results of adding the Pin1 inhibitors of the Formula (II-I) (H-509 (Example 3-20) and H-853 (Example 3-22)). FIG. 6 shows results of adding the Pin1 inhibitors of the Formula (III-I) (H-33 (Example 3-25), H-130 (Example 3-29), H-175 (Example 3-35), and H-614 (Example 3-53)). FIG. 7 shows results of adding the Pin1 inhibitors of the Formula (IV-I) (H-596 (Example 3-54), H-688 (Example 3-58), H-859 (Example 3-66), and H-870 (Example 3-67)). FIGs. 4 to 7 shows that H-77, H-537, and H-688 exhibited high inhibition rate even at 5 µM addition amount. Therefore, they are expected to have therapeutic and prophylactic effects when administered at a low concentration.

### 6-4. Example 4

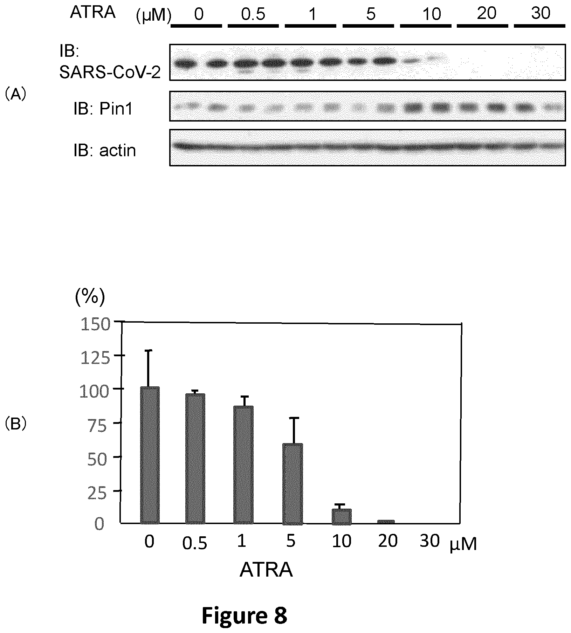

In this Example, the inhibitory effect of SARS-CoV-2 proliferation by ATRA, a therapeutic agent for promyelocytic leukemia known to have inhibitory activity against Pin1. ATRA was added to VeroE6 / TMPRSS2 cells to the final concentrations of 0, 0.5, 1, 5, 10, 20, and 30 µM, to which SARS-CoV-2 was infected at multiplicity of infection (MOI) of 0.01 after 2 hours, followed by recovering cell lysate after 24 hours and then detecting SARS-CoV-2 nucleocapsid, Pin1 and actin as an internal standard in the cells by Western blotting. SARS-CoV-2 proliferation was evaluated on the basis of the expression level of SARS-CoV-2 nucleocapsid in the cells. FIG. 8 (A) shows results of Western blotting. FIG. 8 (B) shows results by real-time PCR evaluation of SARS-CoV-2 RNA level in the culture supernatant 24 hours after infection in the aforementioned experiment. As shown in FIGs. 8, the proliferation of SARS-CoV-2 was suppressed by adding 5 µM or more of ATRA, and in particular, the proliferation of SARS-CoV-2 was substantially suppressed by adding 10 µM or more of ATRA.

### 6-5. Example 5

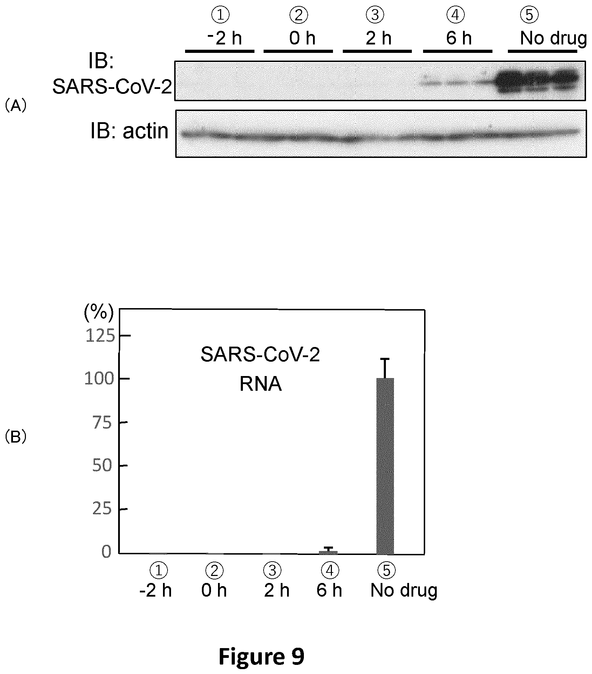

In this Example, it was confirmed that the Pin1 inhibitor H-77 exhibited a suppressive effect even after infection with SARS-CoV-2. VeroE6/TMPRSS2 cells were infected with SARS-CoV-2 at multiplicity of infection (MOI) of 0.01 to 0.02, to which H-77 was added either: 1. 2 hours before the infection, 2. at the time of the infection, 3. 2 hours after the infection, or 4. 6 hours after the infection, to a final concentration of 10 µM. Alternatively, 5. H-77 was not added. Cell lysate was recovered 24 hours after the infection, and then SARS-CoV-2 nucleocapsid in the cells and actin as an internal standard were detected by Western blotting. SARS-CoV-2 proliferation was evaluated on the basis of the expression level of SARS-CoV-2 nucleocapsid in the cells. FIG. 9 (A) shows results of Western blotting. FIG. 9 (B) shows results by real-time PCR evaluation of SARS-CoV-2 RNA level in the culture supernatant 24 hours after infection in the aforementioned experiment. As shown in FIGs. 9, it was confirmed that SARS-CoV-2 proliferation could be suppressed (1) when H-77 was added before the SARS-CoV-2 infection, (2) when H-77 was added at the time of the SARS-CoV-2 infection, and (3, 4) when H-77 was added after the SARS-CoV-2 infection. In other words, it was confirmed that the compound according to the present invention functions both as a prophylactic agent and as a therapeutic agent. However, since SARS-CoV-2 was slightly detected when H-77 was added 6 hours after the infection, it is preferable to administer H-77 as soon as possible after infection.

### 6-6. Example 6 Screening Method

In this Example, therapeutic or prophylactic agents for coronavirus infection caused by SARS-CoV-2 were identified by a screening method comprising steps of: acquiring a library of Pin1 inhibitors using a cell-free assay; and measuring their ability to inhibit proliferation of SARS-CoV-2 virus using a cell-based assay. First, a cell-free assay for measuring cis-to-trans conversion of a phosphorylated serine/threonine-proline motif contained in a substrate peptide was used to measure inhibitory activity against the function of Pin1, and the library of Pin1 inhibitors was acquired (see, Bernhard Janowski, and 3 other authers, Analytical Biochemistry, October 15, 1997, Vol.252(2), p.299-307). Succ-AEPF-pNA (succinyl-alanine-glutamic acid-proline-phenylalanine-p-nitroanilide) was used as the substrate peptide. Approximately 60% of the substrate was retained in cis conformation in LiClItrifluoroethanol. A Pin1 enzyme alone, or the Pin1 enzyme and 2 µM or 20 µM of the test compound were added, and the kinetics of Pin1 cis/trans isomerase activity was measured for 1000 seconds using a UV/Vis spectrophotometer. The enzymatic rate constant of the reaction was determined by fitting the data to a first-order kinetics equation, and the inhibitory activity against the function of Pin1 of the test compound was measured. Examples of the test compound that can be used include the compounds having the chemical structures represented by the Formula (I-I), (II-I), (III-I), (IV-I) or Table 1, or the salts thereof. As a result of the measurement, the Pin1 inhibitor library was generated by collecting compounds whose rate of inhibiting the function of Pin1 (1-enzyme rate constant of the test compound/enzyme rate constant of the Pin1 enzyme alone) was greater than or equal to a predetermined value, for example, 10% or more, preferably 50% or more of the rate of the Pin1 enzyme alone. Then, with respect to the compounds identified as Pin1 inhibitor as a result of the aforementioned measurement, their ability to inhibit proliferation of SARS-CoV-2 virus using a cell-based assay for measuring increases in viral components. For example, the suppressing effect against SARS-CoV-2 proliferation was confirmed by the method described in Example 3-1. As a result, the test compounds that has been shown to have the suppressing effect against SARS-CoV-2 proliferation were identified as therapeutic or prophylactic agents for coronavirus infection caused by SARS-CoV-2. As described above, the screening method comprising the seteps of: acquiring the library of Pin1 inhibitors using the cell-free assay; and measuring their ability to inhibit proliferation of SARS-CoV-2 virus using the cell-based assay allows for the identification of therapeutic or prophylactic agents for coronavirus infection caused by SARS-CoV-2 from a group of the test compounds such as the compounds having the chemical structures represented by the Formula (I-I), (II-I), (III-I), (IV-I) or Table 1, or the salts thereof.

### INDUSTRIAL APPLICABILITY

Both therapeutic and prophylactic agents for viral diseases according to the present invention are useful in the pharmaceutical industry.

## Claims

1. A therapeutic or prophylactic agent for COVID-19 containing a compound with a function of inhibiting Pin1 or a pharmaceutically acceptable salt thereof as an active ingredient.

2. The therapeutic or prophylactic agent according to claim 1, wherein the compound is a compound represented by the following Formula (I-I): 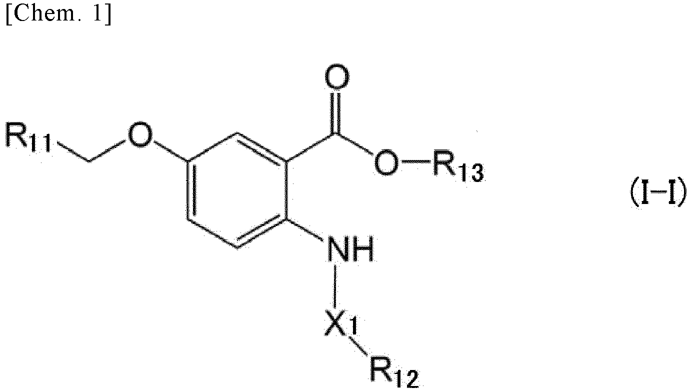 (wherein at least one of R₁₁ and R₁₂ is an optionally substituted polycyclic aryl group, an optionally substituted polycyclic heterocyclic group, or a group represented by the following Formula (I-II): 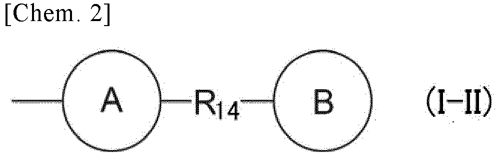 (wherein rings A and B respectively represent an optionally substituted monocyclic or polycyclic aryl group, and R₁₄ represents an optionally substituted alkylene group having 1 to 3 carbon atoms, an optionally substituted alkenylene group having 2 to 3 carbon atoms, or divalent oxy group),
if neither R₁₁ nor R₁₂ is any of the aforementioned groups, R₁₁ or R₁₂ is a hydrogen atom, an optionally substituted hydrocarbon group, or an optionally substituted monocyclic heterocyclic group,
R₁₃ is a hydrogen atom, an optionally substituted hydrocarbon group, or an optionally substituted heterocyclic group, and
X₁ is a single bond, -CO- group, -CO-O-CH₂- group, -CO-CH₂-O- group, -SO₂-group, -CH₂-CO-NH- group, -CH₂-CO- group, -CH₂- group, or -CO-CH₂- group).

3. The therapeutic or prophylactic agent according to claim 2, wherein at least one of the R₁₁ and the R₁₂ is an optionally substituted polycyclic aryl group.

4. The therapeutic or prophylactic agent according to claim 3, wherein at least one of the R₁₁ and the R₁₂ is an optionally substituted naphthyl group.

5. The therapeutic or prophylactic agent according to claim 4, wherein the R₁₁ is an optionally substituted naphthyl group.

6. The therapeutic or prophylactic agent according to any one of claims 2 to 5, wherein the R₁₃ is a hydrogen atom or a methyl group.

7. The therapeutic or prophylactic agent according to any one of claims 2 to 6, wherein X₁ is -CO- group.

8. The therapeutic or prophylactic agent according to claim 1, wherein the compound is a compound represented by the following Formula (II-I): 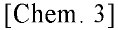 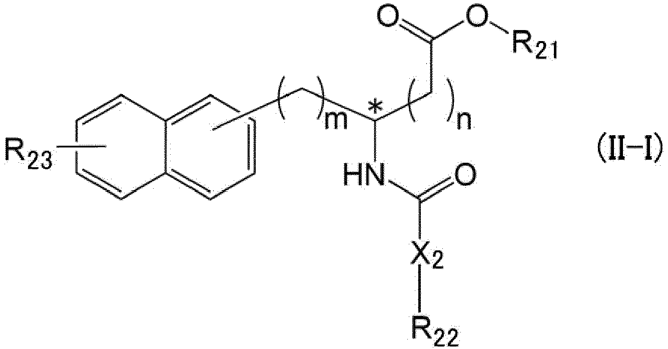 (wherein m represents an integer from 0 to 2, n represents an integer from 0 to 1, and 0≤m+n≤2,
R₂₁ is a hydrogen atom, an optionally substituted hydrocarbon group, an optionally substituted heterocyclic group, or an optionally substituted amino group,
R₂₂ is an optionally substituted aryl group containing a condensed ring of three or more rings, a substituted naphthyl group, or a group represented by the following Formulae (II-II), (II-III), (II-IV), or (II-V): 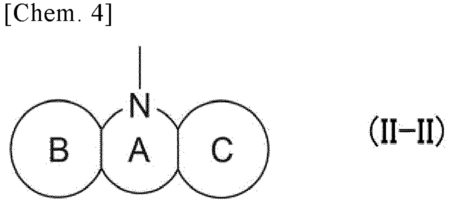 (wherein a ring A represents an optionally substituted monocyclic heterocyclic group, and rings B and C respectively represent an optionally substituted monocyclic or polycyclic aryl group or heterocyclic group, and the rings A, B and C form a condensed ring); 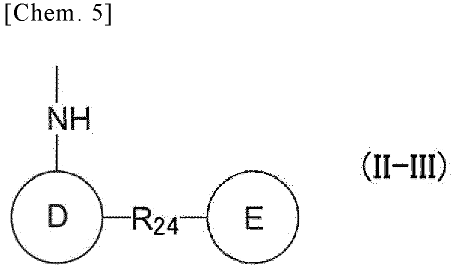 (wherein rings D and E respectively represent an optionally substituted monocyclic or polycyclic aryl group or heterocyclic group, and R₂₄ represents a divalent oxy group or a carbonyl group); 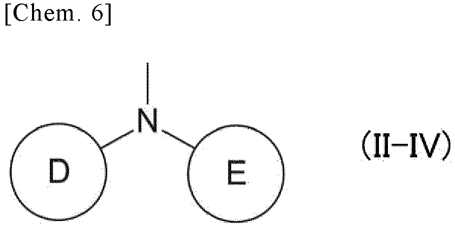 (wherein rings D and E respectively represent an optionally substituted monocyclic or polycyclic aryl group or heterocyclic group); 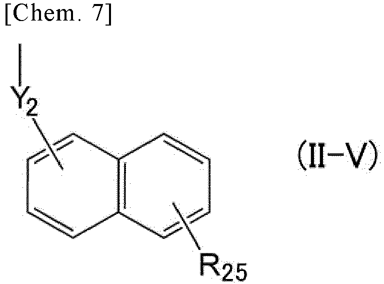 (wherein R₂₅ represents 0 to 7 identical or different substituents attached to a naphthyl group, and Y₂ represents -NH- group or an alkylene group having 1 or 2 carbon atoms),
R₂₃ is 0 to 7 identical or different substituents attached to a naphthyl group, and
X₂ is a single bond, -CH₂- group, or -NH- group).

9. The therapeutic or prophylactic agent according to claim 8, wherein the R₂₂ is a group represented by the following Formula (II-II): 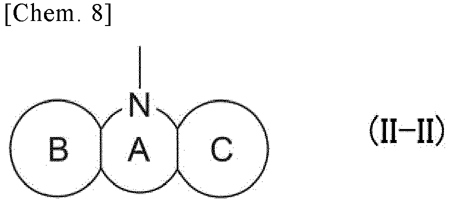 (wherein a ring A represents an optionally substituted monocyclic heterocyclic group, rings B and C respectively represent an optionally substituted monocyclic or polycyclic aryl group or heterocyclic group, and the rings A, B and C form a condensed ring).

10. The therapeutic or prophylactic agent according to claim 8 or 9, wherein the m is 1, and the n is 0.

11. The therapeutic or prophylactic agent according to any one of claims 8 to 10, wherein the R₂₁ is a hydrogen atom.

12. The therapeutic or prophylactic agent according to any one of claims 8 to 11, wherein the X₂ is a single bond.

13. The therapeutic or prophylactic agent according to claim 1, wherein the compound is a compound represented by the following Formula (III-I): 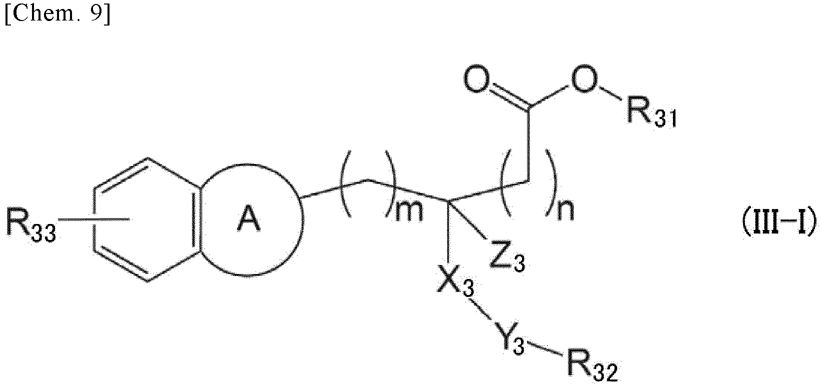 (wherein m represents an integer from 0 to 2, n represents an integer from 0 to 2, and 0≤m+n≤3,
a ring A is an optionally substituted monocyclic aromatic ring or heterocycle,
X₃ is a single bond, -NH- group, -NH-CO- group, -O-CO- group, -CH₂-S-CH₂-group, -CH₂-S-(CH₂)₂-S- group, or -NH-R₃₄- group (R₃₄ is an alkylene group having 1 to 5 carbon atoms, or an alkenylene group having 2 to 5 carbon atoms),
Y₃ is a single bond, -NH- group, -CO- group, -SO₂- group, -O-CO- group, -CO-NR₃₅- group (R₃₅ represents a hydrogen atom, or an optionally substituted alkyl group having 1 to 5 carbon atoms), -O-R₃₆- group (R₃₆ represents an optionally substituted alkylene group having 1 to 5 carbon atoms, or an optionally substituted alkenylene group having 2 to 5 carbon atoms), -NR₃₅-R₃₆- group, -S-R₃₆- group, -CO-R₃₆- group, - SO₂-R₃₆- group, -NR₃₅-CO-R₃₆- group, -R₃₆-NR₃₅-CO- group, an optionally substituted alkylene group having 1 to 6 carbon atoms, or an optionally substituted alkenylene group having 2 to 6 carbon atoms,
Z₃ is a hydrogen atom, an optionally substituted amino group, or an optionally substituted amine,
if the Z₃ is the optionally substituted amine, the X₃ and the Z₃ form a ring,
R₃₁ is a hydrogen atom, an optionally substituted hydrocarbon group, an optionally substituted heterocyclic group, or an optionally substituted amino group,
R₃₂ is an optionally substituted aryl group, an optionally substituted heterocyclic group, an optionally substituted cycloalkyl group, or a group represented by the following Formula (III-II): 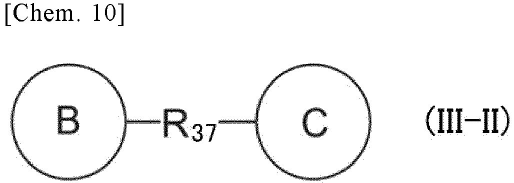 (wherein rings B and C respectively represent an optionally substituted aryl group, an optionally substituted heterocyclic group, or an optionally substituted cycloalkyl group, R₃₇ represents, a single bond, -O- group, -CO- group, -NH- group, -SO₂- group, -CO-NH- group, an optionally substituted alkylene group having 1 to 3 carbon atoms, an optionally substituted alkenylene group having 2 to 3 carbon atoms, -S-R₃₈- group (R₃₈ represents an optionally substituted alkylene group having 1 to 2 carbon atoms), -COR₃₈- group, -O-R₃₈- group, or -SO₂-R₃₈- group, and any of the ring B, ring C and R₃₇ is attached to the Y₃), and
R₃₃ is 0 to 4 identical or different substituents).

14. The therapeutic or prophylactic agent according to claim 13, wherein the m is 1, and the n is 0.

15. The therapeutic or prophylactic agent according to claim 13 or 14, wherein the X₃ is -NH-CO- group, or -O-CO- group.

16. The therapeutic or prophylactic agent according to any one of claims 13 to 15, wherein the ring A is a benzene ring.

17. The therapeutic or prophylactic agent according to any one of claims 13 to 16, wherein the R₃₂ is an optionally substituted polycyclic aryl group, or an optionally substituted polycyclic heterocyclic group.

18. The therapeutic or prophylactic agent according to any one of claims 13 to 17, wherein the Z₃ is a hydrogen atom.

19. The therapeutic or prophylactic agent according to any one of claims 13 to 18, wherein the Y₃ is a single bond, -CH₂- group, or -CH₂-O- group.

20. The therapeutic or prophylactic agent according to any one of claims 13 to 19, wherein the R₃₁ is a hydrogen atom.

21. The therapeutic or prophylactic agent according to claim 1, wherein the compound is a compound represented by the following Formula (IV-1): 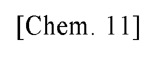 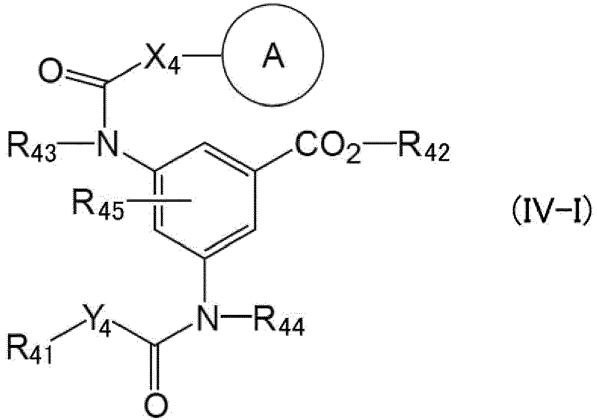 (wherein a ring A represents an optionally substituted monocyclic or polycyclic aromatic ring or heterocycle,
R₄₁ represents an optionally substituted polycyclic aromatic ring or a group represented by any of the following Formulae (IV-II) to (IV-V): 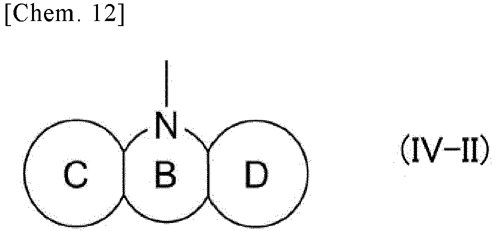 (wherein a ring B represents an optionally substituted monocyclic heterocycle, rings C and D independently represent an optionally substituted monocyclic or polycyclic aromatic ring, heterocycle or cyclic hydrocarbon, and the rings B, C and D form a condensed ring); 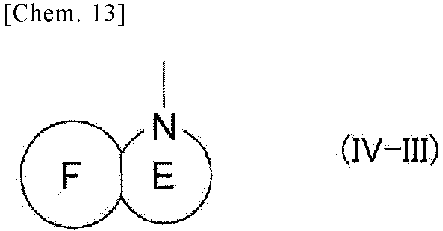 (wherein a ring E represents an optionally substituted monocyclic heterocycle, a ring F represents an optionally substituted monocyclic or polycyclic aromatic ring, heterocycle or cyclic hydrocarbon, and the rings E and F form a condensed ring); 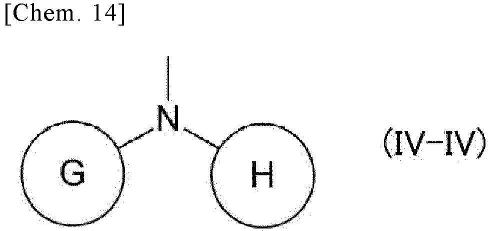 (wherein rings G and H respectively represent an optionally substituted monocyclic or polycyclic aromatic ring or heterocycle); 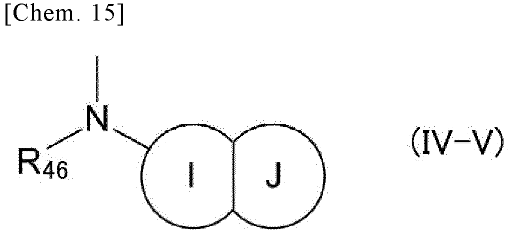 (wherein a ring I represents an optionally substituted monocyclic aromatic ring or heterocycle, a ring J represents an optionally substituted monocyclic or polycyclic aromatic ring, heterocycle or cyclic hydrocarbon, the rings I and J form a condensed ring, and R₄₆ represents a hydrogen atom, an optionally substituted hydrocarbon group, or an optionally substituted heterocyclic group);
R₄₂ represents a hydrogen atom, an optionally substituted hydrocarbon group, an optionally substituted heterocyclic group, or an optionally substituted amino group;
R₄₃ represents a hydrogen atom, an optionally substituted hydrocarbon group, or an optionally substituted heterocyclic group;
R₄₄ represents a hydrogen atom, an optionally substituted hydrocarbon group, or an optionally substituted heterocyclic group;
R₄₅ represents 0 to 3 identical or different substituents attached to a benzene ring;
X₄ represents a single bond, an alkylene group having 1 or 2 carbon atoms, -O-group, -CH₂-O- group, -CH₂-NH-CO- group, or -CH₂-NH-CO-O-CH₂- group; and
Y₄ represents a single bond or an alkylene group having 1 or 2 carbon atoms).

22. The therapeutic or prophylactic agent according to claim 21, wherein the ring A is an optionally substituted polycyclic aromatic ring or heterocycle.

23. The therapeutic or prophylactic agent according to claim 22, wherein the ring A is a ring represented by the following Formula (IV-VI): 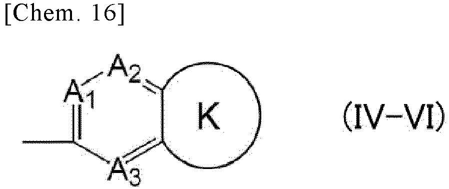 (wherein A₁, A₂, and A₃ independently represent a carbon atom or a nitrogen atom, and a ring K represents an optionally substituted monocyclic or polycyclic aromatic ring, heterocycle or cyclic hydrocarbon).

24. The therapeutic or prophylactic agent according to claim 23, wherein all of A₁, A₂, and A₃ are a carbon atom.

25. The therapeutic or prophylactic agent according to any one of claims 21 to 24, wherein the R₄₁ is preferably a group represented by the following Formula (IV-II): 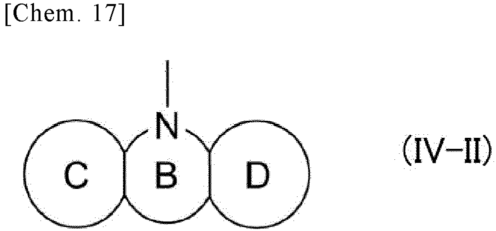 (wherein a ring B represents an optionally substituted monocyclic heterocycle, rings C and D independently represent an optionally substituted monocyclic or polycyclic aromatic ring, heterocycle or cyclic hydrocarbon, and the rings B, C and D form a condensed ring).

26. The therapeutic or prophylactic agent according to any one of claims 21 to 25, wherein the R₄₂ is a hydrogen atom.

27. The therapeutic or prophylactic agent according to any one of claims 21 to 26, wherein the R₄₃ is a hydrogen atom.

28. The therapeutic or prophylactic agent according to any one of claims 21 to 27, wherein the R₄₄ is a hydrogen atom.

29. The therapeutic or prophylactic agent according to any one of claims 21 to 28, wherein the X₄ is a single bond.

30. The therapeutic or prophylactic agent according to any one of claims 21 to 29, wherein the Y₄ is a single bond.

31. A method for screening therapeutic or prophylactic agents for COVID-19, comprising steps of:
A) acquiring a library of Pin1 inhibitors by measuring their activity of inhibiting the function of Pin1 using a cell-free assay for measuring cis-to-trans conversion of a phosphorylated serine/threonine-proline motif contained in a substrate peptide; and
B) measuring ability to inhibit proliferation of SARS-CoV-2 virus of compounds identified as Pin1 inhibitors in the step A using a cell-based assay for measuring increases in viral components.

32. A therapeutic or prophylactic agent for COVID-19 containing the Pin1 inhibitors identified by the screening method according to claim 31 or pharmaceutically acceptable salts thereof as an active ingredient.

33. The therapeutic or prophylactic agent according to any one of claims 1 to 30 and 32, further containing one or more active ingredients of at least one or more other drugs selected from drugs classified as therapeutic or prophylactic agents for coronavirus infections.

34. The therapeutic or prophylactic agent according to any one of claims 1 to 30, 32, and 33 for treating or preventing coronavirus infections in combination with at least one or more other drugs selected from drugs classified as therapeutic or prophylactic agents for coronavirus infections.

35. Use of the therapeutic or prophylactic agent according to any one of claims 1 to 30, 32, and 33 for manufacture of a medicament for treating or preventing coronavirus infection.

36. A method of treating or preventing COVID-19, comprising administering to a subject in need thereof a therapeutically effective amount of the therapeutic or prophylactic agent according to any one of claims 1 to 30, 32, and 33.

37. A compound with a function of inhibiting Pin1 or a pharmaceutically acceptable salt thereof for use in treating or preventing coronavirus infection.
